(19) Europäisches Patentamt
European Patent Office
Office européen des brevets

(11) EP 4 527 937 A1

# (12) EUROPEAN PATENT APPLICATION

(43) Date of publication:
**26.03.2025 Bulletin 2025/13**

(21) Application number: **23198653.0**

(22) Date of filing: **20.09.2023**

(51) International Patent Classification (IPC):
***C12P 19/34*** *(2006.01)* ***C12N 15/10*** *(2006.01)*

(52) Cooperative Patent Classification (CPC):
**C12P 19/34**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **BioNTech SE**
**55131 Mainz (DE)**

(72) Inventor: **SEEFELDT, Alexandra Carolin**
**55131 Mainz (DE)**

(74) Representative: **D Young & Co LLP**
**3 Noble Street**
**London EC2V 7BQ (GB)**

## (54) ONE-POT IN VITRO TRANSCRIPTION OF MULTIPLE DNA MOLECULES TO MULTIPLE RNA MOLECULES

(57) The present invention provides nucleic acid compositions and methods of making nucleic acid compositions. Applications of such nucleic acid compositions include uses in therapy.

EP 4 527 937 A1

## Description

### Technical Field of the Invention

The present invention relates to the technical field of nucleic acid compositions and methods of making nucleic acid compositions. Applications of such nucleic acid compositions include uses in therapy.

### Background to the Invention

Nucleic acid-based compositions, i.e. compositions comprising one or more than one different nucleic acid, have numerous applications that are constantly being developed and expanded upon. In particular, compositions that comprise at least two different nucleic acids, i.e. two nucleic acids having different sequences, are of interest. Possible applications for such compositions include mRNA-based vaccines comprising multiple different mRNA sequences, each encoding one or more different antigens. Such a composition comprising two or more different RNA molecules can be used as a vaccine that comprises/encodes two or more different sets of antigens.
The production of compositions comprising two or more different nucleic acid molecules has typically been carried out in two or more separate stages. Traditionally, there will be a separate production process and reaction vessel for each of the different nucleic acid sequences. Thus, each of the different nucleic acids will be made, e.g. by *in vitro* transcription (IVT) from DNA to RNA, and purified separately. Subsequently, the different nucleic acids produced will be mixed to provide the composition that comprises two or more different nucleic acids.
It is desirable, particularly for therapeutic applications, for compositions that comprise two or more different nucleic acids to comprise pre-determined, known ratios of the two or more different nucleic acids therein. For example, it may be desired to provide a composition comprising equal amounts or concentrations of the different nucleic acids, but it may also be desired to provide a composition comprising, e.g., equal amounts or concentrations of a first and second nucleic acid, and twice the amount or concentration of a third nucleic acid (1:1:2). The specific ratio depends on the application. Thus, when following the traditional separate-stage method outline above, following the separate production of the nucleic acids, the concentration of each of the nucleic acids must be determined and carefully adjusted to meet the requirements of the mixed composition, which is a complicated, expensive and time-consuming process.

### Summary of the Invention

Although separate-stage production of nucleic acid compositions comprising two or more different nucleic acids is widely used and accepted in the art, the present inventors sought to provide an improved method. In particular, the present inventors developed a method that accounts, e.g., for differences in nucleic acid lengths and desired end fractions of RNA, and surprisingly found that the method can be used to produce desired quantities of multiple different nucleic acids in the same IVT reaction. This method was unexpectedly found to be very advantageous, as it was determined to reduce production time by more than a factor of three (when producing a composition comprising three different nucleic acids). This included reducing the number of production reactions and purification steps, as well as allowing the final step of mixing of individual nucleic acids to be omitted. Moreover, the method of the present invention enables the desired ratio of different nucleic acids to be pre-determined, easily achieved, and easily adjusted as needed.
Accordingly, in a first aspect, the present invention provides a method of making a composition comprising 2 or more different RNA molecules, wherein each of the different RNA molecules is obtainable from a different DNA molecule, wherein the method comprises:

a) providing a reaction mixture comprising the DNA molecules, wherein each of the different DNA molecules (*X*) has a concentration according to equation (i):

$$c(DNA_X) = \frac{c(DNA_{total}) \cdot RNA\ Fraction \cdot DNA_x\ length\ fraction}{RNA_x\ length\ fraction} \quad \text{(i)}$$

wherein:

"$c(DNA_x)$" is the concentration of a given DNA molecule *X*;

"$c(DNA_{total})$" is the total concentration of all of the different DNA molecules;

"RNA fraction" is the fraction of the total RNA molecules in the composition that comprises the RNA molecule that

is obtainable from the DNA molecule *X*;

"$DNA_x$ length fraction" is the base pair (bp) length of the DNA molecule *X*, divided by the total bp length of all of the different DNA molecules;

"$RNA_x$ length fraction" is the bp length of the RNA molecule that is obtainable from the DNA molecule *X*, divided by the total bp length of all of the different RNA molecules; and

b) obtaining the RNA molecules from the DNA molecules in the reaction mixture, thereby making the composition.

In a second aspect, the present invention provides a method of making a composition comprising at least a first RNA molecule and a second RNA molecule, wherein the first RNA molecule is obtainable from a first DNA molecule and the second RNA molecule is obtainable from a second DNA molecule, wherein the method comprises:

a) providing a reaction mixture comprising the first DNA molecule and the second DNA molecule; and

b) obtaining the first and second RNA molecules from the first and second DNA molecules respectively;

wherein the first RNA molecule has a greater relative mass than the second RNA molecule, and the second RNA molecule is obtained from the second DNA molecule in a molar amount that is greater than the molar amount of the first RNA molecule that is obtained from the first DNA molecule, such that the first and second RNA molecules are obtained at essentially the same concentrations in the composition.

In a third aspect, the present invention provides the composition obtained or obtainable by the methods of the present invention.

In a fourth aspect, the present invention provides the composition obtained or obtainable by the methods of the present invention, for use as a medicament.

In a fifth aspect, the present invention provides a composition comprising 2 or more different DNA molecules, wherein a different RNA molecule is obtainable from each of the different DNA molecules, wherein each of the different DNA molecules (*X*) has a concentration according to equation (i):

$$c(DNA_X) = \frac{c(DNA_{total}) \cdot RNA\ Fraction \cdot DNA_X\ length\ fraction}{RNA_X\ length\ fraction} \quad \text{(i)}$$

wherein:

"$c(DNA_x)$" is the concentration of a given DNA molecule *X*;

"$c(DNA_{total})$" is the total concentration of all of the DNA molecules;

"RNA fraction" is the fraction of all RNA molecules obtainable from the reaction mixture that comprises the RNA molecule that is obtainable from the DNA molecule *X*;

"$DNA_x$ length fraction" is the base pair (bp) length of the DNA molecule *X*, divided by the total bp length of all of the different DNA molecules;

"$RNA_x$ length fraction" is the bp length of the RNA molecule obtainable from the DNA molecule *X*, divided by the total bp length of all of the different RNA molecules.

## Brief Description of the Figures

Figure 1: Graph showing dependence between total DNA template concentration and resulting IVT yield, in a one-pot reaction that was calculated to produce a 1:1:1 ratio of three product RNAs.

Figure 2: Graph showing dependence between starting ATP/CTP concentration (each of ATP concentration and CTP concentration were initially present at the x-axis values) and resulting IVT yield, in a one-pot reaction comprising

nucleic acid sequences with high A and C fractions.

Figure 3: Results from a ratio assay analysing IVT with three different nucleic acids. Different concentrations of DNA template encoding RNA product 3 were utilised in accordance with the methods of the present invention to generate different ratios.

Figure 4: Results from a ratio assay analysing IVT with three different nucleic acids. Different concentrations of DNA template encoding RNA products 1 and 2 were used in accordance with the methods of the present invention to generate different ratios.

## Detailed Description of the Invention

In a first aspect, the present invention provides a method of making a composition comprising 2 or more different RNA molecules, wherein each of the different RNA molecules is obtainable from a different DNA molecule, wherein the method comprises:

a) providing a reaction mixture comprising the DNA molecules, wherein each of the different DNA molecules (*X*) has a concentration according to equation (i):

$$c(DNA_X) = \frac{c(DNA_{total}) \cdot RNA\ Fraction \cdot DNA_x\ length\ fraction}{RNA_x\ length\ fraction} \quad \text{(i)}$$

wherein:

"c($DNA_x$)" is the concentration of a given DNA molecule *X*;
"c($DNA_{total}$)" is the total concentration of all of the different DNA molecules;
"RNA fraction" is the fraction of the total RNA molecules in the composition that comprises the RNA molecule that is obtainable from the DNA molecule *X*;
"$DNA_x$ length fraction" is the base pair (bp) length of the DNA molecule *X*, divided by the total bp length of all of the different DNA molecules;
"$RNA_x$ length fraction" is the bp length of the RNA molecule that is obtainable from the DNA molecule *X*, divided by the total bp length of all of the different RNA molecules; and

b) obtaining the RNA molecules from the DNA molecules in the reaction mixture, thereby making the composition.

Thus, the present invention provides a method of making a composition comprising two or more different product nucleic acids (RNA), from two or more different template nucleic acids (DNA) which proceeds in a single reaction mixture. This saves time and cost, and is generally more procedurally efficient than prior art methods of producing such compositions. Moreover, the method of the present invention enables the user to determine the "RNA fraction" of each product nucleic acid (RNA) X that will be produced in the resulting composition. This is the proportion of the total resulting product nucleic acids (RNA) that will be made up of each particular product nucleic acid (RNA) X. In other words, this allows the skilled person to determine the ratios between the different product nucleic acids (RNAs) that are produced by the methods of the present invention.

Although most often the template nucleic acids in the methods of the present invention comprise DNA, and the product nucleic acids in the methods of the present invention comprise RNA (as stated in the first aspect), it is to be understood that in embodiments broader applications of the methods and formula of the present invention, such as those including other types of template and product nucleic acids, are possible. For example, both the template and product nucleic acids could comprise DNA, and the product nucleic acids could be obtained from the template nucleic acids by DNA replication or non-standard transcription methods. For convenience, template nucleic acid is generally referred to as DNA and product nucleic acid as RNA herein.

An "RNA molecule" referred to herein means a molecule that comprises RNA polynucleotides. In an embodiment, an RNA molecule comprises one or more of a coding sequence, a non-coding RNA (ncRNA) sequence, a micro RNA (miRNA) sequence or a small interfering (siRNA) sequence. An RNA molecule may comprise modifications such as conjugations, backbone modifications, base modifications and sugar modifications. To an extent, an RNA molecule may also comprise other, non-RNA polynucleotides. In an embodiment, an RNA molecule comprises at least 100%, 99%, 98%, 97%, 96%, 95%, 94%, 93%, 92%, 91%, 90%, 89%, 88%, 87%, 86%, 85%, 84%, 83%, 82%, 81%, 80%, 75%, 70%, 65%, 60%, 55% or 50% RNA polynucleotides.

A "DNA molecule" referred to herein means a molecule that comprises DNA polynucleotides. In an embodiment, a DNA molecule comprises a sequence that is transcribable into an RNA sequence comprising one or more of a non-coding RNA (ncRNA) sequence, a micro RNA (miRNA) sequence or a small interfering (siRNA) sequence. In an embodiment, a DNA molecule further comprises non-transcribed DNA such as one or more of a promoter sequence, PCR template sequence or linearised plasmid sequence. A DNA molecule may comprise modifications such as conjugations, backbone modifications, base modifications and sugar modifications. To an extent, a DNA molecule may also comprise other, non-DNA polynucleotides. In an embodiment, a DNA molecule comprises at least 100%, 99%, 98%, 97%, 96%, 95%, 94%, 93%, 92%, 91%, 90%, 89%, 88%, 87%, 86%, 85%, 84%, 83%, 82%, 81%, 80%, 75%, 70%, 65%, 60%, 55% or 50% DNA polynucleotides.

The present invention relates to a method of making a composition comprising 2 or more different RNA molecules. Generally herein, "different" in the context of e.g. two or more RNA molecules or DNA molecules means that the molecules have different nucleotide sequences. In an embodiment, the different RNA molecules also encode different polypeptide sequences. Thus, it is to be understood that references to "different" RNA or DNA "molecules" do not refer to single molecules perse, but to each different type (sequence) of RNA or DNA molecule that is present. Accordingly, in an embodiment, "two or more different RNA molecules" means "two or more different species of RNA", e.g. two or more RNA species of different sequences. Accordingly, in an embodiment, "RNA molecule" means RNA species. Accordingly, in an embodiment, "DNA molecule" means DNA species.

In an embodiment, each of the different RNA molecules is obtainable from a different DNA molecule by transcription. Thus, in an embodiment, the RNA molecules of the present invention are obtainable from two or more different corresponding DNA molecules. In the present invention, the template DNA molecules are the starting material, and therefore comprise not only a sequence to be transcribed into RNA, but also any non-transcribed sequences, including regulatory sequences such as a promoter sequence. In an embodiment, the DNA molecules comprise a PCR template sequence or linearised plasmid sequence. Thus, for considerations involving the length of the DNA molecules, the full length of the DNA molecules including non-transcribed sequences are to be taken into account. For considerations involving the length of the RNA molecules, the full length of the RNA molecule that will be obtained from the DNA molecule from transcription in the context of the transcription conditions that are employed must be taken into account.

The "reaction mixture" in step a) of the method of the present invention is understood to comprise a single composition in which the (two or more) different DNA molecules of the invention are present. In an embodiment, the reaction mixture is a single composition. In an embodiment, the reaction mixture is comprised in a single vessel. In an embodiment, the (two or more) different DNA molecules of the present invention are comprised in a single vessel.

In the present invention, the concentration of each (i.e. every one of) the different DNA molecules in the reaction mixture that contributes to the RNA obtained is determined via the formula (i). Herein, "*X*" is used to refer to the given DNA molecule in respect of which the concentration is being calculated, but it is to be understood that the calculation is carried out for each different DNA molecule in turn.

$$c(DNA_X) = \frac{c(DNA_{total}) \cdot RNA\ Fraction \cdot DNA_x\ length\ fraction}{RNA_x\ length\ fraction} \quad (i)$$

"c($DNA_X$)" is the concentration of a given DNA molecule *X*. In the present invention, the concentration of each of the different DNA molecules is in terms of the initial concentration in mass concentration, i.e. mass per unit volume, such as mass per unit volume of the reaction mixture. The total DNA concentration is also in terms of the initial concentration in mass concentration, i.e. mass per unit volume, such as mass per unit volume of the reaction mixture.

"c($DNA_{total}$)" is the total concentration of all of the different DNA molecules in the reaction mixture. Thus, the total concentration of DNA to be used in the IVT reaction is pre-determined, and the concentration of each of the different DNA molecules therein is determined as a relative fraction of that total concentration.

"RNA fraction" is the fraction of the total RNA molecules in the composition that comprises the RNA molecule that is obtainable from the DNA molecule *X*, i.e. the DNA molecule for which the concentration is being calculated. Thus, the user can predetermine the fraction that each RNA molecule will represent in the composition that is made following the IVT reaction, and therefore the ratio of RNA molecules in the composition that is made. As an example, there can be considered a reaction mixture comprising three different DNA molecules DNA1, DNA2 and DNA3, which encode three different RNA molecules RNA1, RNA2 and RNA3 respectively. If it is desired to produce a composition comprising a 1:1:1 ratio of RNA1:RNA2:RNA3, the "RNA fraction" when calculating the concentration of DNA1 (or DNA2 or DNA3) that is needed will be one third (0.33). Alternatively, if it is desired to produce a composition comprising a 2:1:1 ratio of RNA1:RNA2:RNA3, the "RNA fraction" when calculating the concentration of DNA1 that is will be one half (0.5). In this latter circumstance, the "RNA fraction" when calculating the concentration of DNA2 or DNA3 that is needed will be one quarter (0.25). Accordingly, in general, the RNA fractions for each DNA/corresponding RNA molecule will add up to 1. In a typical embodiment, each RNA molecule will only be obtainable from one corresponding DNA molecule. However, in an

embodiment where an RNA molecule is obtainable from more than one different DNA molecule in the reaction mixture, the "RNA fraction" for determining the concentration of each DNA molecule from which that RNA molecule can be obtained would be further divided by the total number of different DNA molecules from which that RNA molecule can be obtained.

"$DNA_x$ length fraction" is the base pair (bp) length of the DNA molecule *X* for which the concentration is being determined, divided by the total bp length of all of the different DNA molecules. Thus, the "$DNA_x$ length fraction" is the fraction of the total DNA length which is made up by the DNA molecule for which the concentration is being determined. For example, in a reaction mixture that will comprise three different DNA molecules, DNA1 of 1000bp in length, DNA2 of 1000bp in length and DNA3 of 1000bp in length, the "$DNA_x$ length fraction" for determining the concentration of DNA1 (or DNA2 or DNA3) that is needed will be one third (0.33). To give another example, in a reaction mixture that will comprise three different DNA molecules, DNA1 of 2000bp in length, DNA2 of 1000bp in length and DNA3 of 1000bp in length, the "$DNA_x$ length fraction" for determining the concentration of DNA1 that is needed will be one half (0.5). In this latter circumstance, the "$DNA_x$ length fraction" for determining the concentration of DNA2 or DNA3 that is needed will be one quarter (0.25). Accordingly, in general, the RNA fractions used with each DNA/corresponding RNA molecule will add up to 1.

"$RNA_x$ length fraction" is the bp length of the RNA molecule that is obtainable from the DNA molecule *X* for which the concentration is being determined, divided by the total bp length of all of the different RNA molecules. Thus, the "$RNA_x$ length fraction" is the fraction of the total RNA length which is made up by the RNA molecule that is obtainable from the DNA molecule *X* for which the concentration is being determined. For example, for a reaction mixture that will comprise three different DNA molecules, DNA1 from which RNA1 of 1000bp in length is obtainable, DNA2 from which RNA2 of 1000bp in length is obtainable and DNA3 from which RNA3 of 1000bp in length is obtainable, the "$RNA_x$ length fraction" for determining the concentration of DNA1 (or DNA2 or DNA3) that is needed will be one third (0.33). To give another example, for a reaction mixture that will comprise three different DNA molecules, DNA1 from which RNA1 of 2000bp in length is obtainable, DNA2 from which RNA2 of 1000bp in length is obtainable and DNA3 from which RNA3 of 1000bp in length is obtainable, the "$RNA_x$ length fraction" for determining the concentration of DNA1 that is needed will be one half (0.5). In this latter circumstance, the "$DNA_x$ length fraction" for determining the concentration of DNA2 or DNA3 that is needed will be one quarter (0.25). Accordingly, in general, the RNA fractions used with each DNA/corresponding RNA molecule will add up to 1.

In the context of the present invention, base pair length (bp length) is referred to as the length of the nucleic acids, a concept known to the skilled person which refers to the number of consecutive nucleobases that make up a given nucleic acid. This is essentially interchangeable with nucleotide length (nt length).

In step a), the different DNA molecules are then provided in a reaction mixture comprising the DNA molecules. In an embodiment, this involves adding each of the different DNA molecules to a reaction mixture. In an embodiment, the reaction mixture further comprises the substances needed for transcription to occur, such as RNA polymerase and free RNA nucleotide.

In an embodiment, step a) involves determining the concentration of each of the DNA molecules using equation (i), and then providing a reaction mixture comprising each of the DNA molecules at its respective determined concentration.

Thereafter, in step b) the different product RNA molecules corresponding to each of the DNA molecules in the reaction mixture are obtained from the DNA molecules in the reaction mixture. In embodiment, the RNA molecules are obtained by transcription. In an embodiment, each of the different RNA molecules is obtained as a fraction of the total RNA obtained, which is equivalent to the corresponding "RNA fraction" in formula (i). In an embodiment, each of the different RNA molecules is obtained as a fraction of the total RNA obtained, which is the same as the corresponding "RNA fraction" in formula (i) ± up to 1%, ± up to 2%, ± up to 3%, ± up to 4%, ± up to 5%, ± up to 6%, ± up to 7%, ± up to 8%, ± up to 9%, ± up to 10%, ± up to 11 %, ± up to 12%, ± up to 13%, ± up to 14%, ± up to 15%, ± up to 16%, ± up to 17%, ± up to 18%, ± up to 19%, or ± up to 20%.

For a given RNA, the "RNA fraction" in formula (i) is the pre-determined fraction of the total RNA that is aimed to be produced. However, the actual fraction of the total RNA that the given RNA represents (when produced in step b)) may vary from the predetermined value within a tolerance margin. In an embodiment, the tolerance margin of the obtained fraction is within ±5%, ±10%, ±15%, or ±20% of the pre-determined "RNA fraction" in formula (i) for each RNA. Moreover, for a given RNA, the "RNA fraction" in formula (i) also implies a pre-determined concentration of the given RNA, relative to the total RNA concentration. Thus, the actual concentration of the total RNA that the given RNA represents (when produced in step b)) may vary from the predetermined concentration according to the "RNA fraction" in formula (i), within a tolerance margin. In an embodiment, the tolerance margin of the obtained concentration is within ±5%, ±10%, ±15%, or ±20% of the pre-determined concentration for each RNA.

Accordingly, in an embodiment wherein the RNA fraction is 1/2 for a given RNA, the RNA is obtained at a concentration which is within ±5%, ±10%, ±15%, or ±20% of 1/2 of the total RNA concentration, e.g. ±0.025, ±0.05, ±0.075, or ±0.1 of 0.5 * the total RNA concentration, e.g. 0.475-0.525 * the total RNA concentration, 0.45-0.55 * the total RNA concentration, 0.425-0.575 * the total RNA concentration or 0.4-0.6 * the total RNA concentration. In an embodiment

wherein the RNA fraction is 1/3 for a given RNA, the RNA is obtained at a concentration which is within ±5%, ±10%, ±15%, or ±20% of 1/3 of the total RNA concentration. In an embodiment wherein the RNA fraction is 1/4 for a given RNA, the RNA is obtained at a concentration which is within ±5%, ±10%, ±15%, or ±20% of 1/4 of the total RNA concentration. In an embodiment wherein the RNA fraction is 1/5 for a given RNA, the RNA is obtained at a concentration which is within ±5%, ±10%, ±15%, or ±20% of 1/5 of the total RNA concentration. In an embodiment wherein the RNA fraction is 1/6 for a given RNA, the RNA is obtained at a concentration which is within ±5%, ±10%, ±15%, or ±20% of 1/6 of the total RNA concentration. In an embodiment wherein the RNA fraction is 5/6 for a given RNA, the RNA is obtained at a concentration which is within ±5%, ±10%, ±15%, or ±20% of 5/6 of the total RNA concentration. In an embodiment wherein the RNA fraction is 1/7 for a given RNA, the RNA is obtained at a concentration which is within ±5%, ±10%, ±15%, or ±20% of 1/7 of the total RNA concentration. In an embodiment wherein the RNA fraction is 2/7 for a given RNA, the RNA is obtained at a concentration which is within ±5%, ±10%, ±15%, or ±20% of 2/7 of the total RNA concentration. In an embodiment wherein the RNA fraction is 3/7 for a given RNA, the RNA is obtained at a concentration which is within ±5%, ±10%, ±15%, or ±20% of 3/7 of the total RNA concentration. In an embodiment wherein the RNA fraction is 4/7 for a given RNA, the RNA is obtained at a concentration which is within ±5%, ±10%, ±15%, or ±20% of 4/7 of the total RNA concentration. In an embodiment wherein the RNA fraction is 5/7 for a given RNA, the RNA is obtained at a concentration which is within ±5%, ±10%, ±15%, or ±20% of 5/7 of the total RNA concentration. In an embodiment wherein the RNA fraction is 6/7 for a given RNA, the RNA is obtained at a concentration which is within ±5%, ±10%, ±15%, or ±20% of 6/7 of the total RNA concentration. In an embodiment wherein the RNA fraction is 1/8 for a given RNA, the RNA is obtained at a concentration which is within ±5%, ±10%, ±15%, or ±20% of 1/8 of the total RNA concentration. In an embodiment wherein the RNA fraction is 3/8 for a given RNA, the RNA is obtained at a concentration which is within ±5%, ±10%, ±15%, or ±20% of 3/8 of the total RNA concentration. In an embodiment wherein the RNA fraction is 5/8 for a given RNA, the RNA is obtained at a concentration which is within ±5%, ±10%, ±15%, or ±20% of 5/8 of the total RNA concentration. In an embodiment wherein the RNA fraction is 6/8 for a given RNA, the RNA is obtained at a concentration which is within ±5%, ±10%, ±15%, or ±20% of 6/8 of the total RNA concentration. In an embodiment wherein the RNA fraction is 7/8 for a given RNA, the RNA is obtained at a concentration which is within ±5%, ±10%, ±15%, or ±20% of 7/8 of the total RNA concentration. In an embodiment wherein the RNA fraction is 1/9 for a given RNA, the RNA is obtained at a concentration which is within ±5%, ±10%, ±15%, or ±20% of 1/9 of the total RNA concentration. In an embodiment wherein the RNA fraction is 2/9 for a given RNA, the RNA is obtained at a concentration which is within ±5%, ±10%, ±15%, or ±20% of 2/9 of the total RNA concentration. In an embodiment wherein the RNA fraction is 4/9 for a given RNA, the RNA is obtained at a concentration which is within ±5%, ±10%, ±15%, or ±20% of 4/9 of the total RNA concentration. In an embodiment wherein the RNA fraction is 5/9 for a given RNA, the RNA is obtained at a concentration which is within ±5%, ±10%, ±15%, or ±20% of 5/9 of the total RNA concentration. In an embodiment wherein the RNA fraction is 6/9 for a given RNA, the RNA is obtained at a concentration which is within ±5%, ±10%, ±15%, or ±20% of 6/9 of the total RNA concentration. In an embodiment wherein the RNA fraction is 7/9 for a given RNA, the RNA is obtained at a concentration which is within ±5%, ±10%, ±15%, or ±20% of 7/9 of the total RNA concentration. In an embodiment wherein the RNA fraction is 8/9 for a given RNA, the RNA is obtained at a concentration which is within ±5%, ±10%, ±15%, or ±20% of 8/9 of the total RNA concentration. In an embodiment wherein the RNA fraction is 1/10 for a given RNA, the RNA is obtained at a concentration which is within ±5%, ±10%, ±15%, or ±20% of 1/10 of the total RNA concentration. In an embodiment wherein the RNA fraction is 3/10 for a given RNA, the RNA is obtained at a concentration which is within ±5%, ±10%, ±15%, or ±20% of 3/10 of the total RNA concentration. In an embodiment wherein the RNA fraction is 4/10 for a given RNA, the RNA is obtained at a concentration which is within ±5%, ±10%, ±15%, or ±20% of 4/10 of the total RNA concentration. In an embodiment wherein the RNA fraction is 6/10 for a given RNA, the RNA is obtained at a concentration which is within ±5%, ±10%, ±15%, or ±20% of 6/10 of the total RNA concentration. In an embodiment wherein the RNA fraction is 7/10 for a given RNA, the RNA is obtained at a concentration which is within ±5%, ±10%, ±15%, or ±20% of 7/10 of the total RNA concentration. In an embodiment wherein the RNA fraction is 8/10 for a given RNA, the RNA is obtained at a concentration which is within ±5%, ±10%, ±15%, or ±20% of 8/10 of the total RNA concentration. In an embodiment wherein the RNA fraction is 9/10 for a given RNA, the RNA is obtained at a concentration which is within ±5%, ±10%, ±15%, or ±20% of 9/10 of the total RNA concentration. In this respect, it will be understood that the fraction in question readily corresponds to the ratio of the different RNA molecules in the composition, e.g. a ratio of 1:1:2 corresponds to fractions of 1/4, 1/4 and 1/2 respectively.

In an embodiment, each of the different RNA molecules is obtained as a fraction of the total RNA obtained, which is essentially the same as the corresponding "RNA fraction" in formula (i). It is to be understood that the "corresponding" "RNA fraction" in formula (i) for any given RNA molecule is the "RNA fraction" in formula (i) that was used to determine the initial concentration of the DNA molecule from which the given RNA molecule was obtained.

In an embodiment, the concentrations of each of the RNA molecules obtained in step b) vary only within the pharmaceutically acceptable limits for the composition, i.e. the composition that is produced.

In an embodiment, the concentration of any given RNA molecule obtained in step b) varies only within 80-120% of the mean concentration of all of the RNA molecules. This embodiment may apply when the "RNA fraction" in formula (i) for

each different RNA molecule is the same. In an embodiment, the concentration of any given RNA molecule obtained in step b) varies only within 80-120% of the total concentration of all of the RNA molecules, multiplied by the corresponding "RNA fraction" in formula (i) for that given RNA molecule. This embodiment may apply when the "RNA fraction" in formula (i) for each different RNA molecule is different or the same.

In an embodiment, the composition comprises at least 2, 3, 4, 5, 6, 7, 8, 9 or 10 different RNA molecules.

In an embodiment, the RNA molecules are obtained from the DNA molecules by transcription.

In an embodiment, one or more of the DNA molecules comprises a PCR template, or each of the DNA molecules comprises a PCR template.

In an embodiment, one or more of the DNA molecules comprises a linearised plasmid, or each of the DNA molecules comprises a linearised plasmid.

In an embodiment, at least one of the DNA molecules comprises a PCR template, and at least one of the DNA molecules comprises a linearised plasmid.

Each RNA molecule may encode one or more different polypeptide. In an embodiment, each of the RNA molecules encodes at least 1, 2, 3, 4, 5, 6, 7, 8, 9 or 10 polypeptides. In an embodiment, each of the polypeptides encoded by the same RNA molecule are the same. In an embodiment, each of the polypeptides encoded by the same RNA molecule are different. In an embodiment, the polypeptides are effector polypeptides. In an embodiment, the polypeptides are antigens.

In an embodiment, in step a), the total concentration of all of the DNA molecules in the reaction mixture is predetermined, preferably wherein the total concentration of the DNA molecules is at least 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 2.0, 3.0, 4.0, 5.0, 6.0, 7.0, 8.0, 9.0 or 10 mg/mL. In an embodiment, the total concentration of DNA molecules is 0.05 mg/mL multiplied by the number of different DNA molecules. Thus, in a particular embodiment where there are two different DNA molecules, the total DNA concentration is 0.10 mg/mL. In a particular embodiment where there are three different DNA molecules, the total DNA concentration is 0.15 mg/mL. In a particular embodiment where there are four different DNA molecules, the total DNA concentration is 0.20 mg/mL. In a particular embodiment where there are five different DNA molecules, the total DNA concentration is 0.25 mg/mL. In a particular embodiment where there are six different DNA molecules, the total DNA concentration is 0.30 mg/mL. In a particular embodiment where there are seven different DNA molecules, the total DNA concentration is 0.35 mg/mL. In a particular embodiment where there are eight different DNA molecules, the total DNA concentration is 0.40 mg/mL. In a particular embodiment where there are nine different DNA molecules, the total DNA concentration is 0.45 mg/mL. In a particular embodiment where there are ten different DNA molecules, the total DNA concentration is 0.50 mg/mL. Any total DNA concentration in these embodiments may vary by ± 5%.

In an embodiment, the method of the present invention further comprises:

c) determining the amounts of each of the RNA molecules in the composition.

In an embodiment, determining the amounts of each of the RNA molecules comprises determining the concentration, preferably the mass concentration thereof.

In an embodiment, the method of the present invention further comprises determining that the concentrations of one or more RNA molecules are underrepresented or overrepresented in the composition, and adjusting the concentration of the corresponding DNA molecules by a correction factor accordingly. In an embodiment, the concentration of a RNA molecule is considered underrepresented when it is less than the pharmaceutically acceptable or most pharmaceutically preferable concentration. In an embodiment, the concentration of a RNA molecule is considered overrepresented when it is greater than the pharmaceutically acceptable or most pharmaceutically preferable concentration. In an embodiment, the concentration of a given RNA molecule is considered underrepresented when the fraction of the total RNA that the given RNA molecule represents is less than the corresponding "RNA fraction" in formula (i), such as by more than 1%, 2%, 3%, 4%, 5%, 6%, 7%, 8%, 9%, 10%, 11%, 12%, 13%, 14%, 15%, 16%, 17%, 18%, 19%, 20% of the corresponding "RNA fraction" in formula (i). In an embodiment, the concentration of a given RNA molecule is considered overrepresented when the fraction of the total RNA that the given RNA molecule represents is more than the corresponding "RNA fraction" in formula (i), such as by more than 1%, 2%, 3%, 4%, 5%, 6%, 7%, 8%, 9%, 10%, 11%, 12%, 13%, 14%, 15%, 16%, 17%, 18%, 19%, 20% of the corresponding "RNA fraction" in formula (i).

In an embodiment, the method of the present invention further comprises determining that the concentrations of one or more RNA molecules are underrepresented in the composition, and increasing the concentration of the corresponding DNA molecules by a correction factor accordingly. In an embodiment, increasing the concentration by a correction factor comprises multiplying the initial DNA concentration by a value that is greater than 1. In an embodiment, the method of the present invention further comprises determining that the concentrations of one or more RNA molecules are overrepresented in the composition, and decreasing the concentration of the corresponding DNA molecules by a correction factor accordingly. In an embodiment, decreasing the concentration by a correction factor comprises multiplying the initial DNA concentration by a value that is less than 1 and greater than 0.

The concentrations of the RNA molecules may be determined by any means known to the skilled person. In an embodiment, the concentrations of the RNA molecules are determined by UV spectroscopy, e.g. with a NanoDrop

spectrophotometer. In an embodiment, the concentrations of the RNA molecules are determined with fluorescent dye-based quantification. In an embodiment, the concentrations of the RNA molecules are determined by gel electrophoresis and analysis of the staining intensity. In an embodiment, such methods are used to determine the concentrations of each of the different RNA molecules.

Herein, references to ATP, CTP, GTP and TTP concentrations are molar concentrations, or moles per unit volume.

In an embodiment, the reaction mixture comprises an ATP concentration of at least 6mM, preferably at least 9 mM, preferably at least 10 mM, preferably at least 11 mM, preferably at least 12 mM, preferably at least 13 mM, preferably at least 14 mM. In an embodiment of this type, one or more or all of the RNA molecules obtainable from the DNA molecules in the reaction mixture have high A fractions, such as A fractions that are greater than 25%.

In an embodiment, the reaction mixture comprises a CTP concentration of at least 6mM, preferably at least 9 mM, preferably at least 10 mM, preferably at least 11 mM, preferably at least 12 mM, preferably at least 13 mM, preferably at least 14 mM. In an embodiment of this type, one or more or all of the RNA molecules obtainable from the DNA molecules in the reaction mixture have high C fractions, such as C fractions that are greater than 25%.

In an embodiment, the reaction mixture comprises a GTP concentration of at least 6mM, preferably at least 9 mM, preferably at least 10 mM, preferably at least 11 mM, preferably at least 12 mM, preferably at least 13 mM, preferably at least 14 mM. In an embodiment of this type, one or more or all of the RNA molecules obtainable from the DNA molecules in the reaction mixture have high T fractions, such as T fractions that are greater than 25%.

In an embodiment, the reaction mixture comprises a TTP concentration of at least 6mM, preferably at least 9 mM, preferably at least 10 mM, preferably at least 11 mM, preferably at least 12 mM, preferably at least 13 mM, preferably at least 14 mM. In an embodiment of this type, one or more or all of the RNA molecules obtainable from the DNA molecules in the reaction mixture have high T fractions, such as T fractions that are greater than 25%.

In an embodiment, the reaction mixture comprises a CTP and ATP concentration of at least 6mM each, preferably at least 9 mM each, preferably at least 10 mM each, preferably at least 11 mM each, preferably at least 12 mM each, preferably at least 13 mM each, preferably at least 14 mM each. In an embodiment of this type, the RNA molecules have high C and A fractions, such as C and A fractions that are greater than 25%.

In an embodiment, the reaction mixture comprises a GTP and ATP concentration of at least 6mM each, preferably at least 9 mM each, preferably at least 10 mM each, preferably at least 11 mM each, preferably at least 12 mM each, preferably at least 13 mM each, preferably at least 14 mM each. In an embodiment of this type, one or more or all of the RNA molecules obtainable from the DNA molecules in the reaction mixture have high G and A fractions, such as G and A fractions that are greater than 25%.

In an embodiment, the reaction mixture comprises a TTP and ATP concentration of at least 6mM each, preferably at least 9 mM each, preferably at least 10 mM each, preferably at least 11 mM each, preferably at least 12 mM each, preferably at least 13 mM each, preferably at least 14 mM each. In an embodiment of this type, one or more or all of the RNA molecules obtainable from the DNA molecules in the reaction mixture have high T and A fractions, such as T and A fractions that are greater than 25%.

In an embodiment, the reaction mixture comprises a GTP and CTP concentration of at least 6mM each, preferably at least 9 mM each, preferably at least 10 mM each, preferably at least 11 mM each, preferably at least 12 mM each, preferably at least 13 mM each, preferably at least 14 mM each. In an embodiment of this type, one or more or all of the RNA molecules obtainable from the DNA molecules in the reaction mixture have high G and C fractions, such as G and C fractions that are greater than 25%.

In an embodiment, the reaction mixture comprises a TTP and CTP concentration of at least 6mM each, preferably at least 9 mM each, preferably at least 10 mM each, preferably at least 11 mM each, preferably at least 12 mM each, preferably at least 13 mM each, preferably at least 14 mM each. In an embodiment of this type, one or more or all of the RNA molecules obtainable from the DNA molecules in the reaction mixture have high C and A fractions, such as T and C fractions that are greater than 25%.

In an embodiment, the reaction mixture comprises a TTP and GTP concentration of at least 6mM each, preferably at least 9 mM each, preferably at least 10 mM each, preferably at least 11 mM each, preferably at least 12 mM each, preferably at least 13 mM each, preferably at least 14 mM each. In an embodiment of this type, one or more or all of the RNA molecules obtainable from the DNA molecules in the reaction mixture have high T and G fractions, such as T and G fractions that are greater than 25%.

Herein, A/C/G/T fractions refer to the percentage of the nucleobases within a nucleic acid molecule that are A/C/G/T bases respectively. A modified base is still considered a base of that type, for example a methyl-C base is still considered a C.

In an embodiment, step b) is carried out for at least 105 minutes, at least 180 minutes, or between 105 minutes and 180 minutes inclusive.

**Specific embodiments and applications**

The present invention is particularly useful for providing pre-determined ratios of RNA molecules in the composition that is produced.

In an embodiment, the present invention is for the production of a composition comprising two RNA molecules, "RNA1" and "RNA2". In an embodiment, method is for the production of a composition comprising an RNA1:RNA2 ratio of 1:1, 1:2, 1:3, 1:4, 1:5, 1:6, 1:7, 1:8, 1:9 or 1:10.

In an embodiment, present invention is for the production of a composition comprising three RNA molecules, "RNA1", "RNA2" and "RNA3". In an embodiment, method is for the production of a composition comprising an RNA1: RNA2: RNA3 ratio of 1:1:1, 1:1:2, 1:1:3, 1:1:4, 1:1:5, 1:1:6, 1:1:7, 1:1:8, 1:1:9, 1:1:10, 1:2:1, 1:2:2, 1:2:3, 1:2:4, 1:2:5, 1:2:6, 1:2:7, 1:2:8, 1:2:9, 1:2:10, 1:3:1, 1:3:2, 1:3:3, 1:3:4, 1:3:5, 1:3:6, 1:3:7, 1:3:8, 1:3:9, 1:3:10, 1:4:1, 1:4:2, 1:4:3, 1:4:4, 1:4:5, 1:4:6, 1:4:7, 1:4:8, 1:4:9, 1:4:10, 1:5:1, 1:5:2, 1:5:3, 1:5:4, 1:5:5, 1:5:6, 1:5:7, 1:5:8, 1:5:9, 1:5:10, 1:6:1, 1:6:2, 1:6:3, 1:6:4, 1:6:5, 1:6:6, 1:6:7, 1:6:8, 1:6:9, 1:6:10, 1:7:1, 1:7:2, 1:7:3, 1:7:4, 1:7:5, 1:7:6, 1:7:7, 1:7:8, 1:7:9, 1:7:10, 1:8:1, 1:8:2, 1:8:3, 1:8:4, 1:8:5, 1:8:6, 1:8:7, 1:8:8, 1:8:9, 1:8:10, 1:9:1, 1:9:2, 1:9:3, 1:9:4, 1:9:5, 1:9:6, 1:9:7, 1:9:8, 1:9:9, 1:9:10, 1:10:1, 1:10:2,1:10:3, 1:10:4, 1:10:5, 1:10:6, 1:10:7, 1:10:8, 1:10:9 or 1:10:10.

In an embodiment, the present invention is for the production of a composition comprising four RNA molecules, "RNA1", "RNA2", "RNA3" and "RNA4". In an embodiment, method is for the production of a composition comprising an RNA1:RNA2:RNA3:RNA4 ratio of 1:1:1:1, 1:1:1:2, 1:1:1:3, 1:1:1:4, 1:1:1:5, 1:1:1:6, 1:1:1:7, 1:1:1:8, 1:1:1:9, 1:1:1:10, 1:1:2:1, 1:1:2:2, 1:1:2:3, 1:1:2:4, 1:1:2:5, 1:1:2:6, 1:1:2:7, 1:1:2:8, 1:1:2:9, 1:1:2:10, 1:1:3:1, 1:1:3:2, 1:1:3:3, 1:1:3:4, 1:1:3:5, 1:1:3:6, 1:1:3:7, 1:1:3:8, 1:1:3:9, 1:1:3:10, 1:1:4:1, 1:1:4:2, 1:1:4:3, 1:1:4:4, 1:1:4:5, 1:1:4:6, 1:1:4:7, 1:1:4:8, 1:1:4:9, 1:1:4:10, 1:1:5:1, 1:1:5:2, 1:1:5:3, 1:1:5:4, 1:1:5:5, 1:1:5:6, 1:1:5:7, 1:1:5:8, 1:1:5:9, 1:1:5:10, 1:1:6:1, 1:1:6:2, 1:1:6:3, 1:1:6:4, 1:1:6:5, 1:1:6:6, 1:1:6:7, 1:1:6:8, 1:1:6:9, 1:1:6:10, 1:1:7:1, 1:1:7:2, 1:1:7:3, 1:1:7:4, 1:1:7:5, 1:1:7:6, 1:1:7:7, 1:1:7:8, 1:1:7:9, 1:1:7:10, 1:1:8:1, 1:1:8:2, 1:1:8:3, 1:1:8:4, 1:1:8:5, 1:1:8:6, 1:1:8:7, 1:1:8:8, 1:1:8:9, 1:1:8:10, 1:1:9:1, 1:1:9:2, 1:1:9:3, 1:1:9:4, 1:1:9:5, 1:1:9:6, 1:1:9:7, 1:1:9:8, 1:1:9:9, 1:1:9:10, 1:1:10:1, 1:1:10:2, 1:1:10:3, 1:1:10:4, 1:1:10:5, 1:1:10:6, 1:1:10:7, 1:1:10:8, 1:1:10:9, 1:1:10:10, 1:2:1:1, 1:2:1:2, 1:2:1:3, 1:2:1:4, 1:2:1:5, 1:2:1:6, 1:2:1:7, 1:2:1:8, 1:2:1:9, 1:2:1:10, 1:2:2:1, 1:2:2:2, 1:2:2:3, 1:2:2:4, 1:2:2:5, 1:2:2:6, 1:2:2:7, 1:2:2:8, 1:2:2:9, 1:2:2:10, 1:2:3:1, 1:2:3:2, 1:2:3:3, 1:2:3:4, 1:2:3:5, 1:2:3:6, 1:2:3:7, 1:2:3:8, 1:2:3:9, 1:2:3:10, 1:2:4:1, 1:2:4:2, 1:2:4:3, 1:2:4:4, 1:2:4:5, 1:2:4:6, 1:2:4:7, 1:2:4:8, 1:2:4:9, 1:2:4:10, 1:2:5:1, 1:2:5:2, 1:2:5:3, 1:2:5:4, 1:2:5:5, 1:2:5:6, 1:2:5:7, 1:2:5:8, 1:2:5:9, 1:2:5:10, 1:2:6:1, 1:2:6:2, 1:2:6:3, 1:2:6:4, 1:2:6:5, 1:2:6:6, 1:2:6:7, 1:2:6:8, 1:2:6:9, 1:2:6:10, 1:2:7:1, 1:2:7:2, 1:2:7:3, 1:2:7:4, 1:2:7:5, 1:2:7:6, 1:2:7:7, 1:2:7:8, 1:2:7:9, 1:2:7:10, 1:2:8:1, 1:2:8:2, 1:2:8:3, 1:2:8:4, 1:2:8:5, 1:2:8:6, 1:2:8:7, 1:2:8:8, 1:2:8:9, 1:2:8:10, 1:2:9:1, 1:2:9:2, 1:2:9:3, 1:2:9:4, 1:2:9:5, 1:2:9:6, 1:2:9:7, 1:2:9:8, 1:2:9:9, 1:2:9:10, 1:2:10:1, 1:2:10:2, 1:2:10:3, 1:2:10:4, 1:2:10:5, 1:2:10:6, 1:2:10:7, 1:2:10:8, 1:2:10:9, 1:2:10:10, 1:3:1:1, 1:3:1:2, 1:3:1:3, 1:3:1:4, 1:3:1:5, 1:3:1:6, 1:3:1:7, 1:3:1:8, 1:3:1:9, 1:3:1:10, 1:3:2:1, 1:3:2:2, 1:3:2:3, 1:3:2:4, 1:3:2:5, 1:3:2:6, 1:3:2:7, 1:3:2:8, 1:3:2:9, 1:3:2:10, 1:3:3:1, 1:3:3:2, 1:3:3:3, 1:3:3:4, 1:3:3:5, 1:3:3:6, 1:3:3:7, 1:3:3:8, 1:3:3:9, 1:3:3:10, 1:3:4:1, 1:3:4:2, 1:3:4:3, 1:3:4:4, 1:3:4:5, 1:3:4:6, 1:3:4:7, 1:3:4:8, 1:3:4:9, 1:3:4:10, 1:3:5:1, 1:3:5:2, 1:3:5:3, 1:3:5:4, 1:3:5:5, 1:3:5:6, 1:3:5:7, 1:3:5:8, 1:3:5:9, 1:3:5:10, 1:3:6:1, 1:3:6:2, 1:3:6:3, 1:3:6:4, 1:3:6:5, 1:3:6:6, 1:3:6:7, 1:3:6:8, 1:3:6:9, 1:3:6:10, 1:3:7:1, 1:3:7:2, 1:3:7:3, 1:3:7:4, 1:3:7:5, 1:3:7:6, 1:3:7:7, 1:3:7:8, 1:3:7:9, 1:3:7:10, 1:3:8:1, 1:3:8:2, 1:3:8:3, 1:3:8:4, 1:3:8:5, 1:3:8:6, 1:3:8:7, 1:3:8:8, 1:3:8:9, 1:3:8:10, 1:3:9:1, 1:3:9:2, 1 :3:9:3, 1 :3:9:4, 1 :3:9:5, 1 :3:9:6, 1 :3:9:7, 1 :3:9:8, 1 :3:9:9, 1 :3:9: 1 0, 1 :3: 1 0: 1, 1 :3: 1 0:2, 1:3:10:3, 1:3:10:4, 1:3:10:5, 1:3:10:6, 1:3:10:7, 1:3:10:8, 1:3:10:9, 1:3:10:10, 1:4:1:1, 1:4:1:2, 1:4:1:3, 1:4:1:4, 1:4:1:5, 1:4:1:6, 1:4:1:7, 1:4:1:8, 1:4:1:9, 1:4:1:10, 1:4:2:1, 1:4:2:2, 1:4:2:3, 1:4:2:4, 1:4:2:5, 1:4:2:6, 1:4:2:7, 1:4:2:8, 1:4:2:9, 1:4:2:10, 1:4:3:1, 1:4:3:2, 1:4:3:3, 1:4:3:4, 1:4:3:5, 1:4:3:6, 1:4:3:7, 1:4:3:8, 1:4:3:9, 1:4:3:10, 1:4:4:1, 1:4:4:2, 1:4:4:3, 1:4:4:4, 1:4:4:5, 1:4:4:6, 1:4:4:7, 1:4:4:8, 1:4:4:9, 1:4:4:10, 1:4:5:1, 1:4:5:2, 1:4:5:3, 1:4:5:4, 1:4:5:5, 1:4:5:6, 1:4:5:7, 1:4:5:8, 1:4:5:9, 1:4:5:10, 1:4:6:1, 1:4:6:2, 1:4:6:3, 1:4:6:4, 1:4:6:5, 1:4:6:6, 1:4:6:7, 1:4:6:8, 1:4:6:9, 1:4:6:10, 1:4:7:1, 1:4:7:2, 1:4:7:3, 1:4:7:4, 1:4:7:5, 1:4:7:6, 1:4:7:7, 1:4:7:8, 1:4:7:9, 1:4:7:10, 1:4:8:1, 1:4:8:2, 1:4:8:3, 1:4:8:4, 1:4:8:5, 1:4:8:6, 1:4:8:7, 1:4:8:8, 1:4:8:9, 1:4:8:10, 1:4:9:1, 1:4:9:2, 1:4:9:3, 1:4:9:4, 1:4:9:5, 1:4:9:6, 1:4:9:7, 1:4:9:8, 1:4:9:9, 1:4:9:10, 1:4:10:1, 1:4:10:2, 1:4:10:3, 1:4:10:4, 1:4:10:5, 1:4:10:6, 1:4:10:7, 1:4:10:8, 1:4:10:9, 1:4:10:10, 1:5:1:1, 1:5:1:2, 1:5:1:3, 1:5:1:4, 1:5:1:5, 1:5:1:6, 1:5:1:7, 1:5:1:8, 1:5:1:9, 1:5:1:10, 1:5:2:1, 1:5:2:2, 1:5:2:3, 1:5:2:4, 1:5:2:5, 1:5:2:6, 1:5:2:7, 1:5:2:8, 1:5:2:9, 1:5:2:10, 1:5:3:1, 1:5:3:2, 1:5:3:3, 1:5:3:4, 1:5:3:5, 1:5:3:6, 1:5:3:7, 1:5:3:8, 1:5:3:9, 1:5:3:10, 1:5:4:1, 1:5:4:2, 1:5:4:3, 1:5:4:4, 1:5:4:5, 1:5:4:6, 1:5:4:7, 1:5:4:8, 1:5:4:9, 1:5:4:10, 1:5:5:1, 1:5:5:2, 1:5:5:3, 1:5:5:4, 1:5:5:5, 1:5:5:6, 1:5:5:7, 1:5:5:8, 1:5:5:9, 1:5:5:10, 1:5:6:1, 1:5:6:2, 1:5:6:3, 1:5:6:4, 1:5:6:5, 1:5:6:6, 1:5:6:7, 1:5:6:8, 1:5:6:9, 1:5:6:10, 1:5:7:1, 1:5:7:2, 1:5:7:3, 1:5:7:4, 1:5:7:5, 1:5:7:6, 1:5:7:7, 1:5:7:8, 1:5:7:9, 1:5:7:10, 1:5:8:1, 1:5:8:2, 1:5:8:3, 1:5:8:4, 1:5:8:5, 1:5:8:6, 1:5:8:7, 1:5:8:8, 1:5:8:9, 1:5:8:10, 1:5:9:1, 1:5:9:2, 1:5:9:3, 1:5:9:4, 1:5:9:5, 1:5:9:6, 1:5:9:7, 1:5:9:8, 1:5:9:9, 1:5:9:10, 1:5:10:1, 1:5:10:2, 1:5:10:3, 1:5:10:4, 1:5:10:5, 1:5:10:6, 1:5:10:7, 1:5:10:8, 1:5:10:9, 1:5:10:10, 1:6:1:1, 1:6:1:2, 1:6:1:3, 1:6:1:4, 1:6:1:5, 1:6:1:6, 1:6:1:7, 1:6:1:8, 1:6:1:9, 1:6:1:10, 1:6:2:1, 1:6:2:2, 1:6:2:3, 1:6:2:4, 1:6:2:5, 1:6:2:6, 1:6:2:7, 1:6:2:8, 1:6:2:9, 1:6:2:10, 1:6:3:1, 1:6:3:2, 1:6:3:3, 1:6:3:4, 1:6:3:5, 1:6:3:6, 1:6:3:7, 1:6:3:8, 1:6:3:9, 1:6:3:10, 1:6:4:1, 1:6:4:2, 1:6:4:3, 1:6:4:4, 1:6:4:5, 1:6:4:6, 1:6:4:7, 1:6:4:8, 1:6:4:9, 1:6:4:10, 1:6:5:1, 1:6:5:2, 1:6:5:3, 1:6:5:4, 1:6:5:5, 1:6:5:6, 1:6:5:7, 1:6:5:8, 1:6:5:9, 1:6:5:10, 1:6:6:1, 1:6:6:2, 1:6:6:3, 1:6:6:4, 1:6:6:5, 1:6:6:6, 1:6:6:7, 1:6:6:8, 1:6:6:9, 1:6:6:10, 1:6:7:1, 1:6:7:2, 1:6:7:3, 1:6:7:4, 1:6:7:5,

1:6:7:6, 1:6:7:7, 1:6:7:8, 1:6:7:9, 1:6:7:10, 1:6:8:1, 1:6:8:2, 1:6:8:3, 1:6:8:4, 1:6:8:5, 1:6:8:6, 1:6:8:7, 1:6:8:8, 1:6:8:9, 1:6:8:10, 1:6:9:1, 1:6:9:2, 1:6:9:3, 1:6:9:4, 1:6:9:5, 1:6:9:6, 1:6:9:7, 1:6:9:8, 1:6:9:9, 1:6:9:10, 1:6:10:1, 1:6:10:2, 1:6:10:3, 1:6:10:4, 1:6:10:5, 1:6:10:6, 1:6:10:7, 1:6:10:8, 1:6:10:9, 1:6:10:10, 1:7:1:1, 1:7:1:2, 1:7:1:3, 1:7:1:4, 1:7:1:5, 1:7:1:6, 1:7:1:7, 1:7:1:8, 1:7:1:9, 1:7:1:10, 1:7:2:1, 1:7:2:2, 1:7:2:3, 1:7:2:4, 1:7:2:5, 1:7:2:6, 1:7:2:7, 1:7:2:8, 1:7:2:9, 1:7:2:10, 1:7:3:1, 1:7:3:2, 1:7:3:3, 1:7:3:4, 1:7:3:5, 1:7:3:6, 1:7:3:7, 1:7:3:8, 1:7:3:9, 1:7:3:10, 1:7:4:1, 1:7:4:2, 1:7:4:3, 1:7:4:4, 1:7:4:5, 1:7:4:6, 1:7:4:7, 1:7:4:8, 1:7:4:9, 1:7:4:10, 1:7:5:1, 1:7:5:2, 1:7:5:3, 1:7:5:4, 1:7:5:5, 1:7:5:6, 1:7:5:7, 1:7:5:8, 1:7:5:9, 1:7:5:10, 1:7:6:1, 1:7:6:2, 1:7:6:3, 1:7:6:4, 1:7:6:5, 1:7:6:6, 1:7:6:7, 1:7:6:8, 1:7:6:9, 1:7:6:10, 1:7:7:1, 1:7:7:2, 1:7:7:3, 1:7:7:4, 1:7:7:5, 1:7:7:6, 1:7:7:7, 1:7:7:8, 1:7:7:9, 1:7:7:10, 1:7:8:1, 1:7:8:2, 1:7:8:3, 1:7:8:4, 1:7:8:5, 1:7:8:6, 1:7:8:7, 1:7:8:8, 1:7:8:9, 1:7:8:10, 1:7:9:1, 1:7:9:2, 1:7:9:3, 1:7:9:4, 1:7:9:5, 1:7:9:6, 1:7:9:7, 1:7:9:8, 1:7:9:9, 1:7:9:10, 1:7:10:1, 1:7:10:2, 1:7:10:3, 1:7:10:4, 1:7:10:5, 1:7:10:6, 1:7:10:7, 1:7:10:8, 1:7:10:9, 1:7:10:10, 1:8:1:1, 1:8:1:2, 1:8:1:3, 1:8:1:4, 1:8:1:5, 1:8:1:6, 1:8:1:7, 1:8:1:8, 1:8:1:9, 1:8:1:10, 1:8:2:1, 1:8:2:2, 1:8:2:3, 1:8:2:4, 1:8:2:5, 1:8:2:6, 1:8:2:7, 1:8:2:8, 1:8:2:9, 1:8:2:10, 1:8:3:1, 1:8:3:2, 1:8:3:3, 1:8:3:4, 1:8:3:5, 1:8:3:6, 1:8:3:7, 1:8:3:8, 1:8:3:9, 1:8:3:10, 1:8:4:1, 1:8:4:2, 1:8:4:3, 1:8:4:4, 1:8:4:5, 1:8:4:6, 1:8:4:7, 1:8:4:8, 1:8:4:9, 1:8:4:10, 1:8:5:1, 1:8:5:2, 1:8:5:3, 1:8:5:4, 1:8:5:5, 1:8:5:6, 1:8:5:7, 1:8:5:8, 1:8:5:9, 1:8:5:10, 1:8:6:1, 1:8:6:2, 1:8:6:3, 1:8:6:4, 1:8:6:5, 1:8:6:6, 1:8:6:7, 1:8:6:8, 1:8:6:9, 1:8:6:10, 1:8:7:1, 1:8:7:2, 1:8:7:3, 1:8:7:4, 1:8:7:5, 1:8:7:6, 1:8:7:7, 1:8:7:8, 1:8:7:9, 1:8:7:10, 1:8:8:1, 1:8:8:2, 1:8:8:3, 1:8:8:4, 1:8:8:5, 1:8:8:6, 1:8:8:7, 1:8:8:8, 1:8:8:9, 1:8:8:10, 1:8:9:1, 1:8:9:2, 1:8:9:3, 1:8:9:4, 1:8:9:5, 1:8:9:6, 1:8:9:7, 1:8:9:8, 1:8:9:9, 1:8:9:10, 1:8:10:1, 1:8:10:2, 1:8:10:3, 1:8:10:4, 1:8:10:5, 1:8:10:6, 1:8:10:7, 1:8:10:8, 1:8:10:9, 1:8:10:10, 1:9:1:1, 1:9:1:2, 1:9:1:3, 1:9:1:4, 1:9:1:5, 1:9:1:6, 1:9:1:7, 1:9:1:8, 1:9:1:9, 1:9:1:10, 1:9:2:1, 1:9:2:2, 1:9:2:3, 1:9:2:4, 1:9:2:5, 1:9:2:6, 1:9:2:7, 1:9:2:8, 1:9:2:9, 1:9:2:10, 1:9:3:1, 1:9:3:2, 1:9:3:3, 1:9:3:4, 1:9:3:5, 1:9:3:6, 1:9:3:7, 1:9:3:8, 1:9:3:9, 1:9:3:10, 1:9:4:1, 1:9:4:2, 1:9:4:3, 1:9:4:4, 1:9:4:5, 1:9:4:6, 1:9:4:7, 1:9:4:8, 1:9:4:9, 1:9:4:10, 1:9:5:1, 1:9:5:2, 1:9:5:3, 1:9:5:4, 1:9:5:5, 1:9:5:6, 1:9:5:7, 1:9:5:8, 1:9:5:9, 1:9:5:10, 1:9:6:1, 1:9:6:2, 1:9:6:3, 1:9:6:4, 1:9:6:5, 1:9:6:6, 1:9:6:7, 1:9:6:8, 1:9:6:9, 1:9:6:10, 1:9:7:1, 1:9:7:2, 1:9:7:3, 1:9:7:4, 1:9:7:5, 1:9:7:6, 1:9:7:7, 1:9:7:8, 1:9:7:9, 1:9:7:10, 1:9:8:1, 1:9:8:2, 1:9:8:3, 1:9:8:4, 1:9:8:5, 1:9:8:6, 1:9:8:7, 1:9:8:8, 1:9:8:9, 1:9:8:10, 1:9:9:1, 1:9:9:2, 1:9:9:3, 1:9:9:4, 1:9:9:5, 1:9:9:6, 1:9:9:7, 1:9:9:8, 1:9:9:9, 1:9:9:10, 1:9:10:1, 1:9:10:2, 1:9:10:3, 1:9:10:4, 1:9:10:5, 1:9:10:6, 1:9:10:7, 1:9:10:8, 1:9:10:9, 1:9:10:10, 1:10:1:1, 1:10:1:2, 1:10:1:3, 1:10:1:4, 1:10:1:5, 1:10:1:6, 1:10:1:7, 1:10:1:8, 1:10:1:9, 1:10:1:10, 1:10:2:1, 1:10:2:2, 1:10:2:3, 1:10:2:4, 1:10:2:5, 1:10:2:6, 1:10:2:7, 1:10:2:8, 1:10:2:9, 1:10:2:10, 1:10:3:1, 1:10:3:2, 1:10:3:3, 1:10:3:4, 1:10:3:5, 1:10:3:6, 1:10:3:7, 1:10:3:8, 1:10:3:9, 1:10:3:10, 1:10:4:1, 1:10:4:2, 1:10:4:3, 1:10:4:4, 1:10:4:5, 1:10:4:6, 1:10:4:7, 1:10:4:8, 1:10:4:9, 1:10:4:10, 1:10:5:1, 1:10:5:2, 1:10:5:3, 1:10:5:4, 1:10:5:5, 1:10:5:6, 1:10:5:7, 1:10:5:8, 1:10:5:9, 1:10:5:10, 1:10:6:1, 1:10:6:2, 1:10:6:3, 1:10:6:4, 1:10:6:5, 1:10:6:6, 1:10:6:7, 1:10:6:8, 1:10:6:9, 1:10:6:10, 1:10:7:1, 1:10:7:2, 1:10:7:3, 1:10:7:4, 1:10:7:5, 1:10:7:6, 1:10:7:7, 1:10:7:8, 1:10:7:9, 1:10:7:10, 1:10:8:1, 1:10:8:2, 1:10:8:3, 1:10:8:4, 1:10:8:5, 1:10:8:6, 1:10:8:7, 1:10:8:8, 1:10:8:9, 1:10:8:10, 1:10:9:1, 1:10:9:2, 1:10:9:3, 1:10:9:4, 1:10:9:5, 1:10:9:6, 1:10:9:7, 1:10:9:8, 1:10:9:9, 1:10:9:10, 1:10:10:1, 1:10:10:2, 1:10:10:3, 1:10:10:4, 1:10:10:5, 1:10:10:6, 1:10:10:7, 1:10:10:8, 1:10:10:9 or 1:10:10:10.

In an embodiment where the present invention is particularly useful, different amounts of the at least two different RNA molecules are present in the composition that is produced. Accordingly, in an embodiment where the present invention is particularly useful, the ratio of the different RNA molecules produced is not a 1:1 ratio, a 1:1:1 ratio, a 1:1:1:1 ratio, a 1:1:1:1:1 ratio, a 1:1:1:1:1:1 ratio, a 1:1:1:1:1:1:1 ratio, a 1:1:1:1:1:1:1:1 ratio, a 1:1:1:1:1:1:1:1:1 ratio or a 1:1:1:1:1:1:1:1:1:1 ratio.

The present invention is also particularly useful for providing the desired compositions comprising two or more RNA molecules when the DNA molecules from which those RNA molecules are obtained have a large variance in length.

In an embodiment, at least two of the DNA molecules differ in bp length by more than 10%, 11%, 12%, 13%, 14%, 15%, 16%, 17%, 18%, 19%, 20%, 25%, 30%, 35%, 40%, 50%, 60%, 70%, 80%, 90%, 100%, 150%, 200%, 300%, or 400 %. In an embodiment, at least one of the DNA molecules is up to twice as long, three times longer or four times longer than at least one different DNA molecule.

As such, the present invention is also particularly useful for providing the desired compositions comprising two or more RNA molecules when the DNA molecules from which the RNA molecules are obtained comprise long non-coding sequences, and therefore inherently have a higher probability of variability in length.

In an embodiment, one or more or all of the DNA molecules comprises at least 1000, 1100, 1200, 1300, 1400, 1500, 1600, 1700, 1800, 1900, 2000, 2100, 2200, 2300, 2400, 2500, 2600, 2700, 2800, 2900 or 3000 bp of non-coding sequences. In an embodiment, 20, 30, 40, 50, 60, 70, 80, or 90% of the total length of one or more or all of the DNA molecules comprises non-coding sequence(s).

The present invention is also particularly useful for providing the desired compositions comprising two or more RNA molecules when the RNA molecules have a large variance in length.

In an embodiment, at least two of the RNA molecules differ in bp length by more than 10%, 11%, 12%, 13%, 14%, 15%, 16%, 17%, 18%, 19%, 20%, 25%, 30%, 35%, 40%, 50%, 60%, 70%, 80%, 90%, or 100%. In an embodiment, at least one of the RNA molecules is up to twice as long, three times longer or four times longer than at least one different RNA molecule.

In an embodiment, the concentrations of at least two of the DNA templates used are not equal. In an embodiment,

the concentrations of at least two of the DNA templates used vary from the mean concentration of DNA template by more than 1%, 2%, 3%, 4%, 5%, 10%, 20%, 30%, 40%, 50%, 60%, 70%, 80%, 90% or 100%.

**Further aspects of the invention**

In a second aspect, the present invention provides a method of making a composition comprising at least a first RNA molecule and a second RNA molecule, wherein the first RNA molecule is obtainable from a first DNA molecule and the second RNA molecule is obtainable from a second DNA molecule, wherein the method comprises:

a) providing a reaction mixture comprising the first DNA molecule and the second DNA molecule; and

b) obtaining the first and second RNA molecules from the first and second DNA molecules respectively;

wherein the first RNA molecule has a greater relative mass than the second RNA molecule, and the second RNA molecule is obtained from the second DNA molecule in a molar amount that is greater than the molar amount of the first RNA molecule that is obtained from the first DNA molecule, such that the first and second RNA molecules are obtained at essentially the same concentrations in the composition.

In an alternative to the second aspect, the present invention provides a method of making a composition comprising at least a first RNA molecule and a second RNA molecule, wherein the first RNA molecule is obtainable from a first DNA molecule and the second RNA molecule is obtainable from a second DNA molecule, wherein the method comprises:

a) providing a reaction mixture comprising the first DNA molecule and the second DNA molecule; and

b) obtaining the first and second RNA molecules from the first and second DNA molecules respectively;

wherein the first RNA molecule has a different relative mass to the second RNA molecule, and the second RNA molecule is obtained from the second DNA molecule in a molar amount that is different to the molar amount of the first RNA molecule that is obtained from the first DNA molecule, such that the first and second RNA molecules are obtained in the composition at concentrations that vary by a pre-determined ratio as described herein.

In an alternative to the second aspect, the present invention provides a method of making a composition comprising at least a first RNA molecule and a second RNA molecule, wherein the first RNA molecule is obtainable from a first DNA molecule and the second RNA molecule is obtainable from a second DNA molecule, wherein the method comprises:

a) providing a reaction mixture comprising the first DNA molecule and the second DNA molecule; and

b) obtaining the first and second RNA molecules from the first and second DNA molecules respectively;

wherein the first DNA molecule has a greater relative mass than the second DNA molecule, and the first DNA molecule is present in the reaction mixture at a mass concentration that is greater than the mass concentration of the second DNA molecule, such that the molar amounts of the first and second DNA molecules are essentially the same, and the first and second RNA molecules are obtained at essentially the same molar amounts in the composition.

In an alternative to the second aspect, the present invention provides a method of making a composition comprising at least a first RNA molecule and a second RNA molecule, wherein the first RNA molecule is obtainable from a first DNA molecule and the second RNA molecule is obtainable from a second DNA molecule, wherein the method comprises:

a) providing a reaction mixture comprising the first DNA molecule and the second DNA molecule; and

b) obtaining the first and second RNA molecules from the first and second DNA molecules respectively;

wherein the first DNA molecule has a different relative mass to the second DNA molecule, and the first DNA molecule is present in the reaction mixture at a mass concentration that is different to the mass concentration of the second DNA molecule, such that the molar amounts of the first and second DNA molecules vary by a pre-determined ratio as described herein, and the first and second RNA molecules are obtained in the composition at molar amounts that vary by a pre-determined ratio as described herein.

"Greater relative mass" relates to the mass of the individual RNA molecule per se, which depends on *inter alia* its

composition and length.

In a third aspect, the present invention provides the composition obtained or obtainable by the methods of the present invention. In an embodiment, the composition is a pharmaceutical composition. In an embodiment, the composition comprises a pharmaceutically acceptable carried, adjuvant and/or diluent. In an embodiment, the composition is a vaccine composition. In an embodiment, the composition is a multivalent vaccine composition.

In a fourth aspect, the present invention provides the composition obtained or obtainable by the methods of the present invention, for use as a medicament.

In a fifth aspect, the present invention provides a composition comprising 2 or more different DNA molecules, wherein a different RNA molecule is obtainable from each of the different DNA molecules, wherein each of the different DNA molecules (*X*) has a concentration according to equation (i):

$$c(DNA_X) = \frac{c(DNA_{total}) \cdot RNA\ Fraction \cdot DNA_x\ length\ fraction}{RNA_x\ length\ fraction} \quad \text{(i)}$$

wherein:

"$c(DNA_x)$" is the concentration of a given DNA molecule *X*;
"$c(DNA_{total})$" is the total concentration of all of the DNA molecules;
the "RNA fraction" is the fraction of all RNA molecules obtainable from the reaction mixture that comprises the RNA molecule that is obtainable from the DNA molecule *X*;
the "$DNA_x$ length fraction" is the base pair (bp) length of the DNA molecule *X*, divided by the total bp length of all of the different DNA molecules;
the "$RNA_x$ length fraction" is the bp length of the RNA molecule obtainable from the DNA molecule *X*, divided by the total bp length of all of the different RNA molecules.

In an embodiment, the composition comprising two or more different DNA molecules is a reaction mixture composition.

## Other definitions and descriptions

### Definitions of General Terms

The practice of the present disclosure will employ, unless otherwise indicated, conventional chemistry, biochemistry, cell biology, immunology, and recombinant DNA techniques which are explained in the literature in the field.

Throughout this specification and the claims which follow, unless the context requires otherwise, the word "comprise", and variations such as "comprises" and "comprising", will be understood to imply the inclusion of a stated feature, element, member, integer or step or group of features, elements, members, integers or steps but not the exclusion of any other feature, element, member, integer or step or group of features, elements, members, integers or steps. The term "consisting essentially of" limits the scope of a claim or disclosure to the specified features, elements, members, integers, or steps and those that do not materially affect the basic and novel characteristic(s) of the claim or disclosure. The term "consisting of" limits the scope of a claim or disclosure to the specified features, elements, members, integers, or steps. The term "comprising" encompasses the term "consisting essentially of" which, in turn, encompasses the term "consisting of". Thus, at each occurrence in the present application, the term "comprising" may be replaced with the term "consisting essentially of" or "consisting of". Likewise, at each occurrence in the present application, the term "consisting essentially of" may be replaced with the term "consisting of".

The terms "a", "an" and "the" and similar references used in the context of describing the present disclosure (especially in the context of the claims) are to be construed to cover both the singular and the plural, unless otherwise indicated herein or clearly contradicted by the context.

All methods described herein can be performed in any suitable order unless otherwise indicated herein or otherwise clearly contradicted by the context.

The use of any and all examples, or exemplary language (*e.g.*, "such as"), provided herein is intended merely to better illustrate the present disclosure and does not pose a limitation on the scope of the present disclosure otherwise claimed. No language in the specification should be construed as indicating any non-claimed element essential to the practice of the present disclosure.

The term "optional" or "optionally" as used herein means that the subsequently described event, circumstance or condition may or may not occur, and that the description includes instances where said event, circumstance, or condition occurs and instances in which it does not occur.

Where used herein, "and/or" is to be taken as specific disclosure of each of the two specified features or components with or without the other. For example, "X and/or Y" is to be taken as specific disclosure of each of (i) X, (ii) Y, and (iii) X and Y, just as if each is set out individually herein.

In the context of the present disclosure, the term "about" denotes an interval of accuracy that the person of ordinary skill will understand to still ensure the technical effect of the feature in question. The term typically indicates deviation from the indicated numerical value by ±10%, ±5%, ±4%, ±3%, ±2%, ±1%, ±0.9%, ±0.8%, ±0.7%, ±0.6%, ±0.5%, ±0.4%, ±0.3%, ±0.2%, ±0.1%, ±0.05%, and for example ±0.01%. In some embodiments, "about" indicates deviation from the indicated numerical value by ±10%. In some embodiments, "about" indicates deviation from the indicated numerical value by ±5%. In some embodiments, "about" indicates deviation from the indicated numerical value by ±4%. In some embodiments, "about" indicates deviation from the indicated numerical value by ±3%. In some embodiments, "about" indicates deviation from the indicated numerical value by ±2%. In some embodiments, "about" indicates deviation from the indicated numerical value by ±1%. In some embodiments, "about" indicates deviation from the indicated numerical value by ±0.9%. In some embodiments, "about" indicates deviation from the indicated numerical value by ±0.8%. In some embodiments, "about" indicates deviation from the indicated numerical value by ±0.7%. In some embodiments, "about" indicates deviation from the indicated numerical value by ±0.6%. In some embodiments, "about" indicates deviation from the indicated numerical value by ±0.5%. In some embodiments, "about" indicates deviation from the indicated numerical value by ±0.4%. In some embodiments, "about" indicates deviation from the indicated numerical value by ±0.3%. In some embodiments, "about" indicates deviation from the indicated numerical value by ±0.2%. In some embodiments, "about" indicates deviation from the indicated numerical value by ±0.1%. In some embodiments, "about" indicates deviation from the indicated numerical value by ±0.05%. In some embodiments, "about" indicates deviation from the indicated numerical value by ±0.01%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

Recitation of ranges of values herein is merely intended to serve as a shorthand method of referring individually to each separate value falling within the range. Unless otherwise indicated herein, each individual value is incorporated into the specification as if it were individually recited herein.

Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

As used herein, phrases such as "determining the amount" or "determining expression" or similar phrases with reference to an amino acid sequence (peptide or polypeptide) refer to determining the quantity or presence of an amino acid sequence.

Terms such as "reduce" or "inhibit" as used herein means the ability to cause an overall decrease, for example, of about 5% or greater, about 10% or greater, about 15% or greater, about 20% or greater, about 25% or greater, about 30% or greater, about 40% or greater, about 50% or greater, or about 75% or greater, in the level. The term "inhibit" or similar phrases includes a complete or essentially complete inhibition, *i.e.* a reduction to zero or essentially to zero.

The term "enhance" as used herein means the ability to cause an overall increase, or enhancement, for example, by at least about 5% or greater, about 10% or greater, about 15% or greater, about 20% or greater, about 25% or greater, about 30% or greater, about 40% or greater, about 50% or greater, about 75% or greater, or about 100% or greater in the level.

"Physiological pH" as used herein refers to a pH of about 7.4. In some embodiments, physiological pH is from 7.3 to 7.5. In some embodiments, physiological pH is from 7.35 to 7.45. In some embodiments, physiological pH is 7.3, 7.35, 7.4, 7.45, or 7.5.

As used in the present disclosure, "% w/v" refers to weight by volume percent, which is a unit of concentration measuring the amount of solute in grams (g) expressed as a percent of the total volume of solution in milliliters (mL).

As used in the present disclosure, "% by weight" refers to weight percent, which is a unit of concentration measuring the amount of a substance in grams (g) expressed as a percent of the total weight of the total composition in grams (g).

As used in the present disclosure, "mol %" is defined as the ratio of the number of moles of one component to the total number of moles of all components, multiplied by 100.

The term "recombinant" in the context of the present disclosure means "made through genetic engineering". In one embodiment, a "recombinant object" in the context of the present disclosure is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring. The term "found in nature" means "present in nature" and includes known objects as well as objects that have not yet been discovered and/or isolated from

nature, but that may be discovered and/or isolated in the future from a natural source.

According to various embodiments of the present invention, the composition of the present invention or the RNA obtained in the present invention is taken up by or introduced, *i.e.* transfected or transduced, into a cell which cell may be present *in vitro* or in a subject, e.g. resulting in expression of one or more encoded peptides or polypeptides. The cell may, e.g., express the encoded peptide or polypeptide intracellularly (*e.g.* in the cytoplasm and/or in the nucleus), may secrete the encoded peptide or polypeptide, and/or may express it on the surface.

According to the present disclosure, terms such as "nucleic acid expressing" and "nucleic acid encoding" or similar terms are used interchangeably herein and with respect to a particular peptide or polypeptide mean that the nucleic acid, if present in the appropriate environment, e.g. within a cell, can be expressed to produce said peptide or polypeptide.

The term "portion" refers to a fraction. With respect to a particular structure such as an amino acid sequence or protein the term "portion" thereof may designate a continuous or a discontinuous fraction of said structure.

The terms "part" and "fragment" are used interchangeably herein and refer to a continuous element. For example, a part of a structure such as an amino acid sequence or protein refers to a continuous element of said structure. When used in context of a composition, the term "part" means a portion of the composition. For example, a part of a composition may be any portion from 0.1% to 99.9% (such as 0.1 %, 0.5%, 1%, 5%, 10%, 50%, 90%, or 99%) of said composition.

"Fragment", with reference to an amino acid sequence (peptide or polypeptide), relates to a part of an amino acid sequence, *i.e.* a sequence which represents the amino acid sequence shortened at the N-terminus and/or C-terminus. A fragment shortened at the C-terminus (N-terminal fragment) is obtainable, *e.g.*, by translation of a truncated open reading frame that lacks the 3'-end of the open reading frame. A fragment shortened at the N-terminus (C-terminal fragment) is obtainable, *e.g.*, by translation of a truncated open reading frame that lacks the 5'-end of the open reading frame, as long as the truncated open reading frame comprises a start codon that serves to initiate translation. A fragment of an amino acid sequence comprises, *e.g.*, at least 50 %, at least 60 %, at least 70 %, at least 80%, at least 90% of the amino acid residues from an amino acid sequence. A fragment of an amino acid sequence comprises, e.g., at least 6, in particular at least 8, at least 10, at least 12, at least 15, at least 20, at least 30, at least 50, or at least 100 consecutive amino acids from an amino acid sequence. A fragment of an amino acid sequence comprises, e.g., a sequence of up to 8, in particular up to 10, up to 12, up to 15, up to 20, up to 30 or up to 55, consecutive amino acids of the amino acid sequence.

"Sequence identity" between two nucleic acid sequences indicates the percentage of nucleotides that are identical between the sequences. The terms "% identical" and "% identity" or similar terms are intended to refer, in particular, to the percentage of nucleotides or amino acids which are identical in an optimal alignment between the sequences to be compared. Said percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to identify local regions of corresponding sequences. The optimal alignment for a comparison may be carried out manually or with the aid of the local homology algorithm by Smith and Waterman, 1981, Ads App. Math. 2, 482, with the aid of the local homology algorithm by Neddleman and Wunsch, 1970, J. Mol. Biol. 48, 443, with the aid of the similarity search algorithm by Pearson and Lipman, 1988, Proc. Natl Acad. Sci. USA 88, 2444, or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.). In some embodiments, percent identity of two sequences is determined using the BLASTN or BLASTP algorithm, as available on the United States National Center for Biotechnology Information (NCBI) website (*e.g.*, at blast.ncbi.nlm.nih.gov/Blast.cgi?PAGE_TYPE=BlastSearch&BLAST_SPEC=blast2se q&LINK_LOC=align2seq). In some embodiments, the algorithm parameters used for BLASTN algorithm on the NCBI website include: (i) Expect Threshold set to 10; (ii) Word Size set to 28; (iii) Max matches in a query range set to 0; (iv) Match/Mismatch Scores set to 1, -2; (v) Gap Costs set to Linear; and (vi) the filter for low complexity regions being used. In some embodiments, the algorithm parameters used for BLASTP algorithm on the NCBI website include: (i) Expect Threshold set to 10; (ii) Word Size set to 3; (iii) Max matches in a query range set to 0; (iv) Matrix set to BLOSUM62; (v) Gap Costs set to Existence: 11 Extension: 1; and (vi) conditional compositional score matrix adjustment.

Percentage identity is obtained by determining the number of identical positions at which the sequences to be compared correspond, dividing this number by the number of positions compared (*e.g.*, the number of positions in the reference sequence) and multiplying this result by 100.

In some embodiments, the degree of similarity or identity is given for a region which is at least about 50%, at least about 60%, at least about 70%, at least about 80%, at least about 90% or about 100% of the entire length of the reference sequence. For example, if the reference nucleic acid sequence consists of 200 nucleotides, the degree of identity is given for at least about 100, at least about 120, at least about 140, at least about 160, at least about 180, or about 200 nucleotides, in some embodiments continuous nucleotides. In some embodiments, the degree of similarity or identity is given for the entire length of the reference sequence.

In some embodiments, "isolated" means removed (e.g., purified) from the natural state or from an artificial

composition, such as a composition from a production process. For example, a nucleic acid, peptide or polypeptide naturally present in a living animal is not "isolated", but the same nucleic acid, peptide or polypeptide partially or completely separated from the coexisting materials of its natural state is "isolated". An isolated nucleic acid, peptide or polypeptide can exist in substantially purified form, or can exist in a non-native environment such as, for example, a host cell.

The term "transfection" relates to the introduction of nucleic acids, in particular RNA, into a cell. For purposes of the present disclosure, the term "transfection" also includes the introduction of a nucleic acid into a cell or the uptake of a nucleic acid by such cell, wherein the cell may be present in a subject, *e.g.*, a patient, or the cell may be *in vitro, e.g.*, outside of a patient. Thus, according to the present disclosure, a cell for transfection of a nucleic acid described herein can be present *in vitro* or *in vivo, e.g.* the cell can form part of an organ, a tissue and/or the body of a patient. According to the disclosure, transfection can be transient or stable. For some applications of transfection, it is sufficient if the transfected genetic material is only transiently expressed. RNA can be transfected into cells to transiently express its coded protein. Since the nucleic acid introduced in the transfection process is usually not integrated into the nuclear genome, the foreign nucleic acid will be diluted through mitosis or degraded. Cells allowing episomal amplification of nucleic acids greatly reduce the rate of dilution. If it is desired that the transfected nucleic acid actually remains in the genome of the cell and its daughter cells, a stable transfection must occur. Such stable transfection can be achieved by using virus-based systems or transposon-based systems for transfection, for example. Generally, nucleic acid encoding antigen is transiently transfected into cells. RNA can be transfected into cells to transiently express its coded protein.

Cells which are useful for transfection in the methods described herein include, but are not limited to, cells from an animal cell line, such as Chinese hamster ovary (CHO), K562, HepG2, HEK293T, RAW, and C2C12 cells. In some embodiments, the cells are CHO, K562, HEK293T, RAW, and C2C12 cells. In some embodiments, the cells are Chinese hamster ovary (CHO) cells.

As used herein "endogenous" refers to any material from or produced inside an organism, cell, tissue or system.

As used herein, the term "exogenous" refers to any material introduced from or produced outside an organism, cell, tissue or system.

The term "expression" as used herein is defined as the transcription and/or translation of a particular nucleotide sequence.

In the context of the present disclosure, the term "transcription" relates to a process, wherein the genetic code in a DNA sequence is transcribed into RNA (especially mRNA). Subsequently, the RNA may be translated into peptide or polypeptide.

With respect to RNA, the term "expression" or "translation" relates to the process in the ribosomes of a cell by which a strand of mRNA directs the assembly of a sequence of amino acids to make a peptide or polypeptide.

**Nucleic acids**

The term "nucleic acid" comprises deoxyribonucleic acid (DNA), ribonucleic acid (RNA), combinations thereof, and modified forms thereof. The term comprises genomic DNA, cDNA, mRNA, recombinantly produced and chemically synthesized molecules. A nucleic acid may be present as a single-stranded or double-stranded and linear or covalently circularly closed molecule. A nucleic acid can be isolated. The term "isolated nucleic acid" means, according to the present disclosure, that the nucleic acid (i) was amplified in vitro, for example via polymerase chain reaction (PCR) for DNA or in vitro transcription (using, e.g., an RNA polymerase) for RNA, (ii) was produced recombinantly by cloning, (iii) was purified, for example, by cleavage and separation by gel electrophoresis, or (iv) was synthesized, for example, by chemical synthesis.

The term "nucleoside" (abbreviated herein as "N") relates to compounds which can be thought of as nucleotides without a phosphate group. While a nucleoside is a nucleobase linked to a sugar (e.g., ribose or deoxyribose), a nucleotide is composed of a nucleoside and one or more phosphate groups. Examples of nucleosides include cytidine, uridine, pseudouridine, adenosine, and guanosine.

The five standard nucleosides which usually make up naturally occurring nucleic acids are uridine, adenosine, thymidine, cytidine and guanosine. The five nucleosides are commonly abbreviated to their one letter codes U, A, T, C and G, respectively. However, thymidine is more commonly written as "dT" ("d" represents "deoxy") as it contains a 2'-deoxyribofuranose moiety rather than the ribofuranose ring found in uridine. This is because thymidine is found in deoxyribonucleic acid (DNA) and not ribonucleic acid (RNA). Conversely, uridine is found in RNA and not DNA. The remaining three nucleosides may be found in both RNA and DNA. In RNA, they would be represented as A, C and G, whereas in DNA they would be represented as dA, dC and dG.

A modified purine (A or G) or pyrimidine (C, T, or U) base moiety is preferably modified by one or more alkyl groups, more preferably one or more C1-4 alkyl groups, even more preferably one or more methyl groups. Particular examples of modified purine or pyrimidine base moieties include N7-alkyl-guanine, N6-alkyl-adenine, 5-alkyl-cytosine, 5-alkyl-uracil, and N(1)-alkyl-uracil, such as N7-C1-4 alkyl-guanine, N6-C1-4 alkyl-adenine, 5-C1-4 alkyl-cytosine, 5-C1-4 alkyl-uracil, and N(1)-C1-4 alkyl-uracil, preferably N7-methyl-guanine, N6-methyl-adenine, 5-methyl-cytosine, 5-methyl-uracil, and N(1)-methyl-uracil.

Herein, the term "DNA" relates to a nucleic acid molecule which includes deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

DNA may be recombinant DNA and may be obtained by cloning of a nucleic acid, in particular cDNA. The cDNA may be obtained by reverse transcription of RNA.

The term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered/modified nucleotides can be referred to as analogs of naturally occurring nucleotides, and the corresponding RNAs containing such altered/modified nucleotides (i.e., altered/modified RNAs) can be referred to as analogs of naturally occurring RNAs. A molecule contains "a majority of ribonucleotide residues" if the content of ribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

"RNA" includes mRNA, tRNA, ribosomal RNA (rRNA), small nuclear RNA (snRNA), self-amplifying RNA (saRNA), single-stranded RNA (ssRNA), dsRNA, inhibitory RNA (such as antisense ssRNA, small interfering RNA (siRNA), or microRNA (miRNA)), activating RNA (such as small activating RNA) and immunostimulatory RNA (isRNA). In some embodiments, "RNA" refers to mRNA.

The term "in vitro transcription" or "IVT" as used herein means that the transcription (i.e., the generation of RNA) is conducted in a cell-free manner. I.e., IVT does not use living/cultured cells but rather the transcription machinery extracted from cells (e.g., cell lysates or the isolated components thereof, including an RNA polymerase (preferably T7, T3 or SP6 polymerase)).

In some embodiments, the RNA is single stranded RNA.

In some embodiments, the RNA is mRNA.

In some embodiments, the RNA is generated by RNA in vitro transcription.

In some embodiments, the RNA comprises a 5' cap structure.

In some embodiments, the RNA does not comprise modified ribonucleotides.

In some embodiments, the RNA comprises modified ribonucleotides. In some embodiments, the modified ribonucleotides comprise modified uridines. In some embodiments, the modified uridines comprise N1-methyl-pseudouridine.

In some embodiments, the DNA is present in the form of a vector.

In some embodiments, the vector comprises DNA encoding an amino acid sequence comprising the amino acid sequence of a peptide or polypeptide having biological activity.

In some embodiments, the vector is a DNA vector.

In some embodiments, the composition of the present invention or the RNA obtained in the present invention, which can comprise at least two or more RNA molecules, is formulated with a delivery vehicle.

In some embodiments, the composition of the present invention or the RNA obtained in the present invention is formulated with one or more compounds complexing the RNA molecules.

In some embodiments, the composition of the present invention or the RNA obtained in the present invention is formulated as particles. In an embodiment, the present invention provides particles comprising a composition of the invention. In an embodiment, the present invention provides particles comprising RNA obtained in the invention. In an embodiment, the present invention provides particles comprising RNA that has been purified from a composition of the invention.

In an embodiment, the composition of the present invention is packaged in a particle. In an embodiment, the RNA molecules comprised in the compositions of the present invention are packaged in a particle. In an embodiment, the RNA molecules obtained in the present invention are packaged in a particle. In an embodiment, the particle is a lipoplex particle In an embodiment, the particle is a lipid nanoparticle.

In some embodiments, the composition of the present invention or the RNA obtained in the present invention is formulated as lipoplex particles. In these embodiments, it is preferred that the cells are characterized by a macropinocytosis-mediated RNA uptake mechanism.

In some embodiments, the composition of the present invention or the RNA obtained in the present invention is formulated as lipid nanoparticles.

In some embodiments, the composition of the present invention or the RNA obtained in the present invention comprises at least two RNA molecules, each of which encodes an amino acid sequence comprising the amino acid sequence of a peptide or polypeptide having biological activity.

In some embodiments, the composition of the present invention or the RNA obtained in the present invention comprises at least two RNA molecules, each encoding different amino acid sequences comprising the amino acid sequence of a peptide or polypeptide having biological activity.

In some embodiments, the different amino acid sequences comprise the amino acid sequence of different peptides or polypeptides having biological activity.

In some embodiments, the different peptides or polypeptides having biological activity comprise different antigens.

In some embodiments, the RNA described herein is single-stranded RNA that may be translated into the respective protein upon entering cells, e.g., cells used in the assays described herein and cells of a recipient. In addition to wildtype or codon-optimized sequences encoding the amino acid sequence comprising the amino acid sequence of a peptide or polypeptide having biological activity, e.g., a pharmaceutically active peptide or polypeptide such as antigen sequence, the RNA may contain one or more structural elements optimized for maximal efficacy of the RNA with respect to stability and translational efficiency (5' cap, 5' UTR, 3' UTR, poly(A)-tail). In one embodiment, the RNA contains all of these elements. In one embodiment, beta-S-ARCA(D1) (m27,2'-OGppSpG) or m27,3'-OGppp(m12'-O)ApG may be utilized as specific capping structure at the 5'-end of the RNA drug substances. As 5'-UTR sequence, the 5'-UTR sequence of the human alpha-globin mRNA, optionally with an optimized 'Kozak sequence' to increase translational efficiency may be used. As 3'-UTR sequence, a combination of two sequence elements (FI element) derived from the "amino terminal enhancer of split" (AES) mRNA (called F) and the mitochondrial encoded 12S ribosomal RNA (called I) placed between the coding sequence and the poly(A)-tail to assure higher maximum protein levels and prolonged persistence of the mRNA may be used. These were identified by an ex vivo selection process for sequences that confer RNA stability and augment total protein expression (see WO 2017/060314, herein incorporated by reference). Alternatively, the 3'-UTR may be two re-iterated 3'-UTRs of the human beta-globin mRNA. Furthermore, a poly(A)-tail measuring 110 nucleotides in length, consisting of a stretch of 30 adenosine residues, followed by a 10 nucleotide linker sequence (of random nucleotides) and another 70 adenosine residues may be used. This poly(A)-tail sequence was designed to enhance RNA stability and translational efficiency.

The amino acid sequence comprising the amino acid sequence of a peptide or polypeptide having biological activity, e.g., a pharmaceutically active peptide or polypeptide such as antigen sequence, may comprise amino acid sequences other than the amino acid sequence of a peptide or polypeptide having biological activity. Such other amino acid sequences may support the function or activity of the peptide or polypeptide having biological activity. In some embodiments, such other amino acid sequences comprise an amino acid sequence enhancing antigen processing and/or presentation. Alternatively, or additionally, such other amino acid sequences comprise an amino acid sequence which breaks immunological tolerance. Alternatively, or additionally, such other amino acid sequences comprise an amino acid sequence which produces bioluminescence. Such other amino acid sequences may be useful for determining the amount of the amino acid sequence comprising the amino acid sequence of a peptide or polypeptide having biological activity or a fragment thereof in the assays described herein. In particular, such other amino acid sequences may be useful for quantification by LC-MS/MS analysis.

The composition of the present invention or the RNA obtained in the present invention may be complexed with polymers, proteins and/or lipids, preferably lipids, to generate nucleic acid-particles for administration. If a combination of different nucleic acids is used, the nucleic acids may be complexed together or complexed separately.

**mRNA**

According to the present disclosure, the term "mRNA" means "messenger-RNA" and relates to a "transcript" which may be generated by using a DNA template and may encode a peptide or polypeptide. Typically, an mRNA comprises a 5'-UTR, a peptide/polypeptide coding region, and a 3'-UTR. In the context of the present disclosure, mRNA may be generated by in vitro transcription (IVT) from a DNA template. As set forth above, the in vitro transcription methodology is known to the skilled person, and a variety of in vitro transcription kits is commercially available.

mRNA is single-stranded but may contain self-complementary sequences that allow parts of the mRNA to fold and pair with itself to form double helices.

According to the present disclosure, "dsRNA" means double-stranded RNA and is RNA with two partially or completely complementary strands.

In preferred embodiments of the present disclosure, the mRNA relates to an RNA transcript which encodes a peptide or polypeptide.

In some embodiments, the mRNA which preferably encodes a peptide or polypeptide has a length of at least 45 nucleotides (such as at least 60, at least 90, at least 100, at least 200, at least 300, at least 400, at least 500, at least 600, at least 700, at least 800, at least 900, at least 1,000, at least 1,500, at least 2,000, at least 2,500, at least 3,000, at least 3,500, at least 4,000, at least 4,500, at least 5,000, at least 6,000, at least 7,000, at least 8,000, at least 9,000 nucleotides), preferably up to 15,000, such as up to 14,000, up to 13,000, up to 12,000 nucleotides, up to 11,000 nucleotides or up to 10,000 nucleotides.

As established in the art, mRNA generally contains a 5' untranslated region (5'-UTR), a peptide/polypeptide coding region and a 3' untranslated region (3'-UTR). In some embodiments, the mRNA is produced by in vitro transcription or chemical synthesis. In some embodiments, the mRNA is produced by in vitro transcription using a DNA template. The in vitro transcription methodology is known to the skilled person; cf., e.g., Molecular Cloning: A Laboratory Manual, 4th Edition, M.R. Green and J. Sambrook eds., Cold Spring Harbor Laboratory Press, Cold Spring Harbor 2012. Furthermore, a variety of in vitro transcription kits is commercially available, e.g., from Thermo Fisher Scientific (such as TranscriptAidTM T7 kit, MEGAscript® T7 kit, MAXIscript®), New England BioLabs Inc. (such as HiScribe™ T7 kit, HiScribe™ T7 ARCA mRNA kit), Promega (such as RiboMAX™, HeLaScribe®, Riboprobe® systems), Jena Bioscience (such as SP6 or T7 transcription kits), and Epicentre (such as AmpliScribe™). For providing modified mRNA, correspondingly modified nucleotides, such as modified naturally occurring nucleotides, non-naturally occurring nucleotides and/or modified non-naturally occurring nucleotides, can be incorporated during synthesis (preferably in vitro transcription), or modifications can be effected in and/or added to the mRNA after transcription.

In some embodiments, mRNA is in vitro transcribed mRNA (IVT-RNA) and may be obtained by in vitro transcription of an appropriate DNA template. The promoter for controlling transcription can be any promoter for any RNA polymerase. Particular examples of RNA polymerases are the T7, T3, and SP6 RNA polymerases. Preferably, the in vitro transcription is controlled by a T7 or SP6 promoter. A DNA template for in vitro transcription may be obtained by cloning of a nucleic acid, in particular cDNA, and introducing it into an appropriate vector for in vitro transcription. The cDNA may be obtained by reverse transcription of RNA.

In some embodiments of the present disclosure, the mRNA is "replicon mRNA" or simply a "replicon", in particular "self-replicating mRNA" or "self-amplifying mRNA". In certain embodiments, the replicon or self-replicating mRNA is derived from or comprises elements derived from an ssRNA virus, in particular a positive-stranded ssRNA virus such as an alphavirus. Alphaviruses are typical representatives of positive-stranded RNA viruses. Alphaviruses replicate in the cytoplasm of infected cells (for review of the alphaviral life cycle see José et al., Future Microbiol., 2009, vol. 4, pp. 837-856). The total genome length of many alphaviruses typically ranges between 11,000 and 12,000 nucleotides, and the genomic RNA typically has a 5'-cap, and a 3' poly(A) tail. The genome of alphaviruses encodes non-structural proteins (involved in transcription, modification and replication of viral RNA and in protein modification) and structural proteins (forming the virus particle). There are typically two open reading frames (ORFs) in the genome. The four non-structural proteins (nsP1-nsP4) are typically encoded together by a first ORF beginning near the 5' terminus of the genome, while alphavirus structural proteins are encoded together by a second ORF which is found downstream of the first ORF and extends near the 3' terminus of the genome. Typically, the first ORF is larger than the second ORF, the ratio being roughly 2:1. In cells infected by an alphavirus, only the nucleic acid sequence encoding non-structural proteins is translated from the genomic RNA, while the genetic information encoding structural proteins is translatable from a subgenomic transcript, which is an RNA molecule that resembles eukaryotic messenger RNA (mRNA; Gould et al., 2010, Antiviral Res., vol. 87 pp. 111-124). Following infection, i.e. at early stages of the viral life cycle, the (+) stranded genomic RNA directly acts like a messenger RNA for the translation of the open reading frame encoding the non-structural polyprotein (nsP1234). Alphavirus-derived vectors have been proposed for delivery of foreign genetic information into target cells or target organisms. In simple approaches, the open reading frame encoding alphaviral structural proteins is replaced by an open reading frame encoding a protein of interest. Alphavirus-based trans-replication systems rely on alphavirus nucleotide sequence elements on two separate nucleic acid molecules: one nucleic acid molecule encodes a viral replicase, and the

other nucleic acid molecule is capable of being replicated by said replicase in trans (hence the designation trans-replication system). Trans-replication requires the presence of both these nucleic acid molecules in a given host cell. The nucleic acid molecule capable of being replicated by the replicase in trans must comprise certain alphaviral sequence elements to allow recognition and RNA synthesis by the alphaviral replicase.

In some embodiments of the present disclosure, the mRNA contains one or more modifications, e.g., in order to increase its stability and/or increase translation efficiency and/or decrease immunogenicity and/or decrease cytotoxicity. For example, in order to increase expression of the mRNA, it may be modified within the coding region, i.e., the sequence encoding the expressed peptide or polypeptide, preferably without altering the sequence of the expressed peptide or polypeptide. Such modifications are described, for example, in WO 2007/036366 and PCT/EP2019/056502, and include the following: a 5'-cap structure; an extension or truncation of the naturally occurring poly(A) tail; an alteration of the 5'- and/or 3'-untranslated regions (UTR) such as introduction of a UTR which is not related to the coding region of said RNA; the replacement of one or more naturally occurring nucleotides with synthetic nucleotides; and codon optimization (e.g., to alter, preferably increase, the GC content of the RNA).

In some embodiments, the mRNA comprises a 5'-cap structure. In some embodiments, the mRNA does not have uncapped 5'-triphosphates. In some embodiments, the mRNA may comprise a conventional 5'-cap and/or a 5'-cap analog. The term "conventional 5'-cap" refers to a cap structure found on the 5'-end of an mRNA molecule and generally consists of a guanosine 5'-triphosphate (Gppp) which is connected via its triphosphate moiety to the 5'-end of the next nucleotide of the mRNA (i.e., the guanosine is connected via a 5' to 5' triphosphate linkage to the rest of the mRNA). The guanosine may be methylated at position N7 (resulting in the cap structure m7Gppp). The term "5'-cap analog" includes a 5'-cap which is based on a conventional 5'-cap but which has been modified at either the 2'- or 3'-position of the m7guanosine structure in order to avoid an integration of the 5'-cap analog in the reverse orientation (such 5'-cap analogs are also called anti-reverse cap analogs (ARCAs)). Particularly preferred 5'-cap analogs are those having one or more substitutions at the bridging and non-bridging oxygen in the phosphate bridge, such as phosphorothioate modified 5'-cap analogs at the β-phosphate (such as m27,2'OG(5')ppSp(5')G (referred to as beta-S-ARCA or β-S-ARCA)), as described in PCT/EP2019/056502. Providing an mRNA with a 5'-cap structure as described herein may be achieved by in vitro transcription of a DNA template in presence of a corresponding 5'-cap compound, wherein said 5'-cap structure is co-transcriptionally incorporated into the generated mRNA strand, or the mRNA may be generated, for example, by in vitro transcription, and the 5'-cap structure may be attached to the mRNA post-transcriptionally using capping enzymes, for example, capping enzymes of vaccinia virus.

In some embodiments, the mRNA comprises a 5'-cap structure selected from the group consisting of m27,2'OG(5')ppSp(5')G (in particular its D1 diastereomer), m27,3'OG(5')ppp(5')G, and m27,3'-OGppp(m12'-O)ApG.

In some embodiments, the mRNA comprises a cap0, cap1, or cap2, preferably cap1 or cap2. According to the present disclosure, the term "cap0" means the structure "m7GpppN", wherein N is any nucleoside bearing an OH moiety at position 2'. According to the present disclosure, the term "cap1" means the structure "m7GpppNm", wherein Nm is any nucleoside bearing an OCH3 moiety at position 2'. According to the present disclosure, the term "cap2" means the structure "m7GpppNmNm", wherein each Nm is independently any nucleoside bearing an OCH3 moiety at position 2'.

The D1 diastereomer of beta-S-ARCA (β-S-ARCA) has the following structure:

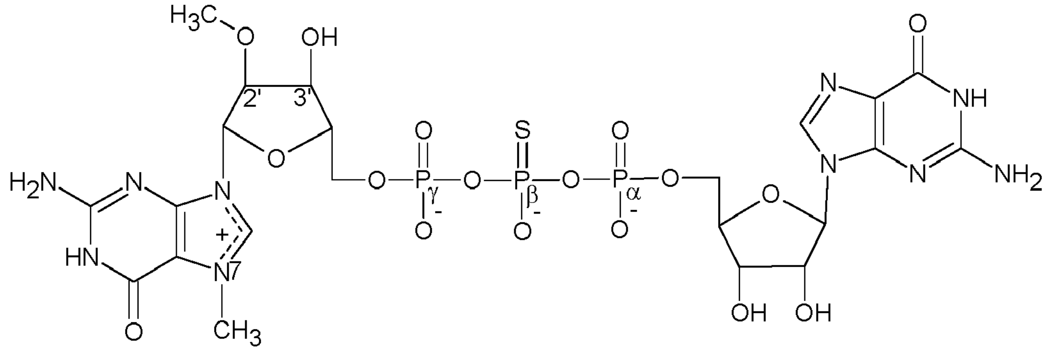

The "D1 diastereomer of beta-S-ARCA" or "beta-S-ARCA(D1)" is the diastereomer of beta-S-ARCA which elutes first on an HPLC column compared to the D2 diastereomer of beta-S-ARCA (beta-S-ARCA(D2)) and thus exhibits a shorter retention time. The HPLC preferably is an analytical HPLC. In some embodiments, a Supelcosil LC-18-T RP column, preferably of the format: 5 μm, 4.6 x 250 mm is used for separation, whereby a flow rate of 1.3 ml/min can be applied. In some embodiments, a gradient of methanol in ammonium acetate, for example, a 0-25% linear gradient of methanol in 0.05 M ammonium acetate, pH = 5.9, within 15 min is used. UV-detection (VWD) can be performed at 260 nm and fluorescence detection (FLD) can be performed with excitation at 280 nm and detection at 337 nm.

The 5'-cap analog m27,3'-OGppp(m12'-O)ApG (also referred to as m27,3'OG(5')ppp(5')m2'-OApG) which is a

building block of a cap1 has the following structure:

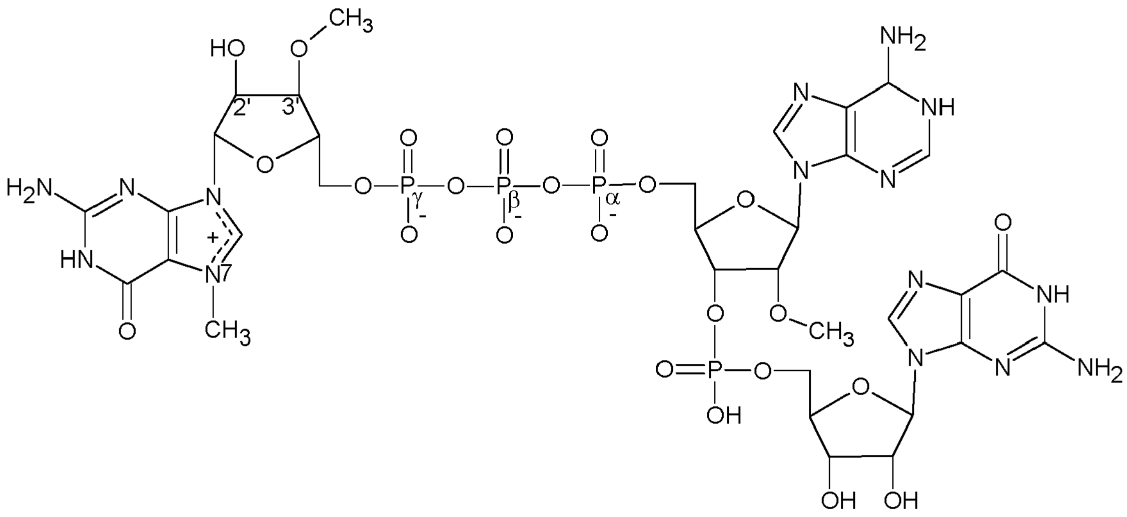

An exemplary cap0 mRNA comprising β-S-ARCA and mRNA has the following structure:

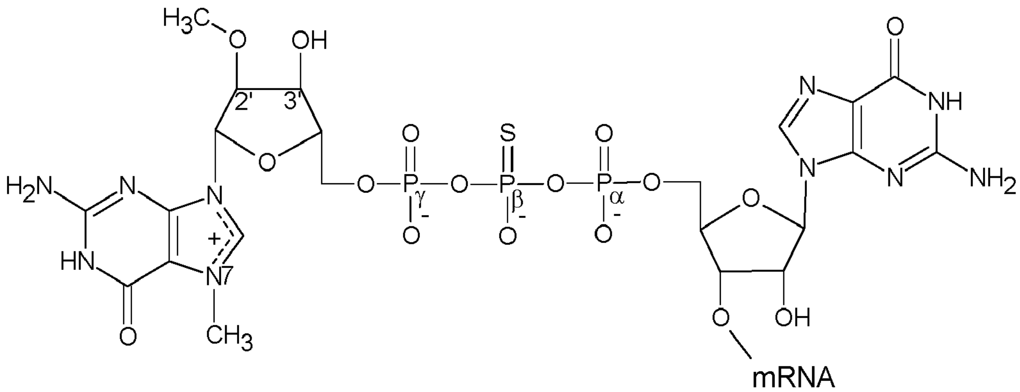

An exemplary cap0 mRNA comprising m27,3'OG(5')ppp(5')G and mRNA has the following structure:

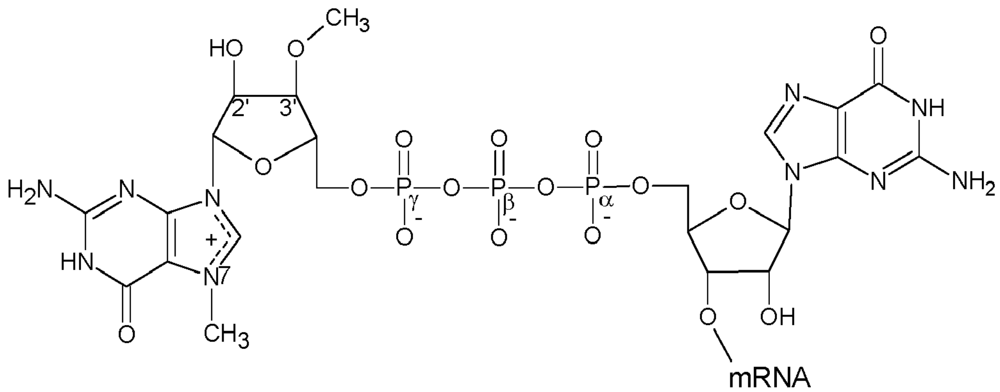

An exemplary cap1 mRNA comprising m27,3'-OGppp(m12'-O)ApG and mRNA has the following structure:

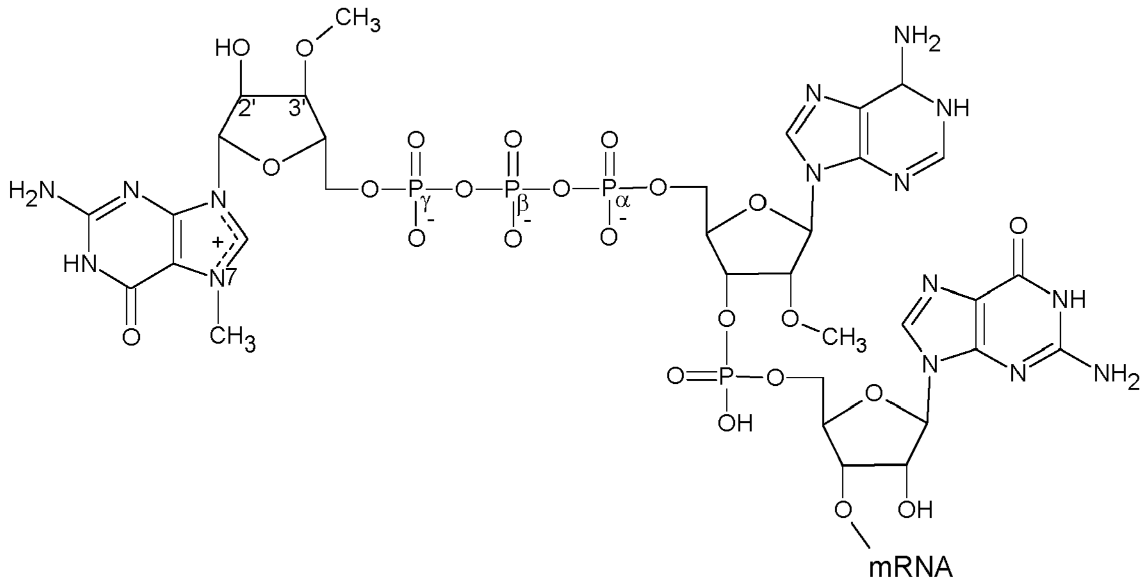

As used herein, the term "poly-A tail" or "poly-A sequence" refers to an uninterrupted or interrupted sequence of adenylate residues which is typically located at the 3'-end of an mRNA molecule. Poly-A tails or poly-A sequences are known to those of skill in the art and may follow the 3'-UTR in the mRNAs described herein. An uninterrupted poly-A tail is characterized by consecutive adenylate residues. In nature, an uninterrupted poly-A tail is typical. mRNAs disclosed herein can have a poly-A tail attached to the free 3'-end of the mRNA by a template-independent RNA polymerase after transcription or a poly-A tail encoded by DNA and transcribed by a template-dependent RNA polymerase.

It has been demonstrated that a poly-A tail of about 120 A nucleotides has a beneficial influence on the levels of mRNA in transfected eukaryotic cells, as well as on the levels of protein that is translated from an open reading frame that is present upstream (5') of the poly-A tail (Holtkamp et al., 2006, Blood, vol. 108, pp. 4009-4017).

The poly-A tail may be of any length. In some embodiments, a poly-A tail comprises, essentially consists of, or consists of at least 20, at least 30, at least 40, at least 80, or at least 100 and up to 500, up to 400, up to 300, up to 200, or up to 150 A nucleotides, and, in particular, about 120 A nucleotides. In this context, "essentially consists of" means that most nucleotides in the poly-A tail, typically at least 75%, at least 80%, at least 85%, at least 90%, at least 95%, at least 96%, at least 97%, at least 98%, or at least 99% by number of nucleotides in the poly-A tail are A nucleotides, but permits that remaining nucleotides are nucleotides other than A nucleotides, such as U nucleotides (uridylate), G nucleotides (guanylate), or C nucleotides (cytidylate). In this context, "consists of' means that all nucleotides in the poly-A tail, i.e., 100% by number of nucleotides in the poly-A tail, are A nucleotides. The term "A nucleotide" or "A" refers to adenylate.

In some embodiments, a poly-A tail is attached during RNA transcription, e.g., during preparation of in vitro transcribed RNA, based on a DNA template comprising repeated dT nucleotides (deoxythymidylate) in the strand complementary to the coding strand. The DNA sequence encoding a poly-A tail (coding strand) is referred to as poly(A) cassette.

In some embodiments, the poly(A) cassette present in the coding strand of DNA essentially consists of dA nucleotides, but is interrupted by a random sequence of the four nucleotides (dA, dC, dG, and dT). Such random sequence may be 5 to 50, 10 to 30, or 10 to 20 nucleotides in length. Such a cassette is disclosed in WO 2016/005324 A1, hereby incorporated by reference. Any poly(A) cassette disclosed in WO 2016/005324 A1 may be used in the present disclosure. A poly(A) cassette that essentially consists of dA nucleotides, but is interrupted by a random sequence having an equal distribution of the four nucleotides (dA, dC, dG, dT) and having a length of e.g., 5 to 50 nucleotides shows, on DNA level, constant propagation of plasmid DNA in E. coli and is still associated, on RNA level, with the beneficial properties with respect to supporting RNA stability and translational efficiency is encompassed. Consequently, in some embodiments, the poly-A tail contained in an mRNA molecule described herein essentially consists of A nucleotides, but is interrupted by a random sequence of the four nucleotides (A, C, G, U). Such random sequence may be 5 to 50, 10 to 30, or 10 to 20 nucleotides in length.

In some embodiments, no nucleotides other than A nucleotides flank a poly-A tail at its 3'-end, i.e., the poly-A tail is not masked or followed at its 3'-end by a nucleotide other than A.

In some embodiments, a poly-A tail may comprise at least 20, at least 30, at least 40, at least 80, or at least 100 and up to 500, up to 400, up to 300, up to 200, or up to 150 nucleotides. In some embodiments, the poly-A tail may essentially consist of at least 20, at least 30, at least 40, at least 80, or at least 100 and up to 500, up to 400, up to 300, up to 200, or up to 150 nucleotides. In some embodiments, the poly-A tail may consist of at least 20, at least 30, at least 40, at

least 80, or at least 100 and up to 500, up to 400, up to 300, up to 200, or up to 150 nucleotides. In some embodiments, the poly-A tail comprises at least 100 nucleotides. In some embodiments, the poly-A tail comprises about 150 nucleotides. In some embodiments, the poly-A tail comprises about 120 nucleotides.

In some embodiments, mRNA used in present disclosure comprises a 5'-UTR and/or a 3'-UTR. The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR). A 5'-UTR, if present, is located at the 5'-end, upstream of the start codon of a protein-encoding region. A 5'-UTR is downstream of the 5'-cap (if present), e.g., directly adjacent to the 5'-cap. A 3'-UTR, if present, is located at the 3'-end, downstream of the termination codon of a protein-encoding region, but the term "3'-UTR" does generally not include the poly-A sequence. Thus, the 3'-UTR is upstream of the poly-A sequence (if present), e.g., directly adjacent to the poly-A sequence. Incorporation of a 3'-UTR into the 3'-non translated region of an RNA (preferably mRNA) molecule can result in an enhancement in translation efficiency. A synergistic effect may be achieved by incorporating two or more of such 3'-UTRs (which are preferably arranged in a head-to-tail orientation; cf., e.g., Holtkamp et al., Blood 108, 4009-4017 (2006)). The 3'-UTRs may be autologous or heterologous to the RNA (e.g., mRNA) into which they are introduced. In certain embodiments, the 3'-UTR is derived from a globin gene or mRNA, such as a gene or mRNA of alpha2-globin, alpha1-globin, or beta-globin, e.g., beta-globin, e.g., human beta-globin. For example, the RNA (e.g., mRNA) may be modified by the replacement of the existing 3'-UTR with or the insertion of one or more, e.g., two copies of a 3'-UTR derived from a globin gene, such as alpha2-globin, alpha1-globin, beta-globin, e.g., beta-globin, e.g., human beta-globin.

The mRNA may have modified ribonucleotides in order to increase its stability and/or decrease immunogenicity and/or decrease cytotoxicity. For example, in some embodiments, uridine in the mRNA described herein is replaced (partially or completely, preferably completely) by a modified nucleoside. In some embodiments, the modified nucleoside is a modified uridine.

In some embodiments, the modified uridine replacing uridine is selected from the group consisting of pseudouridine (ψ), N1-methyl-pseudouridine (m1ψ), 5-methyluridine (m5U), and combinations thereof.

In some embodiments, the modified nucleoside replacing (partially or completely, preferably completely) uridine in the mRNA may be any one or more of 3-methyluridine (m3U), 5-methoxy-uridine (mo5U), 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho5U), 5-aminoallyl-uridine, 5-halo-uridine (e.g., 5-iodo-uridineor 5-bromo-uridine), uridine 5-oxyacetic acid (cmo5U), uridine 5-oxyacetic acid methyl ester (mcmo5U), 5-carboxymethyl-uridine (cm5U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mcm5U), 5-methoxycarbonylmethyl-2-thio-uridine (mcm5s2U), 5-aminomethyl-2-thio-uridine (nm5s2U), 5-methylaminomethyl-uridine (mnm5U), 1-ethyl-pseudouridine, 5-methylaminomethyl-2-thio-uridine (mnm5s2U), 5-methylaminomethyl-2-seleno-uridine (mnm5se2U), 5-carbamoylmethyl-uridine (ncm5U), 5-carboxymethylaminomethyl-uridine (cmnm5U), 5-carboxymethylaminomethyl-2-thio-uridine (cmnm5s2U), 5-propynyl-uridine, 1-propynyl-pseudouridine, 5-taurinomethyl-uridine (τm5U), 1-taurinomethyl-pseudouridine, 5-taurinomethyl-2-thio-uridine(τm5s2U), 1-taurinomethyl-4-thio-pseudouridine), 5-methyl-2-thio-uridine (m5s2U), 1-methyl-4-thio-pseudouridine (m1s4ψ), 4-thio-1-methyl-pseudouridine, 3-methyl-pseudouridine (m3ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m5D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp3U), 1-methyl-3-(3-amino-3-carboxypropyl)pseudouridine (acp3 ψ), 5-(isopentenylaminomethyl)uridine (inm5U), 5-(isopentenylaminomethyl)-2-thio-uridine (inm5s2U), α-thio-uridine, 2'-O-methyluridine (Um), 5,2'-O-dimethyl-uridine (m5Um), 2'-O-methyl-pseudouridine (ψm), 2-thio-2'-O-methyl-uridine (s2Um), 5-methoxycarbonylmethyl-2'-O-methyl-uridine (mcm5Um), 5-carbamoylmethyl-2'-O-methyl-uridine (ncm5Um), 5-carboxymethylaminomethyl-2'-O-methyl-uridine (cmnm5Um), 3,2'-O-dimethyl-uridine (m3Um), 5-(isopentenylaminomethyl)-2'-O-methyl-uridine (inm5Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, or any other modified uridine known in the art.

An RNA (preferably mRNA) which is modified by pseudouridine (replacing partially or completely, preferably completely, uridine) is referred to herein as "ψ-modified", whereas the term "m1ψ-modified" means that the RNA (preferably mRNA) contains N(1)-methylpseudouridine (replacing partially or completely, preferably completely, uridine). Furthermore, the term "m5U-modified" means that the RNA (preferably mRNA) contains 5-methyluridine (replacing partially or completely, preferably completely, uridine). Such ψ- or m1ψ- or m5U-modified RNAs usually exhibit decreased immunogenicity compared to their unmodified forms and, thus, are preferred in applications where the induction of an immune response is to be avoided or minimized. In some embodiments, the RNA (preferably mRNA) contains N(1)-methylpseudouridine replacing completely uridine

The codons of the mRNA used in the present disclosure may further be optimized, e.g., to increase the GC content of the RNA and/or to replace codons which are rare in the cell (or subject) in which the peptide or polypeptide of

interest is to be expressed by codons which are synonymous frequent codons in said cell (or subject). In some embodiments, the amino acid sequence encoded by the mRNA used in the present disclosure is encoded by a coding sequence which is codon-optimized and/or the G/C content of which is increased compared to wild type coding sequence. This also includes embodiments, wherein one or more sequence regions of the coding sequence are codon-optimized and/or increased in the G/C content compared to the corresponding sequence regions of the wild type coding sequence. In some embodiments, the codon-optimization and/or the increase in the G/C content preferably does not change the sequence of the encoded amino acid sequence.

The term "codon-optimized" refers to the alteration of codons in the coding region of a nucleic acid molecule to reflect the typical codon usage of a host organism without preferably altering the amino acid sequence encoded by the nucleic acid molecule. Within the context of the present disclosure, coding regions may be codon-optimized for optimal expression in a subject to be treated using the mRNA described herein. Codon-optimization is based on the finding that the translation efficiency is also determined by a different frequency in the occurrence of tRNAs in cells. Thus, the sequence of mRNA may be modified such that codons for which frequently occurring tRNAs are available are inserted in place of "rare codons".

In some embodiments, the guanosine/cytosine (G/C) content of the coding region of the mRNA described herein is increased compared to the G/C content of the corresponding coding sequence of the wild type RNA, wherein the amino acid sequence encoded by the mRNA is preferably not modified compared to the amino acid sequence encoded by the wild type RNA. This modification of the mRNA sequence is based on the fact that the sequence of any RNA region to be translated is important for efficient translation of that mRNA. Sequences having an increased G (guanosine)/C (cytosine) content are more stable than sequences having an increased A (adenosine)/U (uracil) content. In respect to the fact that several codons code for one and the same amino acid (so-called degeneration of the genetic code), the most favorable codons for the stability can be determined (so-called alternative codon usage). Depending on the amino acid to be encoded by the mRNA, there are various possibilities for modification of the mRNA sequence, compared to its wild type sequence. In particular, codons which contain A and/or U nucleotides can be modified by substituting these codons by other codons, which code for the same amino acids but contain no A and/or U or contain a lower content of A and/or U nucleotides.

In various embodiments, the G/C content of the coding region of the mRNA described herein is increased by at least 10%, at least 20%, at least 30%, at least 40%, at least 50%, at least 55%, or even more compared to the G/C content of the coding region of the wild type RNA.

A combination of the above described modifications, i.e., incorporation of a 5'-cap structure, incorporation of a poly-A sequence, unmasking of a poly-A sequence, alteration of the 5'- and/or 3'-UTR (such as incorporation of one or more 3'-UTRs), replacing one or more naturally occurring nucleotides with synthetic nucleotides (e.g., 5-methylcytidine for cytidine and/or pseudouridine ($\psi$) or N(1)-methylpseudouridine (m1$\psi$) or 5-methyluridine (m5U) for uridine), and codon optimization, has a synergistic influence on the stability of RNA (preferably mRNA) and increase in translation efficiency. Thus, in some embodiments, the mRNA used in the present disclosure contains a combination of at least two, at least three, at least four or all five of the above-mentioned modifications, i.e., (i) incorporation of a 5'-cap structure, (ii) incorporation of a poly-A sequence, unmasking of a poly-A sequence; (iii) alteration of the 5'- and/or 3'-UTR (such as incorporation of one or more 3'-UTRs); (iv) replacing one or more naturally occurring nucleotides with synthetic nucleotides (e.g., 5-methylcytidine for cytidine and/or pseudouridine ($\psi$) or N(1)-methylpseudouridine (m1$\psi$) or 5-methyluridine (m5U) for uridine), and (v) codon optimization.

Some aspects of the disclosure involve the targeted delivery of the mRNA disclosed herein to certain cells or tissues. In some embodiments, the disclosure involves targeting the lymphatic system, in particular secondary lymphoid organs, more specifically spleen. Targeting the lymphatic system, in particular secondary lymphoid organs, more specifically spleen is in particular preferred if the mRNA administered is mRNA encoding an antigen or epitope for inducing an immune response. In some embodiments, the target cell is a spleen cell. In some embodiments, the target cell is an antigen presenting cell such as a professional antigen presenting cell in the spleen. In some embodiments, the target cell is a dendritic cell in the spleen. The "lymphatic system" is part of the circulatory system and an important part of the immune system, comprising a network of lymphatic vessels that carry lymph. The lymphatic system consists of lymphatic organs, a conducting network of lymphatic vessels, and the circulating lymph. The primary or central lymphoid organs generate lymphocytes from immature progenitor cells. The thymus and the bone marrow constitute the primary lymphoid organs. Secondary or peripheral lymphoid organs, which include lymph nodes and the spleen, maintain mature naive lymphocytes and initiate an adaptive immune response.

Lipid-based mRNA delivery systems have an inherent preference to the liver. Liver accumulation is caused by the discontinuous nature of the hepatic vasculature or the lipid metabolism (liposomes and lipid or cholesterol conjugates). In some embodiments, the target organ is liver and the target tissue is liver tissue. The delivery to such target tissue is preferred, in particular, if presence of mRNA or of the encoded peptide or polypeptide in this organ or tissue is desired and/or if it is desired to express large amounts of the encoded peptide or polypeptide and/or if systemic presence of the encoded peptide or polypeptide, in particular in significant amounts, is desired or required.

In some embodiments, after administration of the mRNA particles described herein, at least a portion of the mRNA is delivered to a target cell or target organ. In some embodiments, at least a portion of the mRNA is delivered to the cytosol of the target cell. In some embodiments, the mRNA is mRNA encoding a peptide or polypeptide and the mRNA is translated by the target cell to produce the peptide or polypeptide. In some embodiments, the target cell is a cell in the liver. In some embodiments, the target cell is a muscle cell. In some embodiments, the target cell is an endothelial cell. In some embodiments the target cell is a tumor cell or a cell in the tumor microenvironment. In some embodiments, the target cell is a blood cell. In some embodiments, the target cell is a cell in the lymph nodes. In some embodiments, the target cell is a cell in the lung. In some embodiments, the target cell is a blood cell. In some embodiments, the target cell is a cell in the skin. In some embodiments, the target cell is a spleen cell. In some embodiments, the target cell is an antigen presenting cell such as a professional antigen presenting cell in the spleen. In some embodiments, the target cell is a dendritic cell in the spleen. In some embodiments, the target cell is a T cell. In some embodiments, the target cell is a B cell. In some embodiments, the target cell is a NK cell. In some embodiments, the target cell is a monocyte. Thus, RNA particles described herein may be used for delivering mRNA to such target cell.

**Pharmaceutically active peptides or polypeptides**

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

In some embodiments, the composition of the present invention/RNA obtained in the present invention comprises a nucleic acid sequence encoding one or more functional sequences which can be peptides or polypeptides, preferably a pharmaceutically active peptide or polypeptide.

In a preferred embodiment, nucleic acid such as mRNA used in the present disclosure comprises a nucleic acid sequence encoding a peptide or polypeptide, preferably a pharmaceutically active peptide or polypeptide, and is capable of expressing said peptide or polypeptide, in particular if transferred into a cell or subject. Thus, in some embodiments, the nucleic acid used in the present disclosure contains a coding region (open reading frame (ORF)) encoding a peptide or polypeptide, e.g., encoding a pharmaceutically active peptide or polypeptide. In this respect, an "open reading frame" or "ORF" is a continuous stretch of codons beginning with a start codon and ending with a stop codon. Such nucleic acid encoding a pharmaceutically active peptide or polypeptide is also referred to herein as "pharmaceutically active nucleic acid". In particular, such mRNA encoding a pharmaceutically active peptide or polypeptide is also referred to herein as "pharmaceutically active mRNA".

According to the present disclosure, the term "pharmaceutically active peptide or polypeptide" means a peptide or polypeptide that can be used in the treatment of an individual where the expression of a peptide or polypeptide would be of benefit, e.g., in ameliorating the symptoms of a disease. Preferably, a pharmaceutically active peptide or polypeptide has curative or palliative properties and may be administered to ameliorate, relieve, alleviate, reverse, delay onset of or lessen the severity of one or more symptoms of a disease. In some embodiments, a pharmaceutically active peptide or polypeptide has a positive or advantageous effect on the condition or disease state of an individual when administered to the individual in a therapeutically effective amount. A pharmaceutically active peptide or polypeptide may have prophylactic properties and may be used to delay the onset of a disease or to lessen the severity of such disease. The term "pharmaceutically active peptide or polypeptide" includes entire peptides or polypeptides, and can also refer to pharmaceutically active fragments thereof. It can also include pharmaceutically active variants and/or analogs of a peptide or polypeptide.

Specific examples of pharmaceutically active peptides and polypeptides include, but are not limited to, cytokines, hormones, adhesion molecules, immunoglobulins, immunologically active compounds, growth factors, protease inhibitors, enzymes, receptors, apoptosis regulators, transcription factors, tumor suppressor proteins, structural proteins, reprogramming factors, genomic engineering proteins, and blood proteins.

The term "cytokines" relates to proteins which have a molecular weight of about 5 to 60 kDa and which participate in cell signaling (e.g., paracrine, endocrine, and/or autocrine signaling). In particular, when released, cytokines exert an effect on the behavior of cells around the place of their release. Examples of cytokines include lymphokines, interleukins, chemokines, interferons, and tumor necrosis factors (TNFs). According to the present disclosure, cytokines do not include hormones or growth factors. Cytokines differ from hormones in that (i) they usually act at much more variable concentrations than hormones and (ii) generally are made by a broad range of cells (nearly all nucleated cells can produce cytokines). Interferons are usually characterized by antiviral, antiproliferative and immunomodulatory activities.

Interferons are proteins that alter and regulate the transcription of genes within a cell by binding to interferon receptors on the regulated cell's surface, thereby preventing viral replication within the cells. The interferons can be grouped into two types. IFN-gamma is the sole type II interferon; all others are type I interferons. Particular examples of cytokines include erythropoietin (EPO), colony stimulating factor (CSF), granulocyte colony stimulating factor (G-CSF), granulocyte-macrophage colony stimulating factor (GM-CSF), tumor necrosis factor (TNF), bone morphogenetic protein (BMP), interferon alfa (IFN$\alpha$), interferon beta (IFN$\beta$), interferon gamma (INF$\gamma$), interleukin 2 (IL-2), interleukin 4 (IL-4), interleukin 10 (IL-10), interleukin 11 (IL-11), interleukin 12 (IL-12), interleukin 15 (IL-15), and interleukin 21 (IL-21), as well as variants and derivatives thereof.

In some embodiments, a pharmaceutically active peptide or polypeptide comprises a replacement protein. In these embodiments, the present disclosure provides a method for treatment of a subject having a disorder requiring protein replacement (e.g., protein deficiency disorders) comprising administering to the subject nucleic acid as described herein encoding a replacement protein. The term "protein replacement" refers to the introduction of a protein (including functional variants thereof) into a subject having a deficiency in such protein. The term also refers to the introduction of a protein into a subject otherwise requiring or benefiting from providing a protein, e.g., suffering from protein insufficiency. The term "disorder characterized by a protein deficiency" refers to any disorder that presents with a pathology caused by absent or insufficient amounts of a protein. This term encompasses protein folding disorders, i.e., conformational disorders, that result in a biologically inactive protein product. Protein insufficiency can be involved in infectious diseases, immunosuppression, organ failure, glandular problems, radiation illness, nutritional deficiency, poisoning, or other environmental or external insults.

The term "hormones" relates to a class of signaling molecules produced by glands, wherein signaling usually includes the following steps: (i) synthesis of a hormone in a particular tissue; (ii) storage and secretion; (iii) transport of the hormone to its target; (iv) binding of the hormone by a receptor; (v) relay and amplification of the signal; and (vi) breakdown of the hormone. Hormones differ from cytokines in that (1) hormones usually act in less variable concentrations and (2) generally are made by specific kinds of cells. In some embodiments, a "hormone" is a peptide or polypeptide hormone, such as insulin, vasopressin, prolactin, adrenocorticotropic hormone (ACTH), thyroid hormone, growth hormones (such as human grown hormone or bovine somatotropin), oxytocin, atrial-natriuretic peptide (ANP), glucagon, somatostatin, cholecystokinin, gastrin, and leptins.

The term "adhesion molecules" relates to proteins which are located on the surface of a cell and which are involved in binding of the cell with other cells or with the extracellular matrix (ECM). Adhesion molecules are typically transmembrane receptors and can be classified as calcium-independent (e.g., integrins, immunoglobulin superfamily, lymphocyte homing receptors) and calcium-dependent (cadherins and selectins). Particular examples of adhesion molecules are integrins, lymphocyte homing receptors, selectins (e.g., P-selectin), and addressins.

Integrins are also involved in signal transduction. In particular, upon ligand binding, integrins modulate cell signaling pathways, e.g., pathways of transmembrane protein kinases such as receptor tyrosine kinases (RTK). Such regulation can lead to cellular growth, division, survival, or differentiation or to apoptosis. Particular examples of integrins include: $\alpha1\beta1$, $\alpha2\beta1$, $\alpha3\beta1$, $\alpha4\beta1$, $\alpha5\beta1$, $\alpha6\beta1$, $\alpha7\beta1$, $\alpha L\beta2$, $\alpha M\beta2$, $\alpha IIb\beta3$, $\alpha V\beta1$, $\alpha V\beta3$, $\alpha V\beta5$, $\alpha V\beta6$, $\alpha V\beta8$, and $\alpha6\beta4$.

The term "immunoglobulins" or "immunoglobulin superfamily" refers to molecules which are involved in the recognition, binding, and/or adhesion processes of cells. Molecules belonging to this superfamily share the feature that they contain a region known as immunoglobulin domain or fold. Members of the immunoglobulin superfamily include antibodies (e.g., IgG), T cell receptors (TCRs), major histocompatibility complex (MHC) molecules, co-receptors (e.g., CD4, CD8, CD19), antigen receptor accessory molecules (e.g., CD-3$\gamma$, CD3-$\delta$, CD-3$\varepsilon$, CD79a, CD79b), co-stimulatory or inhibitory molecules (e.g., CD28, CD80, CD86), and other.

The term "immunologically active compound" relates to any compound altering an immune response, e.g., by inducing and/or suppressing maturation of immune cells, inducing and/or suppressing cytokine biosynthesis, and/or altering humoral immunity by stimulating antibody production by B cells. Immunologically active compounds possess potent immunostimulating activity including, but not limited to, antiviral and antitumor activity, and can also down-regulate other aspects of the immune response, for example shifting the immune response away from a TH2 immune response, which is useful for treating a wide range of TH2 mediated diseases. Immunologically active compounds can be useful as vaccine adjuvants. Particular examples of immunologically active compounds include interleukins, colony stimulating factor (CSF), granulocyte colony stimulating factor (G-CSF), granulocyte-macrophage colony stimulating factor (GM-CSF), erythropoietin, tumor necrosis factor (TNF), interferons, integrins, addressins, selectins, homing receptors, and antigens, in particular tumor-associated antigens, pathogen-associated antigens (such as bacterial, parasitic, or viral antigens), allergens, and autoantigens. An immunologically active compound may be a vaccine antigen, i.e., an antigen whose inoculation into a subject induces an immune response.

An "antigen" according to the present disclosure covers any substance that will elicit an immune response and/or any substance against which an immune response or an immune mechanism such as a cellular response and/or humoral response is directed. This also includes situations wherein the antigen is processed into antigen peptides and an immune response or an immune mechanism is directed against one or more antigen peptides, in particular if presented in the

context of MHC molecules. In particular, an "antigen" relates to any substance, such as a peptide or polypeptide, that reacts specifically with antibodies or T-lymphocytes (T-cells). The term "antigen" may comprise a molecule that comprises at least one epitope, such as a T cell epitope. In some embodiments, an antigen is a molecule which, optionally after processing, induces an immune reaction, which may be specific for the antigen (including cells expressing the antigen). In some embodiments, an antigen is a disease-associated antigen, such as a tumor antigen, a viral antigen, or a bacterial antigen, or an epitope derived from such antigen.

The term "autoantigen" or "self-antigen" refers to an antigen which originates from within the body of a subject (i.e., the autoantigen can also be called "autologous antigen") and which produces an abnormally vigorous immune response against this normal part of the body. Such vigorous immune reactions against autoantigens may be the cause of "autoimmune diseases".

According to the present disclosure, any suitable antigen may be used, which is a candidate for an immune response, wherein the immune response may be both a humoral as well as a cellular immune response. In the context of some embodiments of the present disclosure, the antigen is presented by a cell, such as by an antigen presenting cell, in the context of MHC molecules, which results in an immune response against the antigen. An antigen may be a product which corresponds to or is derived from a naturally occurring antigen. Such naturally occurring antigens may include or may be derived from allergens, viruses, bacteria, fungi, parasites and other infectious agents and pathogens or an antigen may also be a tumor antigen. According to the present disclosure, an antigen may correspond to a naturally occurring product, for example, a viral protein, or a part thereof.

The term "disease-associated antigen" is used in its broadest sense to refer to any antigen associated with a disease. A disease-associated antigen is a molecule which contains epitopes that will stimulate a host's immune system to make a cellular antigen-specific immune response and/or a humoral antibody response against the disease. Disease-associated antigens include pathogen-associated antigens, i.e., antigens which are associated with infection by microbes, typically microbial antigens (such as bacterial or viral antigens), or antigens associated with cancer, typically tumors, such as tumor antigens.

In some embodiments, the antigen is a tumor antigen, i.e., a part of a tumor cell, in particular those which primarily occur intracellularly or as surface antigens of tumor cells. In another embodiment, the antigen is a pathogen-associated antigen, i.e., an antigen derived from a pathogen, e.g., from a virus, bacterium, unicellular organism, or parasite, for example a viral antigen such as viral ribonucleoprotein or coat protein. In some embodiments, the antigen should be presented by MHC molecules which results in modulation, in particular activation of cells of the immune system, such as CD4+ and CD8+ lymphocytes, in particular via the modulation of the activity of a T-cell receptor.

The term "tumor antigen" refers to a constituent of cancer cells which may be derived from the cytoplasm, the cell surface or the cell nucleus. In particular, it refers to those antigens which are produced intracellularly or as surface antigens on tumor cells. For example, tumor antigens include the carcinoembryonal antigen, $\alpha$1-fetoprotein, isoferritin, and fetal sulphoglycoprotein, $\alpha$2-H-ferroprotein and $\gamma$-fetoprotein, as well as various virus tumor antigens. According to some embodiments of the present disclosure, a tumor antigen comprises any antigen which is characteristic for tumors or cancers as well as for tumor or cancer cells with respect to type and/or expression level.

The term "viral antigen" refers to any viral component having antigenic properties, i.e., being able to provoke an immune response in an individual. The viral antigen may be a viral ribonucleoprotein or an envelope protein.

The term "bacterial antigen" refers to any bacterial component having antigenic properties, i.e. being able to provoke an immune response in an individual. The bacterial antigen may be derived from the cell wall or cytoplasm membrane of the bacterium.

The term "epitope" refers to an antigenic determinant in a molecule such as an antigen, i.e., to a part in or fragment of the molecule that is recognized by the immune system, for example, that is recognized by antibodies, T cells or B cells, in particular when presented in the context of MHC molecules. An epitope of a protein may comprises a continuous or discontinuous portion of said protein and, e.g., may be between about 5 and about 100, between about 5 and about 50, between about 8 and about 30, or about 10 and about 25 amino acids in length, for example, the epitope may be preferably 9, 10, 11, 12, 13, 14, 15, 16, 17, 18, 19, 20, 21, 22, 23, 24, or 25 amino acids in length. In some embodiments, the epitope in the context of the present disclosure is a T cell epitope.

Terms such as "epitope", "fragment of an antigen", "immunogenic peptide" and "antigen peptide" are used interchangeably herein and, e.g., may relate to an incomplete representation of an antigen which is, e.g., capable of eliciting an immune response against the antigen or a cell expressing or comprising and presenting the antigen. In some embodiments, the terms relate to an immunogenic portion of an antigen. In some embodiments, it is a portion of an antigen that is recognized (i.e., specifically bound) by a T cell receptor, in particular if presented in the context of MHC molecules. Certain preferred immunogenic portions bind to an MHC class I or class II molecule. The term "epitope" refers to a part or fragment of a molecule such as an antigen that is recognized by the immune system. For example, the epitope may be recognized by T cells, B cells or antibodies. An epitope of an antigen may include a continuous or discontinuous portion of the antigen and may be between about 5 and about 100, such as between about 5 and about 50, between about 8 and about 30, or between about 8 and about 25 amino acids in length, for example, the epitope may be 9, 10, 11, 12, 13, 14, 15,

16, 17, 18, 19, 20, 21, 22, 23, 24, or 25 amino acids in length. In some embodiments, an epitope is between about 10 and about 25 amino acids in length. The term "epitope" includes T cell epitopes.

The term "T cell epitope" refers to a part or fragment of a protein that is recognized by a T cell when presented in the context of MHC molecules. The term "major histocompatibility complex" and the abbreviation "MHC" includes MHC class I and MHC class II molecules and relates to a complex of genes which is present in all vertebrates. MHC proteins or molecules are important for signaling between lymphocytes and antigen presenting cells or diseased cells in immune reactions, wherein the MHC proteins or molecules bind peptide epitopes and present them for recognition by T cell receptors on T cells. The proteins encoded by the MHC are expressed on the surface of cells, and display both self-antigens (peptide fragments from the cell itself) and non-self-antigens (e.g., fragments of invading microorganisms) to a T cell. In the case of class I MHC/peptide complexes, the binding peptides are typically about 8 to about 10 amino acids long although longer or shorter peptides may be effective. In the case of class II MHC/peptide complexes, the binding peptides are typically about 10 to about 25 amino acids long and are in particular about 13 to about 18 amino acids long, whereas longer and shorter peptides may be effective.

The peptide and polypeptide antigen can be 2 to 100 amino acids, including for example, 5 amino acids, 10 amino acids, 15 amino acids, 20 amino acids, 25 amino acids, 30 amino acids, 35 amino acids, 40 amino acids, 45 amino acids, or 50 amino acids in length. In some embodiments, a peptide can be greater than 50 amino acids. In some embodiments, the peptide can be greater than 100 amino acids.

The peptide or polypeptide antigen can be any peptide or polypeptide that can induce or increase the ability of the immune system to develop antibodies and T cell responses to the peptide or polypeptide.

In some embodiments, vaccine antigen, i.e., an antigen whose inoculation into a subject induces an immune response, is recognized by an immune effector cell. In some embodiments, the vaccine antigen if recognized by an immune effector cell is able to induce in the presence of appropriate co-stimulatory signals, stimulation, priming and/or expansion of the immune effector cell carrying an antigen receptor recognizing the vaccine antigen. In the context of the embodiments of the present disclosure, the vaccine antigen may be, e.g., presented or present on the surface of a cell, such as an antigen presenting cell. In some embodiments, an antigen is presented by a diseased cell (such as tumor cell or an infected cell). In some embodiments, an antigen receptor is a TCR which binds to an epitope of an antigen presented in the context of MHC. In some embodiments, binding of a TCR when expressed by T cells and/or present on T cells to an antigen presented by cells such as antigen presenting cells results in stimulation, priming and/or expansion of said T cells. In some embodiments, binding of a TCR when expressed by T cells and/or present on T cells to an antigen presented on diseased cells results in cytolysis and/or apoptosis of the diseased cells, wherein said T cells release cytotoxic factors, e.g., perforins and granzymes.

According to some embodiments, an amino acid sequence enhancing antigen processing and/or presentation is fused, either directly or through the linker sequence, to an antigenic peptide or polypeptide. Accordingly, in some embodiments, the nucleic acid (such as RNA and/or DNA) described herein comprises at least one coding region encoding an antigenic peptide or polypeptide and an amino acid sequence enhancing antigen processing and/or presentation.

Such amino acid sequences enhancing antigen processing and/or presentation are preferably located at the C-terminus of the antigenic peptide or polypeptide and linker sequence (and optionally at the C-terminus of an amino acid sequence which breaks immunological tolerance), without being limited thereto. Amino acid sequences enhancing antigen processing and/or presentation as defined herein preferably improve antigen processing and presentation. In one embodiment, the amino acid sequence enhancing antigen processing and/or presentation as defined herein includes, without being limited thereto, sequences derived from the human MHC class I complex (HLA-B51, haplotype A2, B27/B51, Cw2/Cw3). Besides improving antigen processing and presentation such amino acid sequence enhancing antigen processing and/or presentation may also be used for determining expression of an amino acid sequence in the processes described herein.

Accordingly, in particularly preferred embodiments, the RNA described herein comprises at least one coding region encoding an antigenic peptide or polypeptide and an amino acid sequence enhancing antigen processing and/or presentation, said amino acid sequence enhancing antigen processing and/or presentation preferably being fused to the antigenic peptide or polypeptide, more preferably to the C-terminus of the antigenic peptide or polypeptide as described herein.

Furthermore, a secretory sequence may be fused to the N-terminus of the antigenic peptide or polypeptide.

Amino acid sequences derived from tetanus toxoid of Clostridium tetani may be employed to overcome self-tolerance mechanisms in order to efficiently mount an immune response to self-antigens by providing T-cell help during priming.

It is known that tetanus toxoid heavy chain includes epitopes that can bind promiscuously to MHC class II alleles and induce CD4+ memory T cells in almost all tetanus vaccinated individuals. In addition, the combination of tetanus toxoid (TT) helper epitopes with tumor-associated antigens is known to improve the immune stimulation compared to application of tumor-associated antigen alone by providing CD4+-mediated T-cell help during priming. To reduce the risk of stimulating

CD8+ T cells with the tetanus sequences which might compete with the intended induction of tumor antigen-specific T-cell response, not the whole fragment C of tetanus toxoid is used as it is known to contain CD8+ T-cell epitopes.

According to some embodiments, an amino acid sequence which breaks immunological tolerance is fused, either directly or through a linker to the antigenic peptide or polypeptide.

Such amino acid sequences which break immunological tolerance are preferably located at the C-terminus of the antigenic peptide or polypeptide (and optionally at the N-terminus of the amino acid sequence enhancing antigen processing and/or presentation, wherein the amino acid sequence which breaks immunological tolerance and the amino acid sequence enhancing antigen processing and/or presentation may be fused either directly or through a linker. Amino acid sequences which break immunological tolerance as defined herein preferably improve T cell responses. In one embodiment, the amino acid sequence which breaks immunological tolerance as defined herein includes, without being limited thereto, sequences derived from tetanus toxoid-derived helper sequences p2 and p16 (P2P16).

According to some embodiments, an amino acid sequence which produces bioluminescence is fused, either directly or through a linker to the antigenic peptide or polypeptide.

Such amino acid sequences which produces bioluminescence are preferably located at the C-terminus of the antigenic peptide or polypeptide (and optionally at the N-terminus of (i) the amino acid sequence enhancing antigen processing and/or presentation or (ii) the amino acid sequence which breaks immunological tolerance, wherein the amino acid sequence which produces bioluminescence and (i) the amino acid sequence enhancing antigen processing and/or presentation or (ii) the amino acid sequence which breaks immunological tolerance may be fused either directly or through a linker. Amino acid sequences which produce bioluminescence as defined herein preferably improve the determination of the amount of the antigenic peptide or polypeptide. In some embodiments, the amino acid sequence which produces bioluminescence as defined herein produces fluorescence. In some embodiments, the amino acid sequence which produces bioluminescence as defined herein includes, without being limited thereto, sequences derived from Green Fluorescent Protein (GFP), Yellow Fluorescent Protein (YFP), Red Fluorescent Protein (RFP), Blue Fluorescent Protein (EBFP), Cyan Fluorescent Protein (ECFP), their variants (such as enhanced GFP (EGFP), Superfolder GFP (sfGFP), and luciferase.

In the following, embodiments of vaccine RNAs are described, wherein certain terms used when describing elements thereof have the following meanings:

hAg-Kozak: 5'-UTR sequence of the human alpha-globin mRNA with an optimized 'Kozak sequence' to increase translational efficiency.

sec/MITD: Fusion-protein tags derived from the sequence encoding the human MHC class I complex (HLA-B51, haplotype A2, B27/B51, Cw2/Cw3), which have been shown to improve antigen processing and presentation. Sec corresponds to the 78 bp fragment coding for the secretory signal peptide, which guides translocation of the nascent polypeptide chain into the endoplasmatic reticulum. MITD corresponds to the transmembrane and cytoplasmic domain of the MHC class I molecule, also called MHC class I trafficking domain.

Antigen: Sequences encoding the respective antigen/epitope.

Glycine-serine linker (GS): Sequences coding for linker sequences according to the present invention, which, in an embodiment, are glycine-serine linker sequences, short linker peptides predominantly consisting of the amino acids glycine (G) and serine (S), as commonly used for fusion proteins. In a specific embodiment of the present invention, the linker sequence is preceded at its N terminus by a lysine residue and can be represented as follows: GGSGGGGSGGR/K. Thus, part of the amino acid sequence comprising the linker sequence can be represented as follows: K$\Delta$GGSGGGGSGGR/K ($\Delta$ indicates the proteolytic cleavage site). After cleavage, this results in the excising of the linker sequence as follows: GGSGGGGSGGR/K. In an embodiment, the linker sequences of the present invention are GS linkers each comprising at least one residue which is not G or S, wherein the amino acid residue forms the proteolytic cleavage site of a proteolytic enzyme.

P2P16: Sequence coding for tetanus toxoid-derived helper epitopes to break immunological tolerance.

FI element: The 3'-UTR is a combination of two sequence elements derived from the "amino terminal enhancer of split" (AES) mRNA (called F) and the mitochondrial encoded 12S ribosomal RNA (called I). These were identified by an ex vivo selection process for sequences that confer RNA stability and augment total protein expression.

A30L70: A poly(A)-tail measuring 110 nucleotides in length, consisting of a stretch of 30 adenosine residues, followed by a 10 nucleotide linker sequence and another 70 adenosine residues designed to enhance RNA stability and translational efficiency in dendritic cells.

In one embodiment, vaccine RNA described herein has the structure:
beta-S-ARCA(D1)-hAg-Kozak-sec-GS(1)-Antigen-GS(2)-P2P16-GS(3)-MITD-FI-A30L70

In one embodiment, vaccine antigen described herein has the structure:
sec-GS(1)-Antigen-GS(2)-P2P16-GS(3)-MITD

In one embodiment, there are multiple vaccine antigen RNA constructs (nucleic acids) as described herein comprised in one formulation, such as one particle (LNP, LPX, PLX etc.), wherein each vaccine RNA construct comprises a different linker sequence.

In some embodiments, an antigen receptor is an antibody or B cell receptor which binds to an epitope in an antigen. In some embodiments, an antibody or B cell receptor binds to native epitopes of an antigen.

The term "expressed on the cell surface" or "associated with the cell surface" means that a molecule such as an antigen is associated with and located at the plasma membrane of a cell, wherein at least a part of the molecule faces the extracellular space of said cell and is accessible from the outside of said cell, e.g., by antibodies located outside the cell. In this context, a part may be, e.g., at least 4, at least 8, pat least 12, or at least 20 amino acids. The association may be direct or indirect. For example, the association may be by one or more transmembrane domains, one or more lipid anchors, or by the interaction with any other protein, lipid, saccharide, or other structure that can be found on the outer leaflet of the plasma membrane of a cell. For example, a molecule associated with the surface of a cell may be a transmembrane protein having an extracellular portion or may be a protein associated with the surface of a cell by interacting with another protein that is a transmembrane protein.

"Cell surface" or "surface of a cell" is used in accordance with its normal meaning in the art, and thus includes the outside of the cell which is accessible to binding by proteins and other molecules. An antigen is expressed on the surface of cells if it is located at the surface of said cells and is accessible to binding by, e.g., antigen-specific antibodies added to the cells.

The term "extracellular portion" or "exodomain" in the context of the present disclosure refers to a part of a molecule such as a protein that is facing the extracellular space of a cell and preferably is accessible from the outside of said cell, e.g., by binding molecules such as antibodies located outside the cell. In some embodiments, the term refers to one or more extracellular loops or domains or a fragment thereof.

The terms "T cell" and "T lymphocyte" are used interchangeably herein and include T helper cells (CD4+ T cells) and cytotoxic T cells (CTLs, CD8+ T cells) which comprise cytolytic T cells. The term "antigen-specific T cell" or similar terms relate to a T cell which recognizes the antigen to which the T cell is targeted, in particular when presented on the surface of antigen presenting cells or diseased cells such as cancer cells in the context of MHC molecules and preferably exerts effector functions of T cells. T cells are considered to be specific for antigen if the cells kill target cells expressing an antigen. T cell specificity may be evaluated using any of a variety of standard techniques, for example, within a chromium release assay or proliferation assay. Alternatively, synthesis of lymphokines (such as interferon-$\gamma$) can be measured.

The term "target" shall mean an agent such as a cell or tissue which is a target for an immune response such as a cellular immune response. Targets include cells that present an antigen or an antigen epitope, i.e., a peptide fragment derived from an antigen. In some embodiments, the target cell is a cell expressing an antigen and presenting said antigen with class I MHC.

"Antigen processing" refers to the degradation of an antigen into processing products which are fragments of said antigen (e.g., the degradation of a polypeptide into peptides) and the association of one or more of these fragments (e.g., via binding) with MHC molecules for presentation by cells, such as antigen-presenting cells to specific T-cells.

By "antigen-responsive CTL" is meant a CD8+ T-cell that is responsive to an antigen or a peptide derived from said antigen, which is presented with class I MHC on the surface of antigen presenting cells.

According to the disclosure, CTL responsiveness may include sustained calcium flux, cell division, production of cytokines such as IFN-$\gamma$ and TNF-$\alpha$, up-regulation of activation markers such as CD44 and CD69, and specific cytolytic killing of tumor antigen expressing target cells. CTL responsiveness may also be determined using an artificial reporter that accurately indicates CTL responsiveness.

"Activation" or "stimulation", as used herein, refers to the state of a cell that has been sufficiently stimulated to induce detectable cellular proliferation, such as an immune effector cell such as T cell. Activation can also be associated with initiation of signaling pathways, induced cytokine production, and detectable effector functions. The term "activated immune effector cells" refers to, among other things, immune effector cells that are undergoing cell division.

The term "priming" refers to a process wherein an immune effector cell such as a T cell has its first contact with its specific antigen and causes differentiation into effector cells such as effector T cells.

The term "expansion" refers to a process wherein a specific entity is multiplied. In some embodiments, the term is used in the context of an immunological response in which immune effector cells are stimulated by an antigen, proliferate, and the specific immune effector cell recognizing said antigen is amplified. In some embodiments, expansion leads to differentiation of the immune effector cells.

The terms "immune response" and "immune reaction" are used herein interchangeably in their conventional meaning and refer to an integrated bodily response to an antigen and may refer to a cellular immune response, a humoral immune response, or both. According to the disclosure, the term "immune response to" or "immune response against" with respect to an agent such as an antigen, cell or tissue, relates to an immune response such as a cellular response directed against the agent. An immune response may comprise one or more reactions selected from the group consisting of developing antibodies against one or more antigens and expansion of antigen-specific T-lymphocytes, such as CD4+ and

CD8+ T-lymphocytes, e.g. CD8+ T-lymphocytes, which may be detected in various proliferation or cytokine production tests in vitro.

The terms "inducing an immune response" and "eliciting an immune response" and similar terms in the context of the present disclosure refer to the induction of an immune response, such as the induction of a cellular immune response, a humoral immune response, or both. The immune response may be protective/preventive/prophylactic and/or therapeutic. The immune response may be directed against any immunogen or antigen or antigen peptide, such as against a tumor-associated antigen or a pathogen-associated antigen (e.g., an antigen of a virus (such as influenza virus (A, B, or C), CMV or RSV)). "Inducing" in this context may mean that there was no immune response against a particular antigen or pathogen before induction, but it may also mean that there was a certain level of immune response against a particular antigen or pathogen before induction and after induction said immune response is enhanced. Thus, "inducing the immune response" in this context also includes "enhancing the immune response". In some embodiments, after inducing an immune response in an individual, said individual is protected from developing a disease such as an infectious disease or a cancerous disease or the disease condition is ameliorated by inducing an immune response.

The terms "cellular immune response", "cellular response", "cell-mediated immunity" or similar terms are meant to include a cellular response directed to cells characterized by expression of an antigen and/or presentation of an antigen with class I or class II MHC. The cellular response relates to cells called T cells or T lymphocytes which act as either "helpers" or "killers". The helper T cells (also termed CD4+ T cells) play a central role by regulating the immune response and the killer cells (also termed cytotoxic T cells, cytolytic T cells, CD8+ T cells or CTLs) kill cells such as diseased cells.

The term "humoral immune response" refers to a process in living organisms wherein antibodies are produced in response to agents and organisms, which they ultimately neutralize and/or eliminate. The specificity of the antibody response is mediated by T and/or B cells through membrane-associated receptors that bind antigen of a single specificity. Following binding of an appropriate antigen and receipt of various other activating signals, B lymphocytes divide, which produces memory B cells as well as antibody secreting plasma cell clones, each producing antibodies that recognize the identical antigenic epitope as was recognized by its antigen receptor. Memory B lymphocytes remain dormant until they are subsequently activated by their specific antigen. These lymphocytes provide the cellular basis of memory and the resulting escalation in antibody response when re-exposed to a specific antigen.

The term "antibody" as used herein, refers to an immunoglobulin molecule, which is able to specifically bind to an epitope on an antigen. In particular, the term "antibody" refers to a glycoprotein comprising at least two heavy (H) chains and two light (L) chains inter-connected by disulfide bonds. The term "antibody" includes monoclonal antibodies, recombinant antibodies, human antibodies, humanized antibodies, chimeric antibodies and combinations of any of the foregoing. Each heavy chain is comprised of a heavy chain variable region (VH) and a heavy chain constant region (CH). Each light chain is comprised of a light chain variable region (VL) and a light chain constant region (CL). The variable regions and constant regions are also referred to herein as variable domains and constant domains, respectively. The VH and VL regions can be further subdivided into regions of hypervariability, termed complementarity determining regions (CDRs), interspersed with regions that are more conserved, termed framework regions (FRs). Each VH and VL is composed of three CDRs and four FRs, arranged from amino-terminus to carboxy-terminus in the following order: FR1, CDR1, FR2, CDR2, FR3, CDR3, FR4. The CDRs of a VH are termed HCDR1, HCDR2 and HCDR3, the CDRs of a VL are termed LCDR1, LCDR2 and LCDR3. The variable regions of the heavy and light chains contain a binding domain that interacts with an antigen. The constant regions of an antibody comprise the heavy chain constant region (CH) and the light chain constant region (CL), wherein CH can be further subdivided into constant domain CH1, a hinge region, and constant domains CH2 and CH3 (arranged from amino-terminus to carboxy-terminus in the following order: CH1, CH2, CH3). The constant regions of the antibodies may mediate the binding of the immunoglobulin to host tissues or factors, including various cells of the immune system (e.g., effector cells) and the first component (C1q) of the classical complement system. Antibodies can be intact immunoglobulins derived from natural sources or from recombinant sources and can be immunoactive portions of intact immunoglobulins. Antibodies are typically tetramers of immunoglobulin molecules. Antibodies may exist in a variety of forms including, for example, polyclonal antibodies, monoclonal antibodies, Fv, Fab and F(ab)2, as well as single chain antibodies and humanized antibodies.

The term "immunoglobulin" relates to proteins of the immunoglobulin superfamily, such as to antigen receptors such as antibodies or the B cell receptor (BCR). The immunoglobulins are characterized by a structural domain, i.e., the immunoglobulin domain, having a characteristic immunoglobulin (Ig) fold. The term encompasses membrane bound immunoglobulins as well as soluble immunoglobulins. Membrane bound immunoglobulins are also termed surface immunoglobulins or membrane immunoglobulins, which are generally part of the BCR. Soluble immunoglobulins are generally termed antibodies. Immunoglobulins generally comprise several chains, typically two identical heavy chains and two identical light chains which are linked via disulfide bonds. These chains are primarily composed of immunoglobulin domains, such as the VL (variable light chain) domain, CL (constant light chain) domain, VH (variable heavy chain) domain, and the CH (constant heavy chain) domains CH1, CH2, CH3, and CH4. There are five types of mammalian immunoglobulin heavy chains, i.e., $\alpha$, $\delta$, $\varepsilon$, $\gamma$, and $\mu$ which account for the different classes of antibodies, i.e., IgA, IgD, IgE, IgG, and IgM. As opposed to the heavy chains of soluble immunoglobulins, the heavy chains of membrane or surface

immunoglobulins comprise a transmembrane domain and a short cytoplasmic domain at their carboxy-terminus. In mammals there are two types of light chains, i.e., lambda and kappa. The immunoglobulin chains comprise a variable region and a constant region. The constant region is essentially conserved within the different isotypes of the immunoglobulins, wherein the variable part is highly divers and accounts for antigen recognition.

The terms "vaccination" and "immunization" describe the process of treating an individual for therapeutic or prophylactic reasons and relate to the procedure of administering one or more immunogen(s) or antigen(s) or derivatives thereof, in particular in the form of RNA (especially mRNA) coding therefor, as described herein to an individual and stimulating an immune response against said one or more immunogen(s) or antigen(s) or cells characterized by presentation of said one or more immunogen(s) or antigen(s).

By "cell characterized by presentation of an antigen" or "cell presenting an antigen" or "MHC molecules which present an antigen on the surface of an antigen presenting cell" or similar expressions is meant a cell such as a diseased cell, in particular a tumor cell or an infected cell, or an antigen presenting cell presenting the antigen or an antigen peptide, either directly or following processing, in the context of MHC molecules, such as MHC class I and/or MHC class II molecules. In some embodiments, the MHC molecules are MHC class I molecules.

The term "allergen" refers to a kind of antigen which originates from outside the body of a subject (i.e., the allergen can also be called "heterologous antigen") and which produces an abnormally vigorous immune response in which the immune system of the subject fights off a perceived threat that would otherwise be harmless to the subject. "Allergies" are the diseases caused by such vigorous immune reactions against allergens. An allergen usually is an antigen which is able to stimulate a type-I hypersensitivity reaction in atopic individuals through immunoglobulin E (IgE) responses. Particular examples of allergens include allergens derived from peanut proteins (e.g., Ara h 2.02), ovalbumin, grass pollen proteins (e.g., Phl p 5), and proteins of dust mites (e.g., Der p 2).

The term "growth factors" refers to molecules which are able to stimulate cellular growth, proliferation, healing, and/or cellular differentiation. Typically, growth factors act as signaling molecules between cells. The term "growth factors" include particular cytokines and hormones which bind to specific receptors on the surface of their target cells. Examples of growth factors include bone morphogenetic proteins (BMPs), fibroblast growth factors (FGFs), vascular endothelial growth factors (VEGFs), such as VEGFA, epidermal growth factor (EGF), insulin-like growth factor, ephrins, macrophage colony-stimulating factor, granulocyte colony-stimulating factor, granulocyte macrophage colony-stimulating factor, neuregulins, neurotrophins (e.g., brain-derived neurotrophic factor (BDNF), nerve growth factor (NGF)), placental growth factor (PGF), platelet-derived growth factor (PDGF), renalase (RNLS) (anti-apoptotic survival factor), T-cell growth factor (TCGF), thrombopoietin (TPO), transforming growth factors (transforming growth factor alpha (TGF-$\alpha$), transforming growth factor beta (TGF-$\beta$)), and tumor necrosis factor-alpha (TNF-$\alpha$). In some embodiments, a "growth factor" is a peptide or polypeptide growth factor.

The term "protease inhibitors" refers to molecules, in particular peptides or polypeptides, which inhibit the function of proteases. Protease inhibitors can be classified by the protease which is inhibited (e.g., aspartic protease inhibitors) or by their mechanism of action (e.g., suicide inhibitors, such as serpins). Particular examples of protease inhibitors include serpins, such as alpha 1-antitrypsin, aprotinin, and bestatin.

The term "enzymes" refers to macromolecular biological catalysts which accelerate chemical reactions. Like any catalyst, enzymes are not consumed in the reaction they catalyze and do not alter the equilibrium of said reaction. Unlike many other catalysts, enzymes are much more specific. In some embodiments, an enzyme is essential for homeostasis of a subject, e.g., any malfunction (in particular, decreased activity which may be caused by any of mutation, deletion or decreased production) of the enzyme results in a disease. Examples of enzymes include herpes simplex virus type 1 thymidine kinase (HSV1-TK), hexosaminidase, phenylalanine hydroxylase, pseudocholinesterase, and lactase.

The term "receptors" refers to protein molecules which receive signals (in particular chemical signals called ligands) from outside a cell. The binding of a signal (e.g., ligand) to a receptor causes some kind of response of the cell, e.g., the intracellular activation of a kinase. Receptors include transmembrane receptors (such as ion channel-linked (ionotropic) receptors, G protein-linked (metabotropic) receptors, and enzyme-linked receptors) and intracellular receptors (such as cytoplasmic receptors and nuclear receptors). Particular examples of receptors include steroid hormone receptors, growth factor receptors, and peptide receptors (i.e., receptors whose ligands are peptides), such as P-selectin glycoprotein ligand-1 (PSGL-1). The term "growth factor receptors" refers to receptors which bind to growth factors.

The term "apoptosis regulators" refers to molecules, in particular peptides or polypeptides, which modulate apoptosis, i.e., which either activate or inhibit apoptosis. Apoptosis regulators can be grouped into two broad classes: those which modulate mitochondrial function and those which regulate caspases. The first class includes proteins (e.g., BCL-2, BCL-xL) which act to preserve mitochondrial integrity by preventing loss of mitochondrial membrane potential and/or release of pro-apoptotic proteins such as cytochrome C into the cytosol. Also to this first class belong proapoptotic proteins (e.g., BAX, BAK, BIM) which promote release of cytochrome C. The second class includes proteins such as the inhibitors of apoptosis proteins (e.g., XIAP) or FLIP which block the activation of caspases.

The term "transcription factors" relates to proteins which regulate the rate of transcription of genetic information from DNA to messenger RNA, in particular by binding to a specific DNA sequence. Transcription factors may regulate cell

division, cell growth, and cell death throughout life; cell migration and organization during embryonic development; and/or in response to signals from outside the cell, such as a hormone. Transcription factors contain at least one DNA-binding domain which binds to a specific DNA sequence, usually adjacent to the genes which are regulated by the transcription factors. Particular examples of transcription factors include MECP2, FOXP2, FOXP3, the STAT protein family, and the HOX protein family.

The term "tumor suppressor proteins" relates to molecules, in particular peptides or polypeptides, which protect a cell from one step on the path to cancer. Tumor-suppressor proteins (usually encoded by corresponding tumor-suppressor genes) exhibit a weakening or repressive effect on the regulation of the cell cycle and/or promote apoptosis. Their functions may be one or more of the following: repression of genes essential for the continuing of the cell cycle; coupling the cell cycle to DNA damage (as long as damaged DNA is present in a cell, no cell division should take place); initiation of apoptosis, if the damaged DNA cannot be repaired; metastasis suppression (e.g., preventing tumor cells from dispersing, blocking loss of contact inhibition, and inhibiting metastasis); and DNA repair. Particular examples of tumor-suppressor proteins include p53, phosphatase and tensin homolog (PTEN), SWI/SNF (SWItch/Sucrose Non-Fermentable), von Hippel-Lindau tumor suppressor (pVHL), adenomatous polyposis coli (APC), CD95, suppression of tumorigenicity 5 (ST5), suppression of tumorigenicity 5 (ST5), suppression of tumorigenicity 14 (ST14), and Yippee-like 3 (YPEL3).

The term "structural proteins" refers to proteins which confer stiffness and rigidity to otherwise-fluid biological components. Structural proteins are mostly fibrous (such as collagen and elastin) but may also be globular (such as actin and tubulin). Usually, globular proteins are soluble as monomers, but polymerize to form long, fibers which, for example, may make up the cytoskeleton. Other structural proteins are motor proteins (such as myosin, kinesin, and dynein) which are capable of generating mechanical forces, and surfactant proteins. Particular examples of structural proteins include collagen, surfactant protein A, surfactant protein B, surfactant protein C, surfactant protein D, elastin, tubulin, actin, and myosin.

The term "reprogramming factors" or "reprogramming transcription factors" relates to molecules, in particular peptides or polypeptides, which, when expressed in somatic cells optionally together with further agents such as further reprogramming factors, lead to reprogramming or de-differentiation of said somatic cells to cells having stem cell characteristics, in particular pluripotency. Particular examples of reprogramming factors include OCT4, SOX2, c-MYC, KLF4, LIN28, and NANOG.

The term "genomic engineering proteins" relates to proteins which are able to insert, delete or replace DNA in the genome of a subject. Particular examples of genomic engineering proteins include meganucleases, zinc finger nucleases (ZFNs), transcription activator-like effector nucleases (TALENs), and clustered regularly spaced short palindromic repeat-CRISPR-associated protein 9 (CRISPR-Cas9).

The term "blood proteins" relates to peptides or polypeptides which are present in blood plasma of a subject, in particular blood plasma of a healthy subject. Blood proteins have diverse functions such as transport (e.g., albumin, transferrin), enzymatic activity (e.g., thrombin or ceruloplasmin), blood clotting (e.g., fibrinogen), defense against pathogens (e.g., complement components and immunoglobulins), protease inhibitors (e.g., alpha 1-antitrypsin), etc. Particular examples of blood proteins include thrombin, serum albumin, Factor VII, Factor VIII, insulin, Factor IX, Factor X, tissue plasminogen activator, protein C, von Willebrand factor, antithrombin III, glucocerebrosidase, erythropoietin, granulocyte colony stimulating factor (G-CSF), modified Factor VIII, and anticoagulants.

Thus, in some embodiments, the pharmaceutically active peptide or polypeptide is (i) a cytokine, preferably selected from the group consisting of erythropoietin (EPO), interleukin 4 (IL-2), and interleukin 10 (IL-11), more preferably EPO; (ii) an adhesion molecule, in particular an integrin; (iii) an immunoglobulin, in particular an antibody; (iv) an immunologically active compound, in particular an antigen; (v) a hormone, in particular vasopressin, insulin or growth hormone; (vi) a growth factor, in particular VEGFA; (vii) a protease inhibitor, in particular alpha 1-antitrypsin; (viii) an enzyme, preferably selected from the group consisting of herpes simplex virus type 1 thymidine kinase (HSV1-TK), hexosaminidase, phenylalanine hydroxylase, pseudocholinesterase, pancreatic enzymes, and lactase; (ix) a receptor, in particular growth factor receptors; (x) an apoptosis regulator, in particular BAX; (xi) a transcription factor, in particular FOXP3; (xii) a tumor suppressor protein, in particular p53; (xiii) a structural protein, in particular surfactant protein B; (xiv) a reprogramming factor, e.g., selected from the group consisting of OCT4, SOX2, c-MYC, KLF4, LIN28 and NANOG; (xv) a genomic engineering protein, in particular clustered regularly spaced short palindromic repeat-CRISPR-associated protein 9 (CRISPR-Cas9); and (xvi) a blood protein, in particular fibrinogen.

In some embodiments, a pharmaceutically active peptide or polypeptide comprises one or more antigens or one or more epitopes, i.e., administration of the peptide or polypeptide to a subject elicits an immune response against the one or more antigens or one or more epitopes in a subject which may be therapeutic or partially or fully protective.

In some embodiments, the nucleic acid such as mRNA encodes at least one epitope.

In some embodiments, the epitope is derived from a tumor antigen. The tumor antigen may be a "standard" antigen, which is generally known to be expressed in various cancers. The tumor antigen may also be a "neo-antigen", which is specific to an individual's tumor and has not been previously recognized by the immune system. A neo-antigen or neo-epitope may result from one or more cancer-specific mutations in the genome of cancer cells resulting in amino acid

changes. Examples of tumor antigens include, without limitation, p53, ART-4, BAGE, beta-catenin/m, Bcr-abL CAMEL, CAP-1 , CASP-8, CDC27/m, CDK4/m, CEA, the cell surface proteins of the claudin family, such as CLAUD ΓN-6, CLAUDIN-18.2 and CLAUDIN-12, c-MYC, CT, Cyp-B, DAM, ELF2M, ETV6-AML1, G250, GAGE, GnT-V, Gap 100, HAGE, HER-2/neu, HPV-E7, HPV-E6, HAST-2, hTERT (or hTRT), LAGE, LDLR/FUT, MAGE-A, preferably MAGE-A1 , MAGE-A2, MAGE- A3, MAGE-A4, MAGE-A5, MAGE-A6, MAGE-A7, MAGE-A8, MAGE-A9, MAGE-A 10, MAGE-A 11, or MAGE-A12, MAGE-B, MAGE-C, MART- 1 /Melan-A, MC1R, Myosin/m, MUC1, MUM-1, MUM-2, MUM-3, NA88-A, NF1 , NY-ESO-1 , NY-BR-1 , pl90 minor BCR-abL, Pml/RARa, PRAME, proteinase 3, PSA, PSM, RAGE, RU1 or RU2, SAGE, SART-1 or SART-3, SCGB3A2, SCP1 , SCP2, SCP3, SSX, SURVIVIN, TEL/AML1 , TPI/m, TRP-1 , TRP-2, TRP-2/INT2, TPTE, WT, and WT-1.

Cancer mutations vary with each individual. Thus, cancer mutations that encode novel epitopes (neo-epitopes) represent attractive targets in the development of vaccine compositions and immunotherapies. The efficacy of tumor immunotherapy relies on the selection of cancer-specific antigens and epitopes capable of inducing a potent immune response within a host. RNA can be used to deliver patient-specific tumor epitopes to a patient. Dendritic cells (DCs) residing in the spleen represent antigen-presenting cells of particular interest for RNA expression of immunogenic epitopes or antigens such as tumor epitopes. The use of multiple epitopes has been shown to promote therapeutic efficacy in tumor vaccine compositions. Rapid sequencing of the tumor mutanome may provide multiple epitopes for individualized vaccines which can be encoded by mRNA described herein, e.g., as a single polypeptide wherein the epitopes are optionally separated by linkers. In some embodiments of the present disclosure, the mRNA encodes at least one epitope, at least two epitopes, at least three epitopes, at least four epitopes, at least five epitopes, at least six epitopes, at least seven epitopes, at least eight epitopes, at least nine epitopes, or at least ten epitopes. Exemplary embodiments include mRNA that encodes at least five epitopes (termed a "pentatope") and mRNA that encodes at least ten epitopes (termed a "decatope").

In some embodiments, the antigen or epitope is derived from a pathogen-associated antigen, in particular from a viral antigen. In some embodiments, the antigen or epitope is derived from a SARS-CoV-2 S protein, an immunogenic variant thereof, or an immunogenic fragment of the SARS-CoV-2 S protein or the immunogenic variant thereof. Thus, in some embodiments, the mRNA used in the present disclosure encodes an amino acid sequence comprising a SARS-CoV-2 S protein, an immunogenic variant thereof, or an immunogenic fragment of the SARS-CoV-2 S protein or the immunogenic variant thereof.

In some embodiments of the present disclosure the antigen (such as a tumor antigen or vaccine antigen) is preferably administered as single-stranded, 5' capped mRNA that is translated into the respective protein upon entering cells of a subject being administered the RNA. Preferably, the RNA contains structural elements optimized for maximal efficacy of the RNA with respect to stability and translational efficiency (5' cap, 5' UTR, 3' UTR, poly(A) sequence).

In some embodiments, beta-S-ARCA(D1) is utilized as specific capping structure at the 5'-end of the mRNA. In some embodiments, m27,3'-OGppp(m12'-O) ApG is utilized as specific capping structure at the 5'-end of the mRNA. In some embodiments, the 5'-UTR sequence is derived from the human alpha-globin mRNA and optionally has an optimized 'Kozak sequence' to increase translational efficiency. In some embodiments, a combination of two sequence elements (FI element) derived from the "amino terminal enhancer of split" (AES) mRNA (called F) and the mitochondrial encoded 12S ribosomal RNA (called I) are placed between the coding sequence and the poly(A) sequence to assure higher maximum protein levels and prolonged persistence of the mRNA. In some embodiments, two re-iterated 3'-UTRs derived from the human beta-globin mRNA are placed between the coding sequence and the poly(A) sequence to assure higher maximum protein levels and prolonged persistence of the mRNA. In some embodiments, a poly(A) sequence measuring 110 nucleotides in length, consisting of a stretch of 30 adenosine residues, followed by a 10 nucleotide linker sequence and another 70 adenosine residues is used. This poly(A) sequence was designed to enhance RNA stability and translational efficiency.

In some embodiments, mRNA encoding an antigen (such as a tumor antigen or a vaccine antigen) is expressed in cells of the subject treated to provide the antigen. In some embodiments, the mRNA is transiently expressed in cells of the subject. In some embodiments, the mRNA is in vitro transcribed. In some embodiments, expression of the antigen is at the cell surface. In some embodiments, the antigen is expressed and presented in the context of MHC. In some embodiments, expression of the antigen is into the extracellular space, i.e., the antigen is secreted.

The antigen molecule or a procession product thereof, e.g., a fragment thereof, may bind to an antigen receptor such as a BCR or TCR carried by immune effector cells, or to antibodies.

A peptide and polypeptide antigen which is provided to a subject according to the present disclosure by administering mRNA encoding a peptide and polypeptide antigen, wherein the antigen is a vaccine antigen, preferably results in the induction of an immune response, e.g., a humoral and/or cellular immune response in the subject being provided the peptide or polypeptide antigen. Said immune response is preferably directed against a target antigen. Thus, a vaccine antigen may comprise the target antigen, a variant thereof, or a fragment thereof. In some embodiments, such fragment or variant is immunologically equivalent to the target antigen. In the context of the present disclosure, the term "fragment of an antigen" or "variant of an antigen" means an agent which results in the induction of an immune response

which immune response targets the antigen, i.e. a target antigen. Thus, the vaccine antigen may correspond to or may comprise the target antigen, may correspond to or may comprise a fragment of the target antigen or may correspond to or may comprise an antigen which is homologous to the target antigen or a fragment thereof. Thus, according to the present disclosure, a vaccine antigen may comprise an immunogenic fragment of a target antigen or an amino acid sequence being homologous to an immunogenic fragment of a target antigen. An "immunogenic fragment of an antigen" according to the disclosure preferably relates to a fragment of an antigen which is capable of inducing an immune response against the target antigen. The vaccine antigen may be a recombinant antigen.

The term "immunologically equivalent" means that the immunologically equivalent molecule such as the immunologically equivalent amino acid sequence exhibits the same or essentially the same immunological properties and/or exerts the same or essentially the same immunological effects, e.g., with respect to the type of the immunological effect. In the context of the present disclosure, the term "immunologically equivalent" is preferably used with respect to the immunological effects or properties of antigens or antigen variants used for immunization. For example, an amino acid sequence is immunologically equivalent to a reference amino acid sequence if said amino acid sequence when exposed to the immune system of a subject induces an immune reaction having a specificity of reacting with the reference amino acid sequence.

In some embodiments, the mRNA used in the present disclosure is non-immunogenic. RNA encoding an immunostimulant may be administered according to the present disclosure to provide an adjuvant effect. The RNA encoding an immunostimulant may be standard RNA or non-immunogenic RNA.

The term "non-immunogenic RNA" (such as "non-immunogenic mRNA") as used herein refers to RNA that does not induce a response by the immune system upon administration, e.g., to a mammal, or induces a weaker response than would have been induced by the same RNA that differs only in that it has not been subjected to the modifications and treatments that render the non-immunogenic RNA non-immunogenic, i.e., than would have been induced by standard RNA (stdRNA). In certain embodiments, non-immunogenic RNA, which is also termed modified RNA (modRNA) herein, is rendered non-immunogenic by incorporating modified nucleosides suppressing RNA-mediated activation of innate immune receptors into the RNA and/or removing double-stranded RNA (dsRNA).

For rendering the non-immunogenic RNA (especially mRNA) non-immunogenic by the incorporation of modified nucleosides, any modified nucleoside may be used as long as it lowers or suppresses immunogenicity of the RNA. Particularly preferred are modified nucleosides that suppress RNA-mediated activation of innate immune receptors. In some embodiments, the modified nucleosides comprise a replacement of one or more uridines with a nucleoside comprising a modified nucleobase. In some embodiments, the modified nucleobase is a modified uracil. In some embodiments, the nucleoside comprising a modified nucleobase is selected from the group consisting of 3-methyl-uridine (m3U), 5-methoxy-uridine (mo5U), 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho5U), 5-aminoallyl-uridine, 5-halo-uridine (e.g., 5-iodo-uridine or 5-bromo-uridine), uridine 5-oxyacetic acid (cmo5U), uridine 5-oxyacetic acid methyl ester (mcmo5U), 5-carboxymethyl-uridine (cm5U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mcm5U), 5-methoxycarbonylmethyl-2-thio-uridine (mcm5s2U), 5-aminomethyl-2-thio-uridine (nm5s2U), 5-methylaminomethyl-uridine (mnm5U), 1-ethyl-pseudouridine, 5-methylaminomethyl-2-thio-uridine (mnm5s2U), 5-methylaminomethyl-2-seleno-uridine (mnm5se2U), 5-carbamoylmethyl-uridine (ncm5U), 5-carboxymethylaminomethyl-uridine (cmnm5U), 5-carboxymethylaminomethyl-2-thio-uridine (cmnm5s2U), 5-propynyl-uridine, 1-propynyl-pseudouridine, 5-taurinomethyl-uridine (τm5U), 1-taurinomethyl-pseudouridine, 5-taurinomethyl-2-thio-uridine(τm5s2U), 1-taurinomethyl-4-thio-pseudouridine), 5-methyl-2-thio-uridine (m5s2U), 1-methyl-4-thio-pseudouridine (m1s4ψ), 4-thio-1-methyl-pseudouridine, 3-methyl-pseudouridine (m3ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m5D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N 1-methyl-pseudouridine, 3-(3-amino-3-carboxypropyl)uridine (acp3U), 1-methyl-3-(3-amino-3-carboxypropyl)pseudouridine (acp3 ψ), 5-(isopentenylaminomethyl)uridine (inm5U), 5-(isopentenylaminomethyl)-2-thio-uridine (inm5s2U), α-thio-uridine, 2'-O-methyl-uridine (Um), 5,2'-O-dimethyl-uridine (m5Um), 2'-O-methyl-pseudouridine (ψm), 2-thio-2'-O-methyl-uridine (s2Um), 5-methoxycarbonylmethyl-2'-O-methyl-uridine (mcm5Um), 5-carbamoylmethyl-2'-O-methyl-uridine (ncm5Um), 5-carboxymethylaminomethyl-2'-O-methyl-uridine (cmnm5Um), 3,2'-O-dimethyl-uridine (m3Um), 5-(isopentenylaminomethyl)-2'-O-methyl-uridine (inm5Um), 1-thio-uridine, deoxythymidine, 2'-F-ara-uridine, 2'-F-uridine, 2'-OH-ara-uridine, 5-(2-carbomethoxyvinyl) uridine, and 5-[3-(1-E-propenylamino)uridine. In certain embodiments, the nucleoside comprising a modified nucleobase is pseudouridine (ψ), N1-methyl-pseudouridine (m1ψ) or 5-methyl-uridine (m5U), in particular N1-methyl-pseudouridine.

In some embodiments, the replacement of one or more uridines with a nucleoside comprising a modified nucleobase comprises a replacement of at least 1%, at least 2%, at least 3%, at least 4%, at least 5%, at least 10%, at least 25%, at least 50%, at least 75%, at least 90%, at least 95%, at least 96%, at least 97%, at least 98%, at least 99% or 100% of the uridines.

During synthesis of mRNA by in vitro transcription (IVT) using T7 RNA polymerase significant amounts of aberrant products, including double-stranded RNA (dsRNA) are produced due to unconventional activity of the enzyme. dsRNA induces inflammatory cytokines and activates effector enzymes leading to protein synthesis inhibition. dsRNA can be removed from RNA such as IVT RNA, for example, by ion-pair reversed phase HPLC using a non-porous or porous C-18 polystyrene-divinylbenzene (PS-DVB) matrix. Alternatively, an enzymatic based method using E. coli RNaseIII that specifically hydrolyzes dsRNA but not ssRNA, thereby eliminating dsRNA contaminants from IVT RNA preparations can be used. Furthermore, dsRNA can be separated from ssRNA by using a cellulose material. In some embodiments, an RNA preparation is contacted with a cellulose material and the ssRNA is separated from the cellulose material under conditions which allow binding of dsRNA to the cellulose material and do not allow binding of ssRNA to the cellulose material. Suitable methods for providing ssRNA are disclosed, for example, in WO 2017/182524.

As the term is used herein, "remove" or "removal" refers to the characteristic of a population of first substances, such as non-immunogenic RNA, being separated from the proximity of a population of second substances, such as dsRNA, wherein the population of first substances is not necessarily devoid of the second substance, and the population of second substances is not necessarily devoid of the first substance. However, a population of first substances characterized by the removal of a population of second substances has a measurably lower content of second substances as compared to the non-separated mixture of first and second substances.

In some embodiments, the removal of dsRNA (especially mRNA) from non-immunogenic RNA comprises a removal of dsRNA such that less than 10%, less than 5%, less than 4%, less than 3%, less than 2%, less than 1%, less than 0.5%, less than 0.3%, or less than 0.1% of the RNA in the non-immunogenic RNA composition is dsRNA. In some embodiments, the non-immunogenic RNA (especially mRNA) is free or essentially free of dsRNA. In some embodiments, the non-immunogenic RNA (especially mRNA) composition comprises a purified preparation of single-stranded nucleoside modified RNA. For example, in some embodiments, the purified preparation of single-stranded nucleoside modified RNA (especially mRNA) is substantially free of double stranded RNA (dsRNA). In some embodiments, the purified preparation is at least 90%, at least 91%, at least 92%, at least 93 %, at least 94%, at least 95%, at least 96%, at least 97%, at least 98%, at least 99%, at least 99.5%, or at least 99.9% single stranded nucleoside modified RNA, relative to all other nucleic acid molecules (DNA, dsRNA, etc.).

In some embodiments, the non-immunogenic RNA (especially mRNA) is translated in a cell more efficiently than standard RNA with the same sequence. In some embodiments, translation is enhanced by a factor of 2-fold relative to its unmodified counterpart. In some embodiments, translation is enhanced by a 3-fold factor. In some embodiments, translation is enhanced by a 4-fold factor. In some embodiments, translation is enhanced by a 5-fold factor. In some embodiments, translation is enhanced by a 6-fold factor. In some embodiments, translation is enhanced by a 7-fold factor. In some embodiments, translation is enhanced by an 8-fold factor. In some embodiments, translation is enhanced by a 9-fold factor. In some embodiments, translation is enhanced by a 10-fold factor. In some embodiments, translation is enhanced by a 15-fold factor. In some embodiments, translation is enhanced by a 20-fold factor. In some embodiments, translation is enhanced by a 50-fold factor. In some embodiments, translation is enhanced by a 100-fold factor. In some embodiments, translation is enhanced by a 200-fold factor. In some embodiments, translation is enhanced by a 500-fold factor. In some embodiments, translation is enhanced by a 1000-fold factor. In some embodiments, translation is enhanced by a 2000-fold factor. In some embodiments, the factor is 10-1000-fold. In some embodiments, the factor is 10-100-fold. In some embodiments, the factor is 10-200-fold. In some embodiments, the factor is 10-300-fold. In some embodiments, the factor is 10-500-fold. In some embodiments, the factor is 20-1000-fold. In some embodiments, the factor is 30-1000-fold. In some embodiments, the factor is 50-1000-fold. In some embodiments, the factor is 100-1000-fold. In some embodiments, the factor is 200-1000-fold. In some embodiments, translation is enhanced by any other significant amount or range of amounts.

In some embodiments, the non-immunogenic RNA (especially mRNA) exhibits significantly less innate immunogenicity than standard RNA with the same sequence. In some embodiments, the non-immunogenic RNA (especially mRNA) exhibits an innate immune response that is 2-fold less than its unmodified counterpart. In some embodiments, innate immunogenicity is reduced by a 3-fold factor. In some embodiments, innate immunogenicity is reduced by a 4-fold factor. In some embodiments, innate immunogenicity is reduced by a 5-fold factor. In some embodiments, innate immunogenicity is reduced by a 6-fold factor. In some embodiments, innate immunogenicity is reduced by a 7-fold factor. In some embodiments, innate immunogenicity is reduced by a 8-fold factor. In some embodiments, innate immunogenicity is reduced by a 9-fold factor. In some embodiments, innate immunogenicity is reduced by a 10-fold factor. In some embodiments, innate immunogenicity is reduced by a 15-fold factor. In some embodiments, innate immunogenicity is reduced by a 20-fold factor. In some embodiments, innate immunogenicity is reduced by a 50-fold factor. In some embodiments, innate immunogenicity is reduced by a 100-fold factor. In some embodiments, innate immunogenicity is reduced by a 200-fold factor. In some embodiments, innate immunogenicity is reduced by a 500-fold factor. In some embodiments, innate immunogenicity is reduced by a 1000-fold factor. In some embodiments, innate immunogenicity is reduced by a 2000-fold factor.

The term "exhibits significantly less innate immunogenicity" refers to a detectable decrease in innate immunogenicity. In some embodiments, the term refers to a decrease such that an effective amount of the non-immunogenic RNA (especially mRNA) can be administered without triggering a detectable innate immune response. In some embodiments, the term refers to a decrease such that the non-immunogenic RNA (especially mRNA) can be repeatedly administered without eliciting an innate immune response sufficient to detectably reduce production of the protein encoded by the non-immunogenic RNA. In some embodiments, the decrease is such that the non-immunogenic RNA (especially mRNA) can be repeatedly administered without eliciting an innate immune response sufficient to eliminate detectable production of the protein encoded by the non-immunogenic RNA.

"Immunogenicity" is the ability of a foreign substance, such as RNA, to provoke an immune response in the body of a human or other animal. The innate immune system is the component of the immune system that is relatively unspecific and immediate. It is one of two main components of the vertebrate immune system, along with the adaptive immune system.

**Particles**

The composition comprising RNA of the present invention, or the RNA obtained in the present invention, may be present in particles comprising (i) the RNA, and (ii) at least one cationic or cationically ionizable compound such as a polymer or lipid complexing the RNA. Electrostatic interactions between positively charged molecules such as polymers and lipids and negatively charged nucleic acid are involved in particle formation. This results in complexation and spontaneous formation of nucleic acid particles comprising the RNA.

Different types of RNA containing particles have been described previously to be suitable for delivery of RNA in particulate form (cf., *e.g.*, Kaczmarek, J. C. et al., 2017, Genome Medicine 9, 60). For non-viral RNA delivery vehicles, nanoparticle encapsulation of RNA physically protects RNA from degradation and, depending on the specific chemistry, can aid in cellular uptake and endosomal escape.

In the context of the present disclosure, the term "particle" relates to a structured entity formed by molecules or molecule complexes, in particular particle forming compounds. In some embodiments, the particle contains an envelope (*e.g.*, one or more layers or lamellas) made of one or more types of amphiphilic substances (*e.g.*, amphiphilic lipids). In this context, the expression "amphiphilic substance" means that the substance possesses both hydrophilic and lipophilic properties. The envelope may also comprise additional substances (*e.g.*, additional lipids) which do not have to be amphiphilic. Thus, the particle may be a monolamellar or multilamellar structure, wherein the substances constituting the one or more layers or lamellas comprise one or more types of amphiphilic substances (in particular selected from the group consisting of amphiphilic lipids) optionally in combination with additional substances (*e.g.*, additional lipids) which do not have to be amphiphilic. In some embodiments, the term "particle" relates to a micro- or nano-sized structure, such as a micro- or nano-sized compact structure. According to the present disclosure, the term "particle" includes nanoparticles.

An "RNA particle" can be used to deliver RNA to a target site of interest (*e.g.*, cell, tissue, organ, and the like). An RNA particle may be formed from lipids comprising at least one cationic or cationically ionizable lipid or lipid-like material. Without intending to be bound by any theory, it is believed that the cationic or cationically ionizable lipid or lipid-like material combines together with the RNA to form aggregates, and this aggregation results in colloidally stable particles.

Nucleic acid particles (such RNA particles) include lipid nanoparticle (LNP)-based and lipoplex (LPX)-based formulations.

In general, a lipoplex (LPX) is obtainable from mixing two aqueous phases, namely a phase comprising nucleic acid (such as RNA and/or DNA) and a phase comprising a dispersion of lipids. In some embodiments, the lipid phase comprises liposomes.

In some embodiments, liposomes are self-closed unilamellar or multilamellar vesicular particles wherein the lamellae comprise lipid bilayers and the encapsulated lumen comprises an aqueous phase. A prerequisite for using liposomes for nanoparticle formation is that the lipids in the mixture as required are able to form lamellar (bilayer) phases in the applied aqueous environment.

In some embodiments, liposomes comprise unilamellar or multilamellar phospholipid bilayers enclosing an aqueous core (also referred to herein as an aqueous lumen). They may be prepared from materials possessing polar head (hydrophilic) groups and nonpolar tail (hydrophobic) groups. In some embodiments, cationic lipids employed in formulating liposomes designed for the delivery of nucleic acids are amphiphilic in nature and consist of a positively charged (cationic) amine head group linked to a hydrocarbon chain or cholesterol derivative via glycerol.

In some embodiments, lipoplexes are multilamellar liposome-based formulations that form upon electrostatic interaction of cationic liposomes with nucleic acids (such as RNAs and/or DNAs). In some embodiments, formed lipoplexes possess distinct internal arrangements of molecules that arise due to the transformation from liposomal structure into compact nucleic acid-lipoplexes (such as RNA- and/or DNA-lipoplexes). In some embodiments, these formulations are characterized by their poor encapsulation of the nucleic acid (such as RNA) and incomplete entrapment of the nucleic acid (such as RNA).

In some embodiments, an LPX particle comprises an amphiphilic lipid, in particular cationic or cationically

ionizable amphiphilic lipid, and nucleic acid (such as RNA and/or DNA, especially mRNA) as described herein. In some embodiments, electrostatic interactions between positively charged liposomes (made from one or more amphiphilic lipids, in particular cationic or cationically ionizable amphiphilic lipids) and negatively charged nucleic acid (especially mRNA) results in complexation and spontaneous formation of nucleic acid lipoplex particles. Positively charged liposomes may be generally synthesized using a cationic or cationically ionizable amphiphilic lipid, such as DOTMA and/or DODMA, and additional lipids, such as DOPE. In some embodiments, a nucleic acid (such as RNA and/or DNA, especially mRNA) lipoplex particle is a nanoparticle.

In general, a lipid nanoparticle (LNP) is obtainable from direct mixing of nucleic acid (such as RNA and/or DNA) in an aqueous phase with lipids in a phase comprising an organic solvent, such as ethanol. In that case, lipids or lipid mixtures can be used for particle formation, which do not form lamellar (bilayer) phases in water.

In some embodiments, LNPs comprise or consist of a cationic/ionizable lipid and helper lipids such as phospholipids, cholesterol, and/or polyethylene glycol (PEG) lipids. In some embodiments, in the nucleic acid LNPs (such as RNA LNPs, e.g., mRNA LNPs) described herein the nucleic acid (such as RNA, e.g., mRNA) is bound by ionizable lipid that occupies the central core of the LNP. In some embodiments, PEG lipid forms the surface of the LNP, along with phospholipids. In some embodiments, the surface comprises a bilayer. In some embodiments, cholesterol and ionizable lipid in charged and uncharged forms can be distributed throughout the LNP.

In some embodiments, nucleic acid (such as RNA and/or DNA, *e.g.*, mRNA) may be noncovalently associated with a particle as described herein. In embodiments, the nucleic acid (such as RNA and/or DNA, especially mRNA) may be adhered to the outer surface of the particle (surface nucleic acid (such as surface RNA, especially surface mRNA)) and/or may be contained in the particle (encapsulated nucleic acid (such as encapsulated RNA, especially encapsulated mRNA)).

In some embodiments, the particles (e.g., LNPs and LPXs) described herein have a size (such as a diameter) in the range of about 10 to about 2000 nm, such as at least about 15 nm (e.g., at least about 20 nm, at least about 25 nm, at least about 30 nm, at least about 35 nm, at least about 40 nm, at least about 45 nm, at least about 50 nm, at least about 55 nm, at least about 60 nm, at least about 65 nm, at least about 70 nm, at least about 75 nm, at least about 80 nm, at least about 85 nm, at least about 90 nm, at least about 95 nm, or at least about 100 nm) and/or at most 1900 nm (e.g., at most about 1900 nm, at most about 1800 nm, at most about 1700 nm, at most about 1600 nm, at most about 1500 nm, at most about 1400 nm, at most about 1300 nm, at most about 1200 nm, at most about 1100 nm, at most about 1000 nm, at most about 950 nm, at most about 900 nm, at most about 850 nm, at most about 800 nm, at most about 750 nm, at most about 700 nm, at most about 650 nm, at most about 600 nm, at most about 550 nm, or at most about 500 nm), such as in the range of about 20 to about 1500 nm, such as about 30 to about 1200 nm, about 40 to about 1100 nm, about 50 to about 1000 nm, about 60 to about 900 nm, about 70 to 800 nm, about 80 to 700 nm, about 90 to 600 nm, or about 50 to 500 nm or about 100 to 500 nm, such as in the range of 10 to 1000 nm, 15 to 500 nm, 20 to 450 nm, 25 to 400 nm, 30 to 350 nm, 40 to 300 nm, 50 to 250 nm, 60 to 200 nm, or 70 to 150 nm.

In some embodiments, the particles described herein are nanoparticles. The term "nanoparticle" relates to a nano-sized particle comprising nucleic acid (especially mRNA) as described herein and at least one cationic or cationically ionizable lipid, wherein all three external dimensions of the particle are in the nanoscale, i.e., at least about 1 nm and below about 1000 nm. Preferably, the size of a particle is its diameter.

## Pharmaceutical compositions comprising nucleic acid particles

In some embodiments, the composition of the present invention, such as that produced by the methods of the present invention, is a pharmaceutical composition. In an embodiment, this composition may comprise salts, buffers, or other components as further described below. In the most preferred embodiment, the composition is that which comprises two or more different RNA molecules.

In some embodiments, a salt for use in the compositions described herein comprises sodium chloride. Without wishing to be bound by theory, sodium chloride functions as an ionic osmolality agent for preconditioning nucleic acid (such as RNA and/or DNA) prior to mixing with lipids. In some embodiments, the compositions described herein may comprise alternative organic or inorganic salts. Alternative salts include, without limitation, potassium chloride, dipotassium phosphate, monopotassium phosphate, potassium acetate, potassium bicarbonate, potassium sulfate, disodium phosphate, monosodium phosphate, sodium acetate, sodium bicarbonate, sodium sulfate, lithium chloride, magnesium chloride, magnesium phosphate, calcium chloride, and sodium salts of ethylenediaminetetraacetic acid (EDTA).

Generally, compositions for storing nucleic acid particles such as for freezing nucleic acid particles comprise low sodium chloride concentrations, or comprises a low ionic strength. In some embodiments, the sodium chloride is at a concentration from 0 mM to about 50 mM, from 0 mM to about 40 mM, or from about 10 mM to about 50 mM.

According to the present disclosure, the nucleic acid particle compositions described herein have a pH suitable for the stability of the nucleic acid particles and, in particular, for the stability of the nucleic acid. Without wishing to be bound by theory, the use of a buffer system maintains the pH of the particle compositions described herein during manufacturing,

storage and use of the compositions. In some embodiments of the present disclosure, the buffer system may comprise a solvent (in particular, water, such as deionized water, in particular water for injection) and a buffering substance. The buffering substance may be selected from 2-[4-(2-hydroxyethyl)piperazin-1-yl]ethanesulfonic acid (HEPES), 2-amino-2-(hydroxymethyl)propane-1,3-diol (Tris), acetate, and histidine. A preferred buffering substance is HEPES.

Compositions described herein may also comprise a cryoprotectant and/or a surfactant as stabilizer to avoid substantial loss of the product quality and, in particular, substantial loss of nucleic acid (especially mRNA) activity during storage, freezing, and/or lyophilization, for example to reduce or prevent aggregation, particle collapse, nucleic acid (especially mRNA) degradation and/or other types of damage.

In an embodiment, the cryoprotectant is a carbohydrate. The term "carbohydrate", as used herein, refers to and encompasses monosaccharides, disaccharides, trisaccharides, oligosaccharides and polysaccharides.

In an embodiment, the cryoprotectant is a monosaccharide. The term "monosaccharide", as used herein refers to a single carbohydrate unit (e.g., a simple sugar) that cannot be hydrolyzed to simpler carbohydrate units. Exemplary monosaccharide cryoprotectants include glucose, fructose, galactose, xylose, ribose and the like.

In an embodiment, the cryoprotectant is a disaccharide. The term "disaccharide", as used herein refers to a compound or a chemical moiety formed by 2 monosaccharide units that are bonded together through a glycosidic linkage, for example through 1-4 linkages or 1-6 linkages. A disaccharide may be hydrolyzed into two monosaccharides. Exemplary disaccharide cryoprotectants include sucrose, trehalose, lactose, maltose and the like.

The term "trisaccharide" means three sugars linked together to form one molecule. Examples of a trisaccharides include raffinose and melezitose.

In an embodiment, the cryoprotectant is an oligosaccharide. The term "oligosaccharide", as used herein refers to a compound or a chemical moiety formed by 3 to about 15, such as 3 to about 10 monosaccharide units that are bonded together through glycosidic linkages, for example through 1-4 linkages or 1-6 linkages, to form a linear, branched or cyclic structure. Exemplary oligosaccharide cryoprotectants include cyclodextrins, raffinose, melezitose, maltotriose, stachyose, acarbose, and the like. An oligosaccharide can be oxidized or reduced.

In an embodiment, the cryoprotectant is a cyclic oligosaccharide. The term "cyclic oligosaccharide", as used herein refers to a compound or a chemical moiety formed by 3 to about 15, such as 6, 7, 8, 9, or 10 monosaccharide units that are bonded together through glycosidic linkages, for example through 1-4 linkages or 1-6 linkages, to form a cyclic structure. Exemplary cyclic oligosaccharide cryoprotectants include cyclic oligosaccharides that are discrete compounds, such as $\alpha$ cyclodextrin, $\beta$ cyclodextrin, or $\gamma$ cyclodextrin.

Other exemplary cyclic oligosaccharide cryoprotectants include compounds which include a cyclodextrin moiety in a larger molecular structure, such as a polymer that contains a cyclic oligosaccharide moiety. A cyclic oligosaccharide can be oxidized or reduced, for example, oxidized to dicarbonyl forms. The term "cyclodextrin moiety", as used herein refers to cyclodextrin (*e.g.*, an $\alpha$, $\beta$, or $\gamma$ cyclodextrin) radical that is incorporated into, or a part of, a larger molecular structure, such as a polymer. A cyclodextrin moiety can be bonded to one or more other moieties directly, or through an optional linker. A cyclodextrin moiety can be oxidized or reduced, for example, oxidized to dicarbonyl forms.

Carbohydrate cryoprotectants, *e.g.*, cyclic oligosaccharide cryoprotectants, can be derivatized carbohydrates. For example, in an embodiment, the cryoprotectant is a derivatized cyclic oligosaccharide, *e.g.*, a derivatized cyclodextrin, *e.g.*, 2-hydroxypropyl-$\beta$-cyclodextrin, *e.g.*, partially etherified cyclodextrins (*e.g.*, partially etherified $\beta$ cyclodextrins).

An exemplary cryoprotectant is a polysaccharide. The term "polysaccharide", as used herein refers to a compound or a chemical moiety formed by at least 16 monosaccharide units that are bonded together through glycosidic linkages, for example through 1-4 linkages or 1-6 linkages, to form a linear, branched or cyclic structure, and includes polymers that comprise polysaccharides as part of their backbone structure. In backbones, the polysaccharide can be linear or cyclic. Exemplary polysaccharide cryoprotectants include glycogen, amylase, cellulose, dextran, maltodextrin and the like.

In some embodiments, nucleic acid particle compositions may include sucrose. Without wishing to be bound by theory, sucrose functions to promote cryoprotection of the compositions, thereby preventing nucleic acid (especially mRNA) particle aggregation and maintaining chemical and physical stability of the composition. In some embodiments, nucleic acid particle compositions may include alternative cryoprotectants to sucrose. Alternative stabilizers include, without limitation, trehalose and glucose. In a specific embodiment, an alternative stabilizer to sucrose is trehalose or a mixture of sucrose and trehalose.

A preferred cryoprotectant is selected from the group consisting of sucrose, trehalose, glucose, and a combination thereof, such as a combination of sucrose and trehalose. In a preferred embodiment, the cryoprotectant is sucrose.

Some embodiments of the present disclosure contemplate the use of a chelating agent in a nucleic acid composition described herein. Chelating agents refer to chemical compounds that are capable of forming at least two coordinate covalent bonds with a metal ion, thereby generating a stable, water-soluble complex. Without wishing to be bound by theory, chelating agents reduce the concentration of free divalent ions, which may otherwise induce accelerated nucleic acid degradation in the present disclosure. Examples of suitable chelating agents include, without limitation,

ethylenediaminetetraacetic acid (EDTA), a salt of EDTA, desferrioxamine B, deferoxamine, dithiocarb sodium, penicillamine, pentetate calcium, a sodium salt of pentetic acid, succimer, trientine, nitrilotriacetic acid, trans-diaminocyclohexanetetraacetic acid (DCTA), diethylenetriaminepentaacetic acid (DTPA), and bis(aminoethyl)glycolether-N,N,N',N'-tetraacetic acid. In some embodiments, the chelating agent is EDTA or a salt of EDTA. In an exemplary embodiment, the chelating agent is EDTA disodium dihydrate. In some embodiments, the EDTA is at a molar concentration from about 0.05 mM to about 5 mM, from about 0.1 mM to about 2.5 mM or from about 0.25 mM to about 1 mM.

In an alternative embodiment, the nucleic acid particle compositions described herein do not comprise a chelating agent.

Compositions comprising nucleic acids described herein, optionally formulated in particles, may be useful as or for preparing pharmaceutical compositions or medicaments for therapeutic or prophylactic treatments.

The term "pharmaceutical composition" relates to a composition comprising a therapeutically effective agent, preferably together with pharmaceutically acceptable carriers, diluents and/or excipients. Said pharmaceutical composition is useful for treating, preventing, or reducing the severity of a disease by administration of said pharmaceutical composition to a subject.

The pharmaceutical compositions of the present disclosure may comprise one or more adjuvants or may be administered with one or more adjuvants. The term "adjuvant" relates to a compound which prolongs, enhances or accelerates an immune response. Adjuvants comprise a heterogeneous group of compounds such as oil emulsions (*e.g.*, Freund's adjuvants), mineral compounds (such as alum), bacterial products (such as Bordetella pertussis toxin), or immune-stimulating complexes. Examples of adjuvants include, without limitation, LPS, GP96, CpG oligodeoxynucleotides, growth factors, and cytokines, such as monokines, lymphokines, interleukins, chemokines. The chemokines may be IL-1, IL-2, IL-3, IL-4, IL-5, IL-6, IL-7, IL-8, IL-9, IL-10, IL-12, INFa, INF-$\gamma$, GM-CSF, LT-a. Further known adjuvants are aluminum hydroxide, Freund's adjuvant or oil such as Montanide® ISA51. Other suitable adjuvants for use in the present disclosure include lipopeptides, such as Pam3Cys, as well as lipophilic components, such as saponins, trehalose-6,6-dibehenate (TDB), monophosphoryl lipid-A (MPL), monomycoloyl glycerol (MMG), or glucopyranosyl lipid adjuvant (GLA).

The pharmaceutical compositions of the present disclosure may be in a storable form (*e.g.*, in a frozen or lyophilized/freeze-dried form) or in a "ready-to-use form" (*i.e.*, in a form which can be immediately administered to a subject, *e.g.*, without any processing such as diluting). Thus, prior to administration of a storable form of a pharmaceutical composition, this storable form has to be processed or transferred into a ready-to-use or administrable form. *E.g.*, a frozen pharmaceutical composition has to be thawed, or a freeze-dried pharmaceutical composition has to be reconstituted, e.g. by using a suitable solvent (*e.g.*, deionized water, such as water for injection) or liquid (*e.g.*, an aqueous solution).

The pharmaceutical compositions according to the present disclosure are generally applied in a "pharmaceutically effective amount" and in "a pharmaceutically acceptable preparation".

The term "pharmaceutically acceptable" refers to the non-toxicity of a material which does not interact with the action of the active component of the pharmaceutical composition.

The term "pharmaceutically effective amount" refers to the amount which achieves a desired reaction or a desired effect alone or together with further doses. In some embodiments relating to the treatment of a particular disease, the desired reaction may relate to inhibition of the course of the disease. This comprises slowing down the progress of the disease and, in some embodiments, interrupting or reversing the progress of the disease. The desired reaction in a treatment of a disease may also be delay of the onset or a prevention of the onset of said disease or said condition. An effective amount of the pharmaceutical compositions described herein will depend on the condition to be treated, the severity of the disease, the individual parameters of the patient, including age, physiological condition, size and weight, the duration of treatment, the type of an accompanying therapy (if present), the specific route of administration and similar factors. Accordingly, the doses administered of the pharmaceutical compositions described herein may depend on several of such parameters. In the case that a reaction in a patient is insufficient with an initial dose, higher doses (or effectively higher doses achieved by a different, more localized route of administration) may be used.

The pharmaceutical compositions of the present disclosure may contain buffers, preservatives, and optionally other therapeutic agents. In some embodiments, the pharmaceutical compositions of the present disclosure comprise one or more pharmaceutically acceptable carriers, diluents and/or excipients.

Suitable preservatives for use in the pharmaceutical compositions of the present disclosure include, without limitation, benzalkonium chloride, chlorobutanol, paraben and thimerosal.

The term "excipient" as used herein refers to a substance which may be present in a pharmaceutical composition of the present disclosure but is not an active ingredient. Examples of excipients, include without limitation, carriers, binders, diluents, lubricants, thickeners, surface active agents, preservatives, stabilizers, emulsifiers, buffers, flavoring agents, or colorants

The term "diluent" relates a diluting and/or thinning agent. Moreover, the term "diluent" includes any one or more of fluid, liquid or solid suspension and/or mixing media. Examples of suitable diluents include ethanol, glycerol and water.

The term "carrier" refers to a component which may be natural, synthetic, organic, inorganic in which the active

component is combined in order to facilitate, enhance or enable administration of the pharmaceutical composition. A carrier as used herein may be one or more compatible solid or liquid fillers, diluents or encapsulating substances, which are suitable for administration to subject. Suitable carrier include, without limitation, sterile water, Ringer, Ringer lactate, sterile sodium chloride solution, isotonic saline, polyalkylene glycols, hydrogenated naphthalenes and, in particular, biocompatible lactide polymers, lactide/glycolide copolymers or polyoxyethylene/polyoxy-propylene copolymers. In some embodiments, the pharmaceutical composition of the present disclosure includes isotonic saline.

Pharmaceutically acceptable carriers, excipients or diluents for therapeutic use are well known in the pharmaceutical art, and are described, for example, in Remington's Pharmaceutical Sciences, Mack Publishing Co. (A. R Gennaro edit. 1985).

Pharmaceutical carriers, excipients or diluents can be selected with regard to the intended route of administration and standard pharmaceutical practice.

**Routes of administration of pharmaceutical compositions**

In some embodiments, the pharmaceutical compositions described herein may be administered intravenously, intraarterially, subcutaneously, intradermally, dermally, intranodally, intramuscularly, intratumorally, or peritumorally. In some embodiments, the pharmaceutical composition is formulated for local administration or systemic administration. Systemic administration may include enteral administration, which involves absorption through the gastrointestinal tract, or parenteral administration. As used herein, "parenteral administration" refers to the administration in any manner other than through the gastrointestinal tract, such as by intravenous injection. In some embodiments, the pharmaceutical compositions are formulated for systemic administration. In some embodiments, the systemic administration is by intravenous administration.

**Uses of pharmaceutical compositions**

The composition comprising RNA of the present invention/the RNA molecules obtained in the present invention, optionally formulated in particles, may be used in the therapeutic or prophylactic treatment of various diseases, in particular diseases in which provision of a peptide or polypeptide to a subject results in a therapeutic or prophylactic effect. For example, provision of an antigen or epitope which is derived from a virus may be useful in the treatment of a viral disease caused by said virus. Provision of a tumor antigen or epitope may be useful in the treatment of a cancer disease wherein cancer cells express said tumor antigen. Provision of a functional protein or enzyme may be useful in the treatment of genetic disorder characterized by a dysfunctional protein, for example in lysosomal storage diseases (*e.g*. Mucopolysaccharidoses) or factor deficiencies. Provision of a cytokine or a cytokine-fusion may be useful to modulate tumor microenvironment.

The term "disease" (also referred to as "disorder" herein) refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality.

In the present context, the term "treatment", "treating" or "therapeutic intervention" relates to the management and care of a subject for the purpose of combating a condition such as a disease. The term is intended to include the full spectrum of treatments for a given condition from which the subject is suffering, such as administration of the therapeutically effective compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of an individual for the purpose of combating the disease, condition or disorder and includes the administration of the active compounds to prevent the onset of the symptoms or complications.

The term "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a

disease from occurring in an individual. The terms "prophylactic treatment" or "preventive treatment" are used herein interchangeably.

The terms "individual" and "subject" are used herein interchangeably. They refer to a human or another mammal (*e.g.*, mouse, rat, rabbit, dog, cat, cattle, swine, sheep, horse or primate), or any other non-mammal-animal, including birds (chicken), fish or any other animal species that can be afflicted with or is susceptible to a disease (*e.g.*, cancer, infectious diseases) but may or may not have the disease, or may have a need for prophylactic intervention such as vaccination, or may have a need for interventions such as by protein replacement. In many embodiments, the individual is a human being. Unless otherwise stated, the terms "individual" and "subject" do not denote a particular age, and thus encompass adults, elderlies, children, and newborns. In some embodiments of the present disclosure, the "individual" or "subject" is a "patient".

The term "patient" means an individual or subject for treatment, in particular a diseased individual or subject.

The composition of the present invention/RNA obtained in the present invention may be administered to a subject for delivering the nucleic acid to cells of the subject.

The composition of the present invention/RNA obtained in the present invention may be administered to a subject for delivering a therapeutic or prophylactic peptide or polypeptide (e.g., a pharmaceutically active peptide or polypeptide) to the subject, wherein the nucleic acid encodes a therapeutic or prophylactic peptide or polypeptide.

The composition of the present invention/RNA obtained in the present invention may be administered to a subject for treating or preventing a disease in a subject, wherein delivering the nucleic acid to cells of the subject is beneficial in treating or preventing the disease.

The composition of the present invention/RNA obtained in the present invention may be administered to a subject for treating or preventing a disease in a subject, wherein the RNA encodes a therapeutic or prophylactic peptide or polypeptide and wherein delivering the therapeutic or prophylactic peptide or polypeptide to the subject is beneficial in treating or preventing the disease.

In some embodiments, the RNA is present in a composition as described herein.

In some embodiments, the RNA is administered in a pharmaceutically effective amount.

In some embodiments, the subject is a mammal. In some embodiments, the mammal is a human.

In some embodiments of the disclosure, the aim is to induce an immune response by providing a vaccine.

A person skilled in the art will know that one of the principles of immunotherapy and vaccination is based on the fact that an immunoprotective reaction to a disease is produced by immunizing a subject with an antigen or an epitope, which is immunologically relevant with respect to the disease to be treated. Accordingly, RNA described herein are applicable for inducing or enhancing an immune response. RNA described herein are thus useful in a prophylactic and/or therapeutic treatment of a disease involving an antigen or epitope.

In some embodiments of the disclosure, the aim is to treat cancer by vaccination.

In some embodiments of the disclosure, the aim is to provide protection against an infectious disease by vaccination.

In some embodiments of the disclosure, the aim is to provide secreted therapeutic proteins, such as antibodies, bispecific antibodies, cytokines, cytokine fusion proteins, enzymes, to a subject, in particular a subject in need thereof.

In some embodiments of the disclosure, the aim is to provide a protein replacement therapy, such as production of erythropoietin, Factor VII, Von Willebrand factor, β-galactosidase, Alpha-N-acetylglucosaminidase, to a subject, in particular a subject in need thereof.

In some embodiments of the disclosure, the aim is to modulate/reprogram immune cells in the blood.

In some embodiments of the disclosure, the aim is to provide one or more cytokines or cytokine fusions which modulate tumor microenvironment to a subject, in particular a subject in need thereof.

In some embodiments of the disclosure, the aim is to provide one or more cytokines or cytokine fusions which have anti-tumor activity to a subject, in particular a subject in need thereof.

Citation of documents and studies referenced herein is not intended as an admission that any of the foregoing is pertinent prior art. All statements as to the contents of these documents are based on the information available to the applicants and do not constitute any admission as to the correctness of the contents of these documents.

The description (including the following examples) is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles defined herein may be applied to other examples and applications without departing from the spirit and scope of the various embodiments. Thus, the various embodiments are not intended to be limited to the examples described herein and shown, but are to be accorded the scope consistent with the claims.

**Digital droplet PCR**

In some embodiments, the invention comprises determining the amounts of each of the RNA molecules in the

composition.

The 2 or more different RNA molecules that are obtained in the methods and compositions of the present invention may be determined quantitatively via a digital droplet polymerase chain reaction (ddPCR) assay.

In detail, this can be achieved as follows: first, reverse transcription is carried out on the composition comprising 2 or more different RNA molecules that is ultimately obtained in the present invention (the "RNA sample" composition). This results in the provision of a composition comprising n different cDNA (DNA) molecules (*n* = the number of different RNA molecules that was initially present in the composition). Then, a polymerase chain reason (PCR)-based assay is carried out using a first primer set and a single second primer, wherein the first primer set comprises *n* primer species, wherein each primer species is capable of annealing to a first target region of only one of the *n* different DNA molecules in the sample (i.e. is specific to each different DNA molecule in the sample), and the single second primer is capable of annealing to a second target region of all of the *n* DNA molecules in the sample (i.e. is common to all DNA molecules in the sample). PCR is carried out using these primers. The different DNA molecules produced by the PCR-based assay can be measured and qualified in tandem, as a stand-in for the RNA molecules that were present in the original RNA sample (this can be done with ddPCR, further details follow below). Such an assay can therefore measure in parallel the quantitative ratio of the 2 or more RNA molecules in the original RNA sample.

Droplet digital PCR (ddPCR) measures absolute quantities by counting nucleic acid molecules encapsulated in discrete, volumetrically defined water-in-oil droplet partitions. Commercially available systems for ddPCR comprise Droplet Digital™ PCR System (BioRad Laboratories, Hercules, CA, USA) or Rain Drop Plus™ system (RainDance Technologies, Lexington, MA, USA). A PCR reaction is divided into around 20,000 droplets that either contain or not contain template leading to PCR positive and negative droplets that are counted allowing quantitation of template concentration using Poisson distribution algorithm. The droplets are generated using a droplet generator.

ddPCR enables more precise absolute quantification as compared to classic relative quantitation which is limited due to the doubling during each cycle. ddPCR has several advantages over conventional methods (e.g. qPCR) because no standard curve is needed for quantification, it is a more robust method (even sub-optimal primer pairs that lead to false positive or false negative signals will be eventually give a correct concentration due to the Poisson distribution algorithm), and enables precise (diagnostic) quantification (Resolution of standard qPCR: 0.5 cycles (+/-50%) vs. 10% for ddPCR (using acoustic pipetting 1.5% accuracy possible).

Thus, in an embodiment, the method of the present invention further comprises a step of determining the quantitative ratio of the 2 or more RNA molecules present in the RNA sample composition that is obtained in the present invention. In an embodiment, this additional step comprises the sub-steps of:

a) reverse transcription of the 2 or more (*n*) different RNA molecules in the RNA sample composition into cDNA molecules of *n* different DNA molecules; and

b) carrying out a PCR-based assay on the resulting cDNA molecules, the PCR-based assay using a first primer set and a single second primer, wherein the first primer set comprises *n* primer species, wherein each primer species is capable of annealing to a first target region of only one of the *n* different DNA molecules in the sample, and the single second primer is capable of annealing to a second target region of all of the *n* different DNA molecules in the sample; optionally wherein the PCR-based assay is ddPCR.

## Examples

### Example 1: Production of a 1:1:1 RNA composition from PCR templates

Production of a composition comprising three different RNA molecules at a ratio of 1:1:1 by mass, with PCR template DNA starting material was carried out. This included IVT followed by tangential flow filtration (TFF)-only purification and sterile filtering. To meet the desired ratio, it was determined that shorter RNA molecules would need to be transcribed more often (higher molar amount of RNA molecules) to reach the same mass (or mass concentration) as a longer RNA molecule. Also, it was determined that the length of the DNA templates would be crucial. DNA templates are always longer than their corresponding RNA products, as the DNA molecules have to contain at least the promoter sequence, this example including a PCR template. Thus, equation (i) was developed, bringing all of these parameters into consideration.

$$c(DNA_X) = \frac{c(DNA_{total}) \cdot RNA\ Fraction\ \cdot DNA_x\ length\ fraction}{RNA_x\ length\ fraction}$$

The RNA fraction was determined based on a desired 1:1:1 ratio for three RNA molecules, RNA1-1, RNA1-2 and RNA1-3. The total amount of DNA to be used was determined by titration, as 0.03mg/ml. Based on this desired ratio, total

DNA concentration and the known lengths of the DNA molecules and RNA molecules, the initial concentrations of each DNA template to be used were determined using formula (i). The calculated values are shown in Table 1 below.

Table 1: Calculating PCR template DNA concentrations for 1:1:1 composition.

| RNA ID | RNA length (nt) | RNA length fraction | RNA Fraction | DNA length (nt) | DNA length fraction | DNA concentration (mg/mL) |
|---|---|---|---|---|---|---|
| RNA1 | 1718 | 0.355 | 0.333 | 1815 | 0.354 | 0.01 |
| RNA2 | 1454 | 0.301 | 0.333 | 1551 | 0.303 | 0.0101 |
| RNA3 | 1661 | 0.344 | 0.333 | 1761 | 0.343 | 0.01 |
| Total | 4833 | N/A | N/A | 5127 | N/A | 0.03 |

In this particular example, the very similar DNA and RNA molecule length, and identical RNA fractions, led to similar concentrations of each DNA template molecule within the reaction mixture. Using the calculated DNA concentrations, IVT was carried out and the entire process development path was performed. Ratios of RNA molecules produced were within acceptable limits.

**Example 2: Varying parameters of the one-pot IVT reaction**

As in Example 1, the one-pot IVT reaction using PCR template DNA molecules was also carried out in the following experiments. Parameters of the novel one-pot IVT reaction were varied and the effects of these parameters were determined.

*Example 2. 1 Total DNA concentration*

In this experiment, the total amount of DNA (PCR templates) was titrated, and the resulting data was analyzed for yield. The results are listed in Table 2 below.

Table 2: Effects of total DNA concentration on IVT yield.

| Mix # | Total DNA concentration (mg/mL) | Yield (mg/mL) |
|---|---|---|
| 1 | 0.015 | 7.41 |
| 2 | | 7.70 |
| 3 | 0.02 | 7.81 |
| 4 | | 7.83 |
| 5 | 0.03 | 8.19 |
| 6 | | 8.02 |
| 7 | 0.05 | 8.16 |
| 8 | | 8.01 |
| 9 | 0.06 | 8.14 |
| 10 | | 8.38 |

The IVT yield increased with increasing concentration of the DNA template, as shown in Figure 1. The increase in yield plateaued with a DNA concentration between 0.03 and 0.05 mg/mL. For further experiments the DNA template concentration was set to 0.03 mg/mL, because the yield was high, but the DNA template concentration was low enough to reduce costs and less amount of protein needs to be separated from the reaction.

*Example 2.2 ATP/CTP concentration*

The RNA sequences to be produced in the one-pot IVT reaction all had high C-content and A-content. Thus, the effects of adjusting CTP and ATP starting concentrations were investigated. ATP and CTP were titrated from 9 mM to 12.6

mM. The results are listed in Table 3 below.

Table 3: Effects of ATP/CTP concentration on IVT yield and RNA ratios.

| Mix # | ATP/CTP concentration (mM) | Yield (mg/mL) | RNA1 (ratio) | RNA2 (ratio) | RNA3 (ratio) |
|---|---|---|---|---|---|
| 1 | 9.0 | 7.27 | 35 | 34 | 32 |
| 2 | | 7.16 | 35 | 32 | 33 |
| 3 | 9.9 | 7.09 | 34 | 31 | 35 |
| 4 | | 7.19 | 33 | 32 | 35 |
| 5 | 10.8 | 7.73 | 34 | 33 | 33 |
| 6 | | 7.67 | 34 | 31 | 34 |
| 7 | 11.7 | 8.92 | 32 | 34 | 34 |
| 8 | | 8.89 | 33 | 34 | 34 |
| 9 | 12.6 | 9.32 | 36 | 32 | 32 |
| 10 | | 9.47 | 33 | 33 | 34 |

It was determined that higher amounts of ATP and CTP had positive effects on integrity, yield and dsRNA content. The IVT yield in particular was drastically impacted. The data have been plotted in Figure 2. The IVT-yield was affected by additional ATP and CTP in the starting reaction. The IVT yield increased with increasing amounts of NTPs (9 mM ATP/CTP were the starting condition), and from around 11.7 mM ATP/CTP, the IVT-yield plateaued.

Further, the ratio of the three RNAs in the mixture was determined by droplet digital PCR (ddPCR) and in this example of a desired 1:1:1 ratio each RNA was represented by 33% in theory. The specification range was set to 20% of the theoretical value meaning an acceptance range 33% ± 6%. The data showed that the ratio was correct for all ten reaction conditions and independent of the CTP/ATP starting concentration (Table 3).

Additional titration experiments were performed to further characterize the concentration ranges. ATP/CTP concentrations tested ranged from 11.7 mM to 13.5 mM. The results are listed in Table 4 below.

Table 4: Results of ATP/CTP concentration on IVT yield and integrity.

| Mix # | ATP/CTP concentration (mM) | Yield (mg/mL) | Integrity (%) |
|---|---|---|---|
| 1 | 9.0 | 6.98 | 87 |
| 2 | | 6.75 | 88 |
| 3 | 11.7 | 8.07 | 91 |
| 4 | | 9.20 | 92 |
| 5 | 12.15 | 8.30 | 91 |
| 6 | | 9.41 | 92 |
| 7 | 12.6 | 9.12 | 91 |
| 8 | | 8.64 | 91 |
| 9 | 13.5 | 9.24 | 93 |
| 10 | | 8.80 | 92 |

As listed in Table 4, the integrity of the IVT production increased with increasing amounts of ATP and CTP. Based on these data, the dsRNA was reduced, and the capping efficiency was slightly increased. For such a reaction, the ATP and CTP concentration was deemed favourable at 12.2 mM.

*Other parameters*

With increasing amounts of ATP and CTP, it was presumed that more Mg2+ cations would be needed for the reaction as more PPi is produced, precipitating more of these ions out of solution. To study the effect of Mg2+ cations further, additional amount of Mg2+ were titrated. However, there were no effects of adding more Mg2+-cations on the ratio.

**Example 3: Production of a 1:1:1 RNA composition from linearised plasmids**

Certain DNA sequences can be difficult to clone into appropriate PCR template forms, due to e.g. high CG-content and high content of sequence repetitions, which can lead to problems with template generation by PCR as well as the specificity of the analytics primer. Thus, it may be advantageous to start the one-pot IVT reaction from DNA comprised in less refined forms, such as linearised plasmid DNA.

As opposed to PCR DNA templates, when using linearised plasmid DNA for the production of RNA molecules according to the present invention, the difference in non-transcribed DNA from one DNA template to another is more significant. However, the robust and adaptable method developed by the present inventors using formula (i) is capable of accounting for this change in weighting.

The IVT one-pot reaction was then carried out using linearised plasmids as the DNA templates, to provide a composition comprising RNA1, RNA2 and RNA3, with a 1:1:1 ratio. The required concentrations of each linearised plasmid DNA template were calculated as shown in Table 5 below.

Table 5: Calculating linearised plasmid DNA concentrations for 1:1:1 composition.

| RNA ID | RNA length (nt) | RNA length fraction | RNA Fraction | DNA length (nt) | DNA length fraction | DNA concentration (mg/mL) |
|---|---|---|---|---|---|---|
| RNA1 | 1718 | 0.355 | 0.333 | 5259 | 0.340 | 0.0957 |
| RNA2 | 1454 | 0.301 | 0.333 | 4995 | 0.323 | 0.1074 |
| RNA3 | 1661 | 0.344 | 0.333 | 5205 | 0.337 | 0.0980 |
| Total | 4833 | N/A | N/A | 15459 | N/A | 0.3 |

Based on the necessary DNA concentration values that were determined, the sequences were then successfully transcribed in a one pot reaction using linearized plasmid DNA.

Due to the high C-content of the RNA molecules, it was further investigated whether IVT reaction conditions could be optimized with additional CTP included in the beginning of the reaction. The results of this are shown in Table 6 below.

Table 6: Effects of CTP concentration on yield.

| Mix # | CTP concentration (mM) | Yield (mg/mL) |
|---|---|---|
| 1 | 9.0 | 6.9 |
| 2 | | 6.8 |
| 5 | 10.8 | 8.2 |
| 6 | | 8.2 |
| 7 | 11.7 | 8.8 |
| 8 | | 8.8 |
| 9 | 12.6 | 9.3 |
| 10 | | 9.3 |

As shown in the table above, transcription yield increased with increasing amount of CTP.

**Example 4: varying the ratio of obtained RNA molecules**

A great advantage of doing a one-pot IVT reaction is in the reduction in time. However, the one-pot IVT reaction following formula (i) also benefits from improved flexibility. If the desired ratio of RNA molecules within a certain composition changes, e.g. during pre-clinical and clinical trials, one-pot IVT production can easily be adjusted according to formula (i) to accommodate for this. To investigate whether such flexibility was possible, we tested different ratios, i.e. aiming to achieve different RNA fractions, by adding - for one template - two times, 4 times and 8 times amount of the DNA template. The results showed that, following the one-pot IVT reaction, these changes in DNA ratio input successfully corresponded to the provision of the right ratio of RNA molecules in the resulting RNA composition.

The RNA fractions of the RNAs was first set to 1:1:1 (RNA1 : RNA2: RNA3) for obtaining the corresponding mass

concentrations of RNA in the resulting composition. It was then considered whether obtaining a larger mass concentration of, e.g., RNA3 could be beneficial for certain applications. Therefore, the flexibility of changing the RNA ratio was explored for potential use in later applications. Different one-pot IVT experiments were performed, changing the mass concentration of the DNA template from which RNA3 is transcribed, and detecting the RNA ratio in the resulting composition, following the reaction. As a baseline, at least 0.01 μg/μL per DNA template were used, e.g., for a ratio of 1:1:4, 0.01 μg/μL of the RNA1 and RNA2 DNA templates were used, and 0.04 μg/μL of the RNA3 DNA template was used. The data for the RNA3 DNA template titration is shown in Figure 3.

The different RNA ratios detected were comparable to the theoretical values. Thus, it was determined that the RNA ratios can be adapted quickly by changing the input DNA template concentration that is used in the one-pot IVT reaction.

These experiments included a large amount of DNA that needed to be removed by DNase I digestion. Thus, the inventors investigated whether low amounts of DNA could be used to reduce the costs and potential negative effects on dsRNA. In a second experiment, the amount of the of total DNA template concentration of the 1:1:4 mixture was titrated. The results are listed in Table 7 below.

Table 7: Effects of total DNA concentration on yield.

| Mix # | Total DNA concentration (mg/mL) | Yield (mg/mL) |
|---|---|---|
| 1 | 0.015 | 8.00 |
| 2 | | 8.31 |
| 3 | 0.02 | 9.00 |
| 4 | | 8.93 |
| 5 | 0.03 | 9.98 |
| 6 | | 9.51 |
| 7 | 0.05 | 9.79 |
| 8 | | 9.80 |
| 9 | 0.06 | 9.76 |
| 10 | | 10.05 |

As listed in the table above, the IVT-yield increased with increasing total DNA template concentration, but started to plateau at a DNA concentration of 0.04 μg/μL. Further increasing the amount of DNA template resulted in a slight increase in the dsRNA content. The RNA ratio was not affected by the total DNA template concentration and so the optimal DNA concentration for such a 1:1:4 one-pot IVT reaction was considered to be least 0.04 μg/μL.

Furthermore, to obtain a complete dataset regarding the flexible altering of RNA ratios in the method of the present invention, the ratios for RNA1 and RNA2 were also altered. The data are shown in Figure 16, and demonstrated the possibility of flexibly generating tailored mixes of the three different RNA molecules.

**Example 5: Production of a 1:1:1:1 RNA composition from linearised plasmids**

The same approach was used to produce a composition comprising 4 different RNA molecules, encoding for 8 different antigens (2 antigens per RNA molecule). The task was to optimize the reaction for a 1:1:1:1 ratio. As a starting material, linearised plasmid DNA molecules were used. Linearised plasmid DNA molecules are longer compared to the equivalent PCR templates, since linearised plasmids comprise, e.g., genes for selection markers and origins of replication. This means that the non-transcribed DNA was a more significant factor in this experiment. Also, in this experiment the RNA molecules to be obtained were variable in length, with the shortest RNA 1283 nt and the longest 3002 nt. Using the formula (i), this resulted in the values that are shown in Table 8 below.

Table 8: Calculating linearised plasmid DNA concentrations for 1:1:1:1 composition.

| RNA ID | RNA length (nt) | RNA length fraction | RNA Fraction | DNA length (nt) | DNA length fraction | DNA concentration (mg/mL) |
|---|---|---|---|---|---|---|
| RNA1 | 1859 | 0.219 | 0.25 | 5400 | 0.238 | 0.0544 |

(continued)

| RNA ID | RNA length (nt) | RNA length fraction | RNA Fraction | DNA length (nt) | DNA length fraction | DNA concentration (mg/mL) |
|---|---|---|---|---|---|---|
| RNA2 | 3002 | 0.353 | 0.25 | 6561 | 0.289 | 0.0409 |
| RNA3 | 2354 | 0.277 | 0.25 | 5895 | 0.260 | 0.0469 |
| RNA4 | 1283 | 0.151 | 0.25 | 4824 | 0.213 | 0.0704 |
| Total | 8498 | N/A | N/A | 22680 | N/A | 0.2 |

The one-pot IVT reaction was performed with using the concentration coming out from the calculation, resulting in a RNA ratio within the acceptance criteria.

It was further determined that producing modified RNA molecules, the buffer composition used, the NTP composition and the concentration of capping reagent had no influence on the ratio of RNA molecules that was obtained.

**Example 6: determining the quantitative ratio between at least 2 different RNA molecules in the same RNA sample composition using ddPCR**

This experiment further details the use of ddPCR to determine the quantitative ratio between the (at least 2) different RNA molecules that are present in the RNA sample compositions that are ultimately obtained in the present invention.

*Materials and Methods:*

*cDNA synthesis*

Complementary DNA synthesis was performed according to the manufacturer's protocol using the SuperScript IV First-Strand synthesis kit (Invitrogen). The RNA mixture samples were diluted to 5 ng/μL. For one reaction, 5 ng of RNA mixture, 1 μL of 10 μM cDNA primer, and 1 μL of 10 mM dNTPs were combined, and the volume was adjusted to 13.5 μL with $H_2O$. The cDNA primer anneals in the 3'UTR, partially covering the poly(A)-tail. Generally, a master mix containing RNA sample, primers, dNTP, and water was made and divided into four tubes to measure the sample in triplicate and one negative control. The RNA was denatured at 80°C for 5 min, snap-cooled on ice for more than 1 min, and 4 μL of 5× SuperScript IV buffer, 1 μL of 40 U/μL RNase inhibitor, 1 μL of 0.1 M DTT, and 0.5 μL of 200 U/μL SuperScript IV reverse transcriptase were added. For the negative control, 0.5 μL of water were added instead of the reverse transcriptase. Samples were incubated in a PCR cycler with the following program: 55°C for 10 min, 80°C for 10 min, hold at 4°C. Afterwards, 0.5 μL RNase H (2 U/μL) was added and the sample incubated for 20 min at 37°C. The cDNA samples were either stored at -20°C in DNA low binding tubes or directly processed for ddPCR.

*Droplet digital PCR*

Droplet digital PCR was conducted on a QX200/C1000 system (Bio-Rad) according to the manufacturer's instructions. The cDNA was always freshly diluted to a concentration of ~1000 CN/μL. For one reaction, 5.5 μL of cDNA, 11 μL of 2x ddPCR SuperMix (Bio-Rad), 0.25 μM dual-labelled HEX-BHQ1-probe, 0.9 μM of RNA-specific forward primer, and 0.9 μM of common reverse primer were combined in a final volume of 22 μL. Each sample was measure in triplicate, together with one negative control per sample. After oil droplet generation, the samples were incubated in the C1000 thermocycler (Bio-Rad) and the following thermal program was executed:

Activation step 1) 600s at 95°C,
Denaturation step 2) 30s at 94°C,
Annealing and extension step 3) 60s at 63°C,
Enzyme inactivation step 4) 600s at 98°C.

Step 2 and 3 were repeated 40 times. After PCR completion, the droplet fluorescence was read with the QX200 droplet reader (Bio-Rad).

*Data Analysis*

Data were analyzed using QuantaSoft version 1.7.4.0917 (Bio-Rad). QuantaSoft is generally able to automatically distinguish and define positive and negative populations. In the rare case that this was not possible, the threshold was set manually. The software automatically calculates the CN/μL. For RNA ratio calculation, the CN/μL were converted into mass/volume concentration (g/μL) according to the following formula:

$$\frac{g}{\mu L} = \left(\frac{CN}{\mu L}\right) * \frac{MW}{6.022 * 10^{23}}$$

where MW is the molecular weight of the RNAs. Afterwards, the ratios (in %) were calculated with the following formulas:

$$\%A = \frac{A\,[\frac{mass}{volume}]}{(A + B + C + D)\,[\frac{mass}{volume}]} * 100$$

To calculate the ratio for RNA B, C or D, the concentration of the respective RNA must be given in the numerator.

*Results:*

To test the accuracy of the method for RNA ratio measurement, several solutions containing different RNAs mixed in different ratios were measured. To evaluate the accuracy of the method, the "%recovery" was calculated, which expresses how close the measured value is to the theoretical value according to the following equation:

$$\%recovery = \frac{mean\ of\ measured\ values}{theoretical\ value} * 100$$

The %recovery spanned between 89.3% and 119.4%. The ratio of the four RNAs was calculated as the amount of one RNA in solution with respect to the other three; that is, if the amount of one RNA was lower, the amount of the other three would increase in a complementary fashion. Thus, the same difference between the measured and the theoretical values results in different %recovery values. For completeness in estimating the accuracy of the method, "delta" was also evaluated, where delta is the mathematical distance of the measurement for both the RNAs in one mixture from the target value. The maximum delta over all the tested RNA mixes is = 3, which further confirms the accuracy of the method.

Table 9: Measuring the quantitative ratio between at least two different RNA molecules present in the sane composition using ddPCR.

| RNA | Theoretica l RNA ratio (%) | Measured RNA ratio (%) | | | | | % recovery | Delta |
|---|---|---|---|---|---|---|---|---|
| | | Repea t 1 | Repea t2 | Repea t3 | Mean (%) | SD (%) | | |
| A | 26 | 27 | 27 | 27 | 27 | 0.3 | 103.5 | 1 |
| **B** | 27 | 27 | 27 | 27 | 27 | 0.2 | 100.4 | 0 |
| **C** | 24 | 23 | 23 | 23 | 23 | 0.3 | 95.8 | -1 |
| **D** | 23 | 23 | 23 | 23 | 23 | 0.3 | 99.9 | 0 |
| | | | | | | | | |
| **A** | 30 | 32 | 34 | 25 | 30 | 4.5 | 101.4 | 0 |
| **B** | 32 | 32 | 33 | 35 | 33 | 1.6 | 104.3 | 1 |
| **C** | 19 | 17 | 14 | 20 | 17 | 3.1 | 89.3 | -2 |
| **D** | 18 | 19 | 19 | 20 | 19 | 0.4 | 107.0 | 1 |
| | | | | | | | | |
| **A** | 21 | 22 | 22 | 22 | 22 | 0.1 | 106.4 | 1 |

(continued)

| RNA | Theoretica l RNA ratio (%) | Measured RNA ratio (%) | | | | | % recovery | Delta |
|---|---|---|---|---|---|---|---|---|
| | | Repea t 1 | Repea t2 | Repea t3 | Mean (%) | SD (%) | | |
| **B** | 22 | 22 | 22 | 22 | 22 | 0.2 | 99.6 | 0 |
| **C** | 29 | 27 | 28 | 28 | 28 | 0.2 | 95.2 | -1 |
| **D** | 28 | 28 | 28 | 28 | 28 | 0.3 | 100.4 | 0 |
| | | | | | | | | |
| **A** | 23 | 23 | 24 | 24 | 24 | 0.2 | 102.3 | 1 |
| **B** | 26 | 26 | 26 | 27 | 26 | 0.3 | 101.8 | 0 |
| **C** | 27 | 26 | 26 | 26 | 26 | 0.2 | 97.2 | -1 |
| **D** | 24 | 24 | 24 | 24 | 24 | 0.3 | 99.0 | 0 |
| | | | | | | | | |
| **A** | 35 | 38 | 38 | 37 | 37 | 0.3 | 106.7 | 2 |
| **B** | 27 | 26 | 27 | 27 | 27 | 0.2 | 98.5 | 0 |
| **C** | 24 | 22 | 22 | 22 | 22 | 0.2 | 92.4 | -2 |
| **D** | 14 | 14 | 14 | 14 | 14 | 0.4 | 99.1 | 0 |
| | | | | | | | | |
| **A** | 15 | 18 | 18 | 18 | 18 | 0.3 | 119.4 | 3 |
| **B** | 28 | 27 | 27 | 27 | 27 | 0.4 | 95.9 | -1 |
| **C** | 24 | 24 | 22 | 23 | 23 | 0.7 | 96.3 | -1 |
| **D** | 32 | 32 | 32 | 33 | 32 | 0.4 | 100.3 | 0 |

The data in Table 9 reports the mean and standard deviation (SD) of the triplicates as well as the %recovery (mean of measured values ÷ theoretical value × 100) and delta (the mathematical distance of the measurement from the theoretical value). The "Theoretical RNA ratio" column compared with the "Mean" column verifies that the measured ratio reflects the theoretical value with minimal deviation.

Thus, ddPCR can be used to verify and determined the quantitative ratio of the at least 2 different RNA molecules that are present in the RNA sample compositions of the present invention.

## Discussion

The one-pot IVT process is robust and scalable. The production includes one day of IVT and a second day for the purification. In contrast, the traditional separate-stage production of a composition comprising three different RNA molecules would result in a total of at least seven production days, as well as the need for the RNA molecules to be mixed in the right ratio in the end. Thus, the one-pot IVT process was developed to keep production time and costs low, to produce three or more RNAs in the right ratio within a one pot reaction, by mixing the DNA templates. It was surprisingly found that such a process occurring in a single reaction mixture could successfully provide good yields of RNA molecules, in the desired ratios.

It was also demonstrated that the RNA ratio could be flexibly changed with the one-pot IVT method, by adapting the DNA template concentration according to the formula (i) accordingly. Through such a process, it was shown that total DNA concentration can be reduced to as little as 0.04 mg/mL, while successfully producing a composition comprising three RNA molecules at desired ratios.

## Claims

1. A method of making a composition comprising 2 or more different RNA molecules, wherein each of the different RNA molecules is obtainable from a different DNA molecule, wherein the method comprises:

a) providing a reaction mixture comprising the DNA molecules, wherein each of the different DNA molecules (*X*) has a concentration according to equation (i):

$$c(DNA_X) = \frac{c(DNA_{total})\cdot RNA\ Fraction \cdot DNA_x\ length\ fraction}{RNA_x\ length\ fraction} \quad (i)$$

wherein:

"$c(DNA_x)$" is the concentration of a given DNA molecule *X*;
"$c(DNA_{total})$" is the total concentration of all of the different DNA molecules;
"RNA fraction" is the fraction of the total RNA molecules in the composition that comprises the RNA molecule that is obtainable from the DNA molecule *X*;
"$DNA_x$ length fraction" is the base pair (bp) length of the DNA molecule *X*, divided by the total bp length of all of the different DNA molecules;
"$RNA_x$ length fraction" is the bp length of the RNA molecule that is obtainable from the DNA molecule *X*, divided by the total bp length of all of the different RNA molecules; and

b) obtaining the RNA molecules from the DNA molecules in the reaction mixture, thereby making the composition.

**2.** The method of claim 1, wherein step a) involves determining the concentration of each of the DNA molecules using equation (i), and then providing a reaction mixture comprising each of the DNA molecules at its respective determined concentration.

**3.** The method of claim 1 or 2, wherein the concentrations of each of the RNA molecules obtained in step b) vary only within the pharmaceutically acceptable limits for the composition.

**4.** The method of any one of claims 1 to 3, wherein the composition comprises at least 2, 3, 4, 5, 6, 7, 8, 9 or 10 different RNA molecules.

**5.** The method of any one of claims 1 to 4, wherein the RNA molecules are obtained from the DNA molecules by transcription.

**6.** The method of any one of claims 1 to 5, wherein one or more of the DNA molecules comprises a PCR template, or each of the DNA molecules comprises a PCR template.

**7.** The method of any one of claims 1 to 6, wherein one or more of the DNA molecules comprises a linearised plasmid, or each of the DNA molecules comprises a linearised plasmid.

**8.** The method of any one of claims 1 to 7, wherein at least one of the DNA molecules comprises a PCR template, and at least one of the DNA molecules comprises a linearised plasmid.

**9.** The method of any one of claims 1 to 8, wherein each of the RNA molecules encodes at least 1, 2, 3, 4, 5, 6, 7, 8, 9 or 10 polypeptides.

**10.** The method of any one of claims 1 to 9, wherein, in step a), the total concentration of all of the DNA molecules in the reaction mixture is predetermined, preferably wherein the total concentration of the DNA molecules is at least 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 2.0, 3.0, 4.0, 5.0, 6.0, 7.0, 8.0, 9.0 or 10 mg/mL.

**11.** The method of any one of claims 1 to 10, wherein the method further comprises:
c) determining the amounts of each of the RNA molecules in the composition.

**12.** The method of any one of claims 1 to 11, wherein the reaction mixture comprises a CTP and ATP concentration of at least 6mM each, preferably at least 9 mM each, preferably at least 10 mM each, preferably at least 11 mM each, preferably at least 12 mM each, preferably at least 13 mM each, preferably at least 14 mM each.

**13.** The method of any one of claims 1 to 12, wherein step b) is carried out for at least 105 minutes, at least 180 minutes, or

between 105 minutes and 180 minutes inclusive.

**14.** A method of making a composition comprising at least a first RNA molecule and a second RNA molecule, wherein the first RNA molecule is obtainable from a first DNA molecule and the second RNA molecule is obtainable from a second DNA molecule, wherein the method comprises:

a) providing a reaction mixture comprising the first DNA molecule and the second DNA molecule; and
b) obtaining the first and second RNA molecules from the first and second DNA molecules respectively;

wherein the first RNA molecule has a greater relative mass than the second RNA molecule, and the second RNA molecule is obtained from the second DNA molecule in a molar amount that is greater than the molar amount of the first RNA molecule that is obtained from the first DNA molecule, such that the first and second RNA molecules are obtained at essentially the same concentrations in the composition.

**15.** A composition obtained or obtainable by the method of any one of claims 1 to 14.

**16.** The composition of claim 15, for use as a medicament.

**17.** A composition comprising 2 or more different DNA molecules, wherein a different RNA molecule is obtainable from each of the different DNA molecules, wherein each of the different DNA molecules (*X*) has a concentration according to equation (i):

$$c(DNA_X) = \frac{c(DNA_{total}) \cdot RNA\ Fraction \cdot DNA_x\ length\ fraction}{RNA_x\ length\ fraction} \quad \text{(i)}$$

wherein:

"c($DNA_x$)" is the concentration of a given DNA molecule *X*;
"c($DNA_{total}$)" is the total concentration of all of the DNA molecules;
"RNA fraction" is the fraction of all RNA molecules obtainable from the reaction mixture that comprises the RNA molecule that is obtainable from the DNA molecule *X*;
"$DNA_x$ length fraction" is the base pair (bp) length of the DNA molecule *X*, divided by the total bp length of all of the different DNA molecules;
"$RNA_x$ length fraction" is the bp length of the RNA molecule obtainable from the DNA molecule *X*, divided by the total bp length of all of the different RNA molecules.

Figure 1

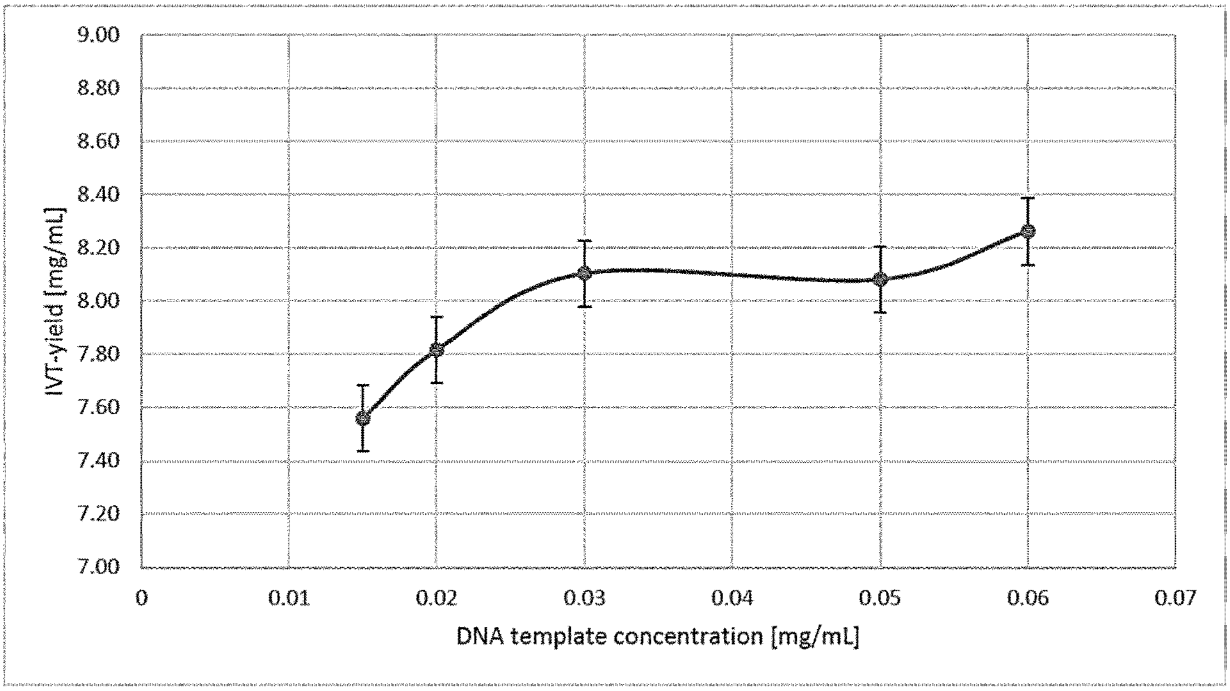
9.00
8.80
8.60
8.40
8.20
8.00
7.80
7.60
7.40
7.20
7.00
IVT-yield [mg/mL]
0
0.01
0.02
0.03
0.04
0.05
0.06
0.07
DNA template concentration [mg/mL]

Figure 2

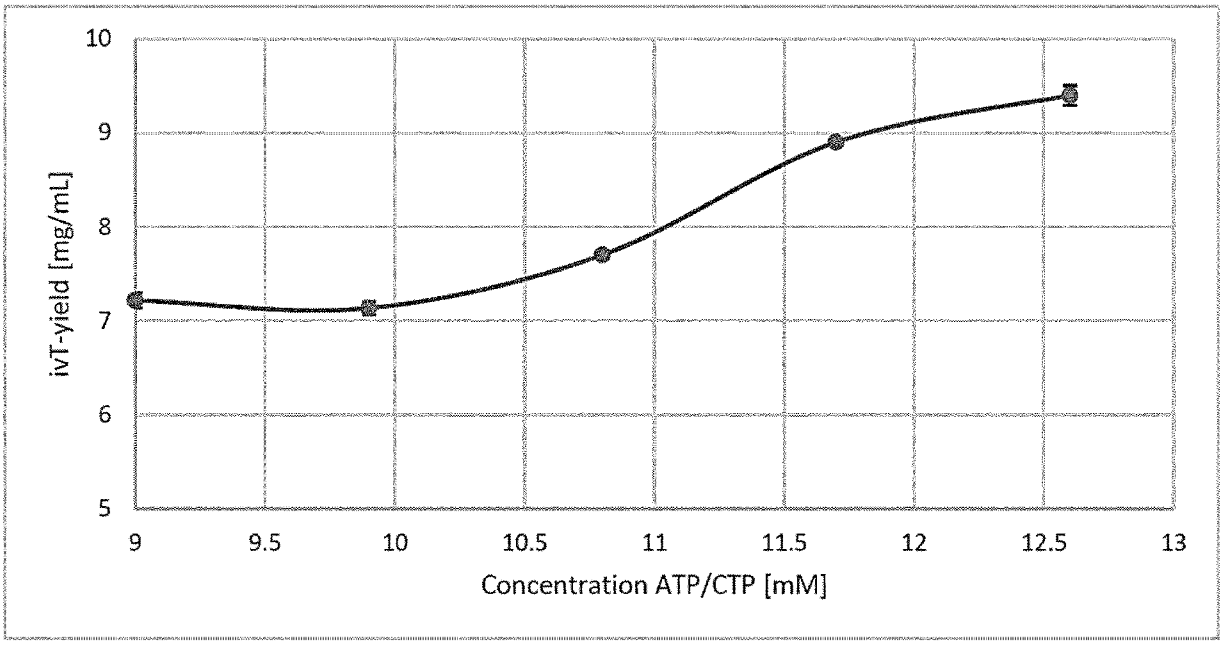
10
9
8
7
6
5
ivT-yield [mg/mL]
9
9.5
10
10.5
11
11.5
12
12.5
13
Concentration ATP/CTP [mM]

Figure 3

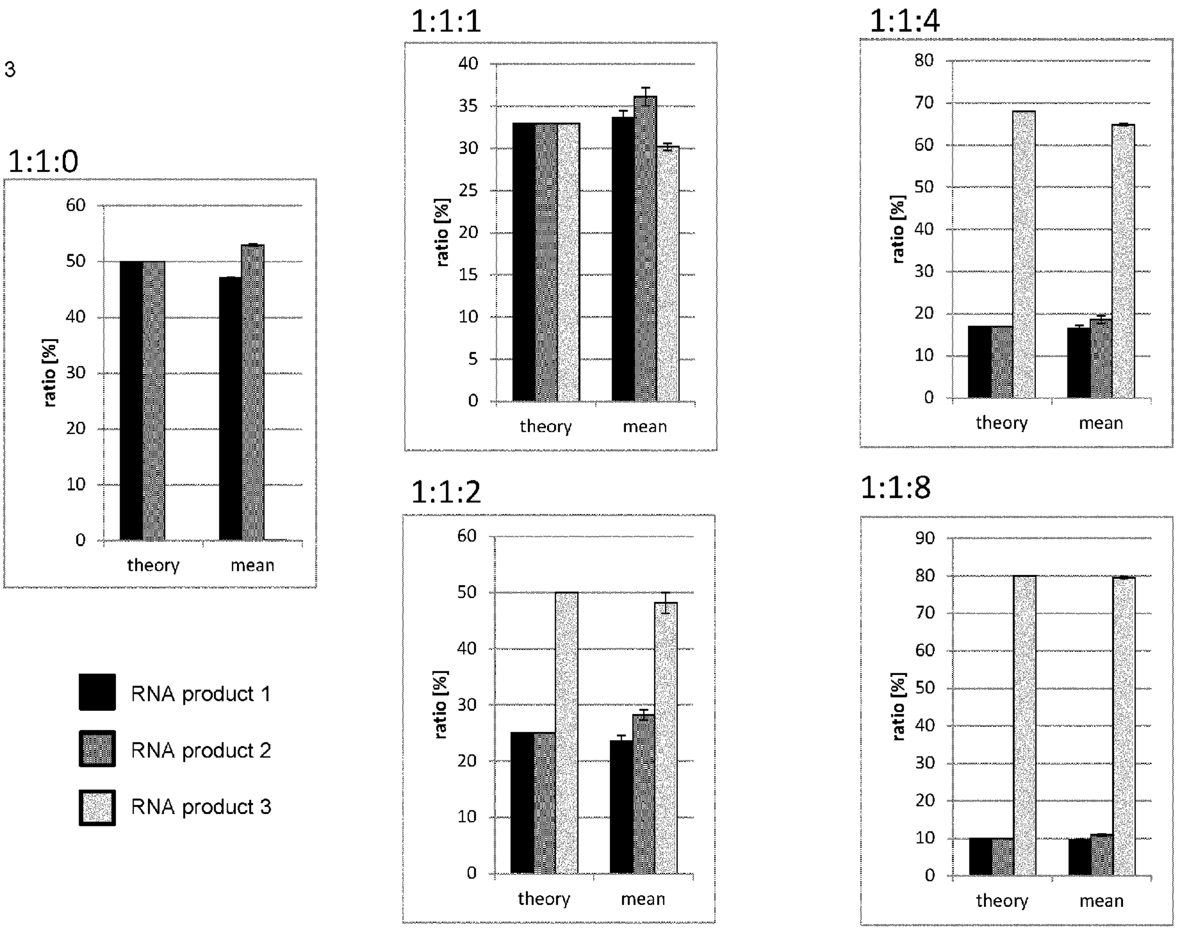
1:1:0
1:1:1
1:1:2
1:1:4
1:1:8
ratio [%]
theory
mean
RNA product 1
RNA product 2
RNA product 3

Figure 4

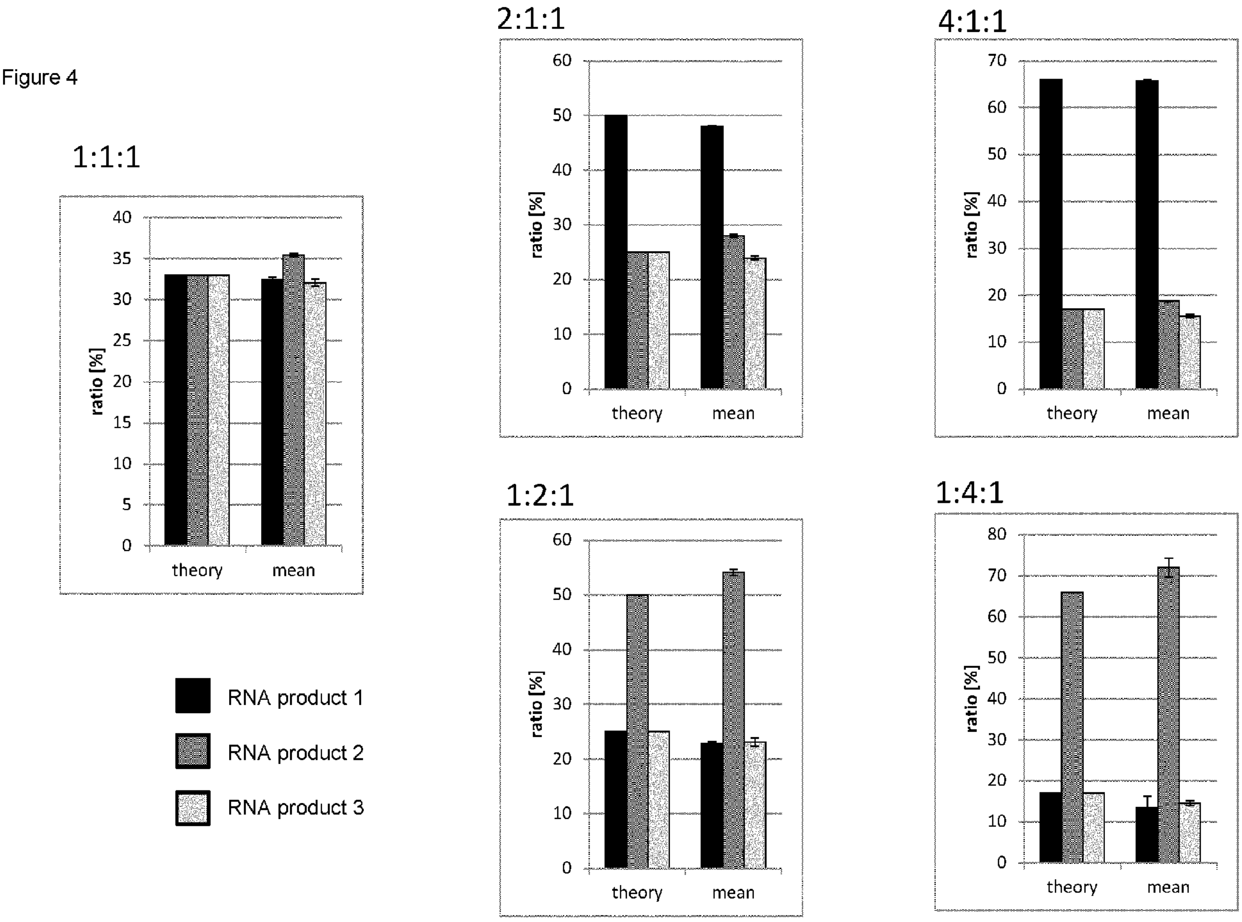
1:1:1
ratio [%]
theory
mean
2:1:1
ratio [%]
theory
mean
4:1:1
ratio [%]
theory
mean
1:2:1
ratio [%]
theory
mean
1:4:1
ratio [%]
theory
mean
RNA product 1
RNA product 2
RNA product 3

Europäisches Patentamt
European Patent Office
Office européen des brevets

# EUROPEAN SEARCH REPORT

Application Number
EP 23 19 8653

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 319 622 A1 (CUREVAC AG [DE]) 16 May 2018 (2018-05-16) * [0076],[0078],[0093],[0116]-[0120],[0354], [0357],[0365]; claim 1 * | 1-17 | INV. C12P19/34 C12N15/10 |
| X | MARRAS S. A. E.: "Real-time measurement of in vitro transcription", NUCLEIC ACIDS RESEARCH, vol. 32, no. 9, 17 May 2004 (2004-05-17), pages e72-e72, XP093133422, GB ISSN: 1362-4962, DOI: 10.1093/nar/gnh068 * page 1 of 6, column 2, paragraph 1 - page 2 of 6, column 1, paragraph 1 * * page 4 of 6, column 2, paragraph 2 - page 5 of 6, column 1, paragraph 1; figures 4,5 * | 1-5,9, 11,14, 15,17 | |
| X | MATTHEW B KERBY ET AL: "Early In Vitro Transcription Termination in Human H5 Influenza Viral RNA Synthesis", APPLIED BIOCHEMISTRY AND BIOTECHNOLOGY, HUMANA PRESS INC, NEW YORK, vol. 164, no. 4, 5 January 2011 (2011-01-05), pages 497-513, XP019898381, ISSN: 1559-0291, DOI: 10.1007/S12010-010-9152-4 * page 501, paragraph 3 * * page 503, paragraph 2; figures 2,8 * -/-- | 1-5,9, 10, 12-15,17 | TECHNICAL FIELDS SEARCHED (IPC) C12P C40B C12N |

The present search report has been drawn up for all claims

2

| Place of search | Date of completion of the search | Examiner |
|---|---|---|
| Berlin | 26 February 2024 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 19 8653

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | & Anonymous: "MEGAscript(TM) SP6 Transcription Kit User Manual", , 1 January 2012 (2012-01-01), pages 1-36, XP093134448, Retrieved from the Internet: URL:https://www.thermofisher.com/document-connect/document-connect.html?url=https://assets.thermofisher.com/TFS-Assets%2FLSG%2Fmanuals%2F1330M_G.pdf * the whole document * ----- | 1-17 | |
| | | | TECHNICAL FIELDS SEARCHED (IPC) |

The present search report has been drawn up for all claims

2

| Place of search | Date of completion of the search | Examiner |
|---|---|---|
| Berlin | 26 February 2024 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 19 8653

This annex lists the patent family members relating to the patent documents cited in the above-mentioned European search report. The members are as contained in the European Patent Office EDP file on
The European Patent Office is in no way liable for these particulars which are merely given for the purpose of information.

26-02-2024

| Patent document cited in search report | Publication date | Patent family member(s) | Publication date |
|---|---|---|---|
| EP 3319622 A1 | 16-05-2018 | AU 2016375021 A1 | 07-06-2018 |
| | | CA 3009551 A1 | 29-06-2017 |
| | | CN 108778308 A | 09-11-2018 |
| | | DK 3319622 T3 | 04-05-2020 |
| | | EP 3319622 A1 | 16-05-2018 |
| | | EP 3701963 A1 | 02-09-2020 |
| | | IL 259378 A | 31-07-2018 |
| | | JP 6949845 B2 | 13-10-2021 |
| | | JP 2019503678 A | 14-02-2019 |
| | | KR 20180107109 A | 01-10-2018 |
| | | SG 11201804398X A | 30-07-2018 |
| | | US 2019083602 A1 | 21-03-2019 |
| | | WO 2017109134 A1 | 29-06-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017060314 A
- WO 2007036366 A
- EP 2019056502 W
- WO 2016005324 A1
- WO 2017182524 A


### Non-patent literature cited in the description


- **SMITH** ; **WATERMAN**. *Ads App. Math.*, 1981, vol. 2, 482
- **NEDDLEMAN** ; **WUNSCH**. *J. Mol. Biol.*, 1970, vol. 48, 443
- **PEARSON** ; **LIPMAN**. *Proc. Natl Acad. Sci. USA*, 1988, vol. 88, 2444
- Molecular Cloning: A Laboratory Manual. Cold Spring Harbor Laboratory Press, 2012
- **JOSÉ et al.** *Future Microbiol.*, 2009, vol. 4, 837-856
- **GOULD et al.** *Antiviral Res.*, 2010, vol. 87, 111-124
- **HOLTKAMP et al.** *Blood*, 2006, vol. 108, 4009-4017
- **KACZMAREK, J. C. et al.** *Genome Medicine*, 2017, vol. 9, 60